# EUROPÄISCHE PATENTANMELDUNG

(11) **EP 2 062 983 A2**
(43) Veröffentlichungstag der Anmeldung: **27.05.2009**
(21) Anmeldenummer: 09002019.9
(22) Anmeldetag: 07.12.2004
(51) Int. Cl.: C12Q 1/70, G01N 33/53

(54) **Primer und Sonden zum Nachweis genitaler HPV-Genotypen**

(30) Priorität: 10.12.2003 DE 10357677
(62) Teilanmeldung aus: 04803571.1
(71) Anmelder: Greiner Bio-One GmbH, 72636 Frickenhausen (DE)
(72) Erfinder: Kranewitter, Wolfgang, 4020 Linz (AT); Mittermayr, Christian, 4111 Walding (AT); Winner, Florian, 4232 Hagenberg (AT); Iftner, Thomas, 72145 Hirrlingen (DE)
(74) Vertreter: Schrell, Andreas

(57) **Zusammenfassung**

Die vorliegende Erfindung betrifft Oligonucleotide, die als Primer zur Amplifikation von DNA genitaler humaner Papillomaviren (HPV) geeignet sind, Oligonucleotide, die als Sonden zur Typisierung genitaler HPV-Genotypen geeignet sind, Verfahren zur Amplifikation der DNA genitaler humaner Papillomaviren, Verfahren zum Nachweis und/oder zur Bestimmung genitaler HPV-Genotypen, die Oligonucleotide umfassende Nucleotid-Microarrays und Kits sowie die Verwendung der Oligonucleotide zur Amplifikation beziehungsweise Typisierung genitaler HPV-Genotypen, zur Diagnose und/oder Früherkennung von Krankheiten sowie zur Herstellung von Mitteln zur Diagnose von Krankheiten.

## Beschreibung

Die vorliegende Erfindung betrifft Oligonucleotide, die als Primer zur Amplifikation von DNA genitaler humaner Papillomaviren (HPV) geeignet sind, Oligonucleotide und Nucleinsäuremoleküle, die als Sonden zum Nachweis und/oder zur Bestimmung genitaler HPV-Genotypen eingesetzt werden können, Verfahren zur Amplifikation der DNA genitaler humaner Papillomaviren, Verfahren zum Nachweis und/oder zur Bestimmung genitaler HPV-Genotypen, die Oligonucleotide und Nucleinsäuremoleküle umfassende Nucleotid-Arrays und Kits sowie die Verwendung der Oligonucleotide und Nucleinsäuremoleküle zur Amplifikation beziehungsweise zum Nachweis und/oder zur Bestimmung genitaler HPV-Genotypen, zur Diagnose und/oder Früherkennung von Krankheiten sowie zur Herstellung von Mitteln zur Diagnose von Krankheiten.

Infektionen mit dem in der Bevölkerung weit verbreiteten humanpathogenen Papilloma-Virus gehören zu den häufigsten sexuell übertragbaren Viruserkrankungen. Die Ansteckung kann jedoch auch beim Neugeborenen über den Geburtsweg erfolgen. Als Folgen einer HPV-Erkrankung treten meist harmlose Hauterscheinungen auf. Bis heute wurden etwa hundert verschiedene Typen dieses Virus nachgewiesen. Papillomaviren werden unterteilt in kutane Typen, die vornehmlich in verhornendem Epithel Läsionen hervorrufen, und Mukosa-Typen, die insbesondere die Schleimhäute infizieren. Eine weitere Unterteilung erfolgt in mit gutartigen Läsionen assoziierten Typen (low risk types; Niedrig-Risiko-Typen) und solchen, die mit präneoplastischen und malignen Epithelveränderungen einhergehen (high risk types; Hoch-Risiko-Typen). Bekannte Hoch-Risiko-Typen sind beispielsweise die Typen 16, 18, 26, 31, 33, 35, 39, 45, 51, 52, 53, 56, 58, 59, 66, 68, 73, und 82. Bekannte Niedrig-Risiko-HPV-Typen sind beispielsweise die Typen 6, 11, 40, 42, 53, 54, 57 und MM8.

Etwa 25 der Papillomavirus-Typen stehen mit Erkrankungen im Genitalbereich der Frau in ursächlichem Zusammenhang. HPV-Infektionen manifestieren sich im ano-genitalen Bereich der Frau insbesondere in Form kondylomatöser, dysplastischer und neoplastischer Läsionen. Da 99,7 % aller Cervix-Karzinome Papillomavirus-DNA enthalten, wird inzwischen allgemein akzeptiert, dass humane Papilloma-Viren notwendige Risikofaktoren für die Entstehung dieser Krebserkrankung darstellen. Epidemiologische sowie molekulare Studien haben gezeigt, dass eine dauerhafte Infektion mit Hoch-Risiko-HPV-Typen, insbesondere den HPV-Typen 16 und 18, maßgeblich an der Entstehung der Cervix-Karzinoms beteiligt ist.

Sowohl beim Gebärmutterhalskrebs als auch bei anderen Krebserkrankungen, die epidemiologisch mit Papillomaviren assoziiert sind, beispielsweise bestimmten Formen des Hautkrebses, handelt es sich um vermeidbare Erkrankungen, wenn frühzeitige Erkennung und Behandlung gesichert sind. Ein zuverlässiges Verfahren zur Diagnose einer vorliegenden HPV-Infektion ist deshalb Voraussetzung für eine zielgerichtete Therapie.

Diagnostisch sind drei Formen der HPV-Infektion zu unterscheiden, nämlich die klinischen, subklinischen und latenten HPV-Infektionen. HPV-assoziierte Epithelveränderungen des subklinischen beziehungsweise klinisch-manifesten Stadiums der Infektion lassen sich zytologisch relativ gut erfassen. Frühstadien des Cervix-Karzinoms werden daher derzeit hauptsächlich durch Entnahme eines Zellabstriches von der Portio beziehungsweise Cervix uteri in Kombination mit der Kolposkopie erfasst. Obwohl die zytologischen Verfahren dazu beigetragen haben, dass die Inzidenz von Cervix-Karzinomen in den letzten Jahren signifikant gesenkt werden konnte, liefern sie dennoch keine vollkommen befriedigenden Ergebnisse. Eine Prognose über die Weiterentwicklung einzelner Läsionen ist nicht möglich. Die zytologischen Verfahren sind darüber hinaus mit einem relativ großen subjektiven Fehler behaftet und nicht standardisiert.

Da eine Anzucht und kulturelle Vermehrung von HPV nicht möglich ist, beruht der labortechnische HPV-Nachweis insbesondere auf der Erfassung viraler DNA oder Hüllproteine. Mittels immunhistochemischer Färbung lassen sich beispielsweise Gruppen-spezifische Antigene (Kapsidantigene) der Papillomaviren identifizieren. Dieser Test hat jedoch aufgrund seiner geringen Sensitivität nur geringe Aussagekraft. Auch serologische Untersuchungen zum Nachweis HPVspezifischer Antikörper im Serum von Patienten sind ohne Bedeutung für die Diagnostik, da sich selbst bei Patienten mit Cervix-Karzinom nur bei 50% aller Fälle Antikörper nachweisen lassen.

Eine große diagnostische Bedeutung haben Verfahren zum Nachweis viraler Nucleinsäuren in klinischen Gewebeproben. Besondere Bedeutung haben dabei solche Verfahren, die eine Unterscheidung zwischen individuellen Typen einer Niedrig- und Hoch-Risiko-HPV-Infektion ermöglichen, da nur die HPV-Typen HPV16, HPV18, HPV31 , HPV33, HPV35, HPV39, HPV45, HPV51, HPV52, HPV56, HPV58, HPV59, HPV66, HPV68 und HPV82 mit der Entwicklung von Cervix-Karzinomen in situ assoziiert sind, wobei wahrscheinlich auch HPV53 karzinogen ist.

Ein bekanntes Verfahren zum Nachweis von HPV-Nucleinsäuren ist das HCM (Hybrid Capture Microplate)-Verfahren der Firma Digene (HC2 HPV DNA-Test), dem ein signalverstärkendes Hybridisierungsverfahren zugrunde liegt. Als Hybridisierungssonden werden HPVspezifische RNA-Sequenzen eingesetzt. Nach Inkubation der Sonden mit denaturierter HPV-DNA aus infiziertem Gewebe werden die gebildeten RNA/DNA-Hybride über spezifische Antikörper an der Mikroplatten-Oberfläche einer Mikroplatte gebunden. Der Nachweis der RNA/DNA-Hybride erfolgt über einen zweiten Antikörper, der mit alkalischer Phosphatase markiert ist. Dieses Enzym kann nach Zugabe bestimmter Substanzen messbares Licht erzeugen. Das HCM-Verfahren erlaubt eine subgruppen-spezifische Differenzierung zwischen den low risk-Typen 6, 11, 42, 43 und 44 und den high risk-Typen 16, 18, 31, 33, 35, 39, 45, 51, 52, 56, 58, 59 und 68 und kann daher bei der Differentialdiagnostik unklarer zytologischer Befunde hilfreich sein. Ein Nachteil dieses Verfahrens ist allerdings, dass einige high risk-Typen nicht erfasst werden und dass es darüber hinaus zu einer Kreuzreaktion zwischen beiden Subgruppen kommt.

Viele im Stand der Technik bekannten Verfahren zum Nachweis viraler Nucleinsäuren in klinischen Gewebeproben beruhen auf vorausgehender HPV-Genamplifikation. Als sensitivstes Verfahren hat sich dabei das PCR-Verfahren erwiesen. Mittels derartiger Verfahren kann ein relativ breites Spektrum von HPV-Viren nachgewiesen und anschließend auch einer Typisierung unterworfen werden, wobei die Typisierung hauptsächlich mittels Sequenz-Analyse des PCR-Amplifikationsproduktes erfolgt. Die Typisierung erlaubt eine genaue Charakterisierung des HPV-Typs nicht nur bei Einzel-, sondern auch bei Mehrfach- beziehungsweise Mischinfektionen und ermöglicht so eine über den zytologischen Befund hinausgehende Beurteilung des onkogenen Potentials der nachgewiesenen HPV-Genotypen.

Snijders et al. J. Gen. Virol., 71 (1990), 173-181, und Surentheran et al., J. Clin. Path., 51 (1998), 606-610, beschreiben PCR-Verfahren zum Nachweis von HPV-DNA, wobei Primer-Paare eingesetzt werden, die innerhalb des HPV-Strukturgens L1 liegen. Das amplifizierte Genfragment wird dann anschließend sequenziert, um eine Typisierung der nachgewiesenen HPV-Typen vorzunehmen. Nachteilig bei beiden Verfahren ist allerdings, dass das L1-Gen weniger gut konserviert ist als andere Bereiche der HPV-DNA. Mittels der beschriebenen Verfahren kann daher nur eine begrenzte Anzahl von HPV-Typen erfasst werden. Beispielsweise lassen sich mittels der von Snijder et al. beschriebenen Primer einige HPV-Typen wie HPV30, HPV39 und HPV51 nur mit stark verminderter Sensitivität nachweisen. Darüber hinaus ergeben einige HPV-Typen wie HPV18 bei Verwendung der von Snijder et al. beschriebenen Primer zusätzliche Banden.

Die DE 100 09 143 A1 beschreibt ein PCR-Verfahren zum Nachweis einer allgemeinen HPV-Infektion, insbesondere jedoch von HPV-DNA im Anogenitalbereich, wobei 2 Primer-Paare eingesetzt werden, die innerhalb des konservierten HPV-Gens E1 liegen. Allerdings lassen sich nur die unter Verwendung eines Primer-Paares erhaltenen Amplifikationsprodukte zur sicheren Typisierung einsetzen, nicht jedoch die unter Verwendung des zweiten Primer-Paares erhaltenen Amplifikationsprodukte. Nachteilig ist ferner, dass die Typisierung erfolgt, indem die Amplifikationsprodukte entweder sequenziert oder mittels Temperaturgradienten-Gelelektrophorese untersucht werden müssen. Sowohl die Sequenzierung als auch die Durchführung einer Temperaturgradienten-Gelelektrophorese sind arbeits- und kostenintensive Verfahren.

Ein erheblicher Nachteil der bekannten kommerziellen Tests zum Nachweis und zur Typisierung von HPV-Genotypen besteht daher vor allem darin, dass diese ein nur eingeschränktes HPV-Typenspektrum nachweisen, wobei insbesondere seltene genitale HPV-Typen nur unzureichend erfasst werden, obwohl ein hohes onkogenes Potential einiger dieser Virustypen bekannt ist. Nachteilig ist ferner, dass nach der Amplifikation in jedem Fall eine zeit- und kostenintensive Sequenzierung durchgeführt werden muss, um eine Typisierung vornehmen zu können.

Das der vorliegenden Erfindung zugrunde liegende technische Problem besteht in der Bereitstellung von Mitteln und Verfahren, die einen schnellen und zweifelsfreien Nachweis beziehungsweise eine einfache und schnelle Typisierung insbesondere genitaler Hoch-Risiko-HPV-Genotypen erlauben und die die im Stand der Technik bekannten Nachteile nicht aufweisen, das heißt, insbesondere alle die HPV-Typen erfassen, von denen bekannt ist, dass sie mit Krebserkrankungen der Frau assoziiert sind.

Die vorliegende Erfindung löst das ihr zugrunde liegende technische Problem durch die Bereitstellung von Oligonucleotiden, die als Forward-Primer und Reverse-Primer zur Amplifikation eines Nucleinsäure-Bereiches eines genitalen humanen Papillomavirus (HPV) eingesetzt werden können. Die vorliegende Erfindung löst das ihr zugrunde liegende technische Problem insbesondere durch die Bereitstellung eines Oligonucleotides, das als Forward-Primer zur Amplifikation eines Nucleinsäure-Bereiches eines genitalen humanen Papillomavirus (HPV) eingesetzt werden kann, und das die Sequenz 5'-CAR GCI AAA WWW KTD AAR GAY TGT G-3' oder 5'-CAR GCN AAA WWW KTD AAR GAY TGT G-3' (SEQ ID Nr. 1) aufweist, wobei R = A oder G ist, W = T oder A ist, K = T oder G ist, I = Inosin ist, N = A, T, G oder C ist, D = A, T oder G ist und Y = C oder T ist.

Das als Forward-Primer eingesetzte erfindungsgemäße Oligonucleotid ist vorzugsweise ausgewählt aus der Gruppe bestehend aus:
a) einem Oligonucleotid mit der Sequenz 5'-CAR GCI AAA TAT KTR AAA GAT TGT G-3' oder 5'-CAR GCN AAA TAT KTR AAA GAT TGT G-3' (SEQ ID Nr. 2),
b) einem Oligonucleotid mit der Sequenz 5'-CAR GCA AAA TAT GTW AAG GAT TGT G-3' (SEQ ID Nr. 3),
c) einem Oligonucleotid mit der Sequenz 5'-CAR GCW AAA ATT GTA AAR GAT TGT G-3' (SEQ ID Nr. 4),
d) einem Oligonucleotid mit der Sequenz 5'-CAA GCA AAA ATA GTA AAR GAC TGT G-3' (SEQ ID Nr. 5) und
e) einem Oligonucleotid mit der Sequenz 5'-CAR GCA AAA TAT GTA AAA GAC TGT G-3' (SEQ ID Nr. 6),
wobei R = A oder G ist, W = T oder A ist, K = T oder G ist, I = Inosin ist und N = A, T, G oder C ist.

In besonders bevorzugter Ausführungsform der Erfindung wird als Forward-Primer zur Amplifikation der Nucleinsäure genitaler humaner Papillomaviren ein äquimolares Gemisch der Oligonucleotide mit den in SEQ ID Nr. 2 bis 6 dargestellten Nucleotidsequenzen eingesetzt.

Die vorliegende Erfindung löst das ihr zugrunde liegende technische Problem auch durch die Bereitstellung eines Oligonucleotids, das als Primer, insbesondere als Reverse-Primer, zur Amplifikation eines Nucleinsäure-Bereiches eines genitalen humanen Papillomavirus eingesetzt werden kann, wobei das Oligonucleotid die Sequenz 5'-ARY GGY TSY ARC CAA AAR TGR CT-3' (SEQ ID Nr. 7) aufweist, wobei R = A oder G ist, Y = C oder T ist und S = C oder G ist.

Die vorliegende Erfindung löst das ihr zugrunde liegende technische Problem auch durch die Bereitstellung eines Oligonucleotides, das als Sonde zum Nachweis und/oder zur Bestimmung genitaler HPV-Genotypen eingesetzt werden kann, ausgewählt aus der Gruppe bestehend aus:
1) einem Oligonucleotid mit der in SEQ ID Nr. 8, 9 oder 117 dargestellten Nucleotidsequenz zum Nachweis und/oder zur Bestimmung des HPV6-Genotyps, insbesondere des HPV6b-Genotyps,
2) einem Oligonucleotid mit der in SEQ ID Nr. 10, 11, 12, 13, 14, 15, 16, 17 oder 118 dargestellten Nucleotidsequenz zum Nachweis und/oder zur Bestimmung des HPV11-Genotyps,
3) einem Oligonucleotid mit der in SEQ ID Nr. 18, 19 oder 20 dargestellten Nucleotidsequenz zum Nachweis und/oder zur Bestimmung des HPV16-Genotyps,
4) einem Oligonucleotid mit der in SEQ ID Nr. 21, 22, 23, 24 oder 119 dargestellten Nucleotidsequenz zum Nachweis und/oder zur Bestimmung des HPV18-Genotyps,
5) einem Oligonucleotid mit der in SEQ ID Nr. 25, 26, 27 oder 28 dargestellten Nucleotidsequenz zum Nachweis und/oder zur Bestimmung des HPV26-Genotyps,
6) einem Oligonucleotid mit der in SEQ ID Nr. 29, 30, 31 oder 120 dargestellten Nucleotidsequenz zum Nachweis und/oder zur Bestimmung des HPV31-Genotyps,
7) einem Oligonucleotid mit der in SEQ ID Nr. 32, 33 oder 34 dargestellten Nucleotidsequenz zum Nachweis und/oder zur Bestimmung des HPV33-Genotyps,
8) einem Oligonucleotid mit der in SEQ ID Nr. 35, 36, 37, 38 oder 39 dargestellten Nucleotidsequenz zum Nachweis und/oder zur Bestimmung des HPV34-Genotyps,
9) einem Oligonucleotid mit der in SEQ ID Nr. 40 oder 41 dargestellten Nucleotidsequenz zum Nachweis und/oder zur Bestimmung des HPV35-Genotyps, insbesondere des HPV35h-Genotyps,
10) einem Oligonucleotid mit der in SEQ ID Nr. 42, 43, 44, 45 oder 46 dargestellten Nucleotidsequenz zum Nachweis und/oder zur Bestimmung HPV39-Genotyps,
11) einem Oligonucleotid mit der in SEQ ID Nr. 47, 48, 49 oder 50 dargestellten Nucleotidsequenz zum Nachweis und/oder zur Bestimmung des HPV40-Genotyps,
12) einem Oligonucleotid mit der in SEQ ID Nr. 51, 52 oder 121 dargestellten Nucleotidsequenz zum Nachweis und/oder zur Bestimmung des HPV42-Genotyps,
13) einem Oligonucleotid mit der in SEQ ID Nr. 122 dargestellten Nucleotidsequenz zum Nachweis und/oder zur Bestimmung des HPV43-Genotyps,
14) einem Oligonucleotid mit der in SEQ ID Nr. 53, 54, 55 oder 123 dargestellten Nucleotidsequenz zum Nachweis und/oder zur Bestimmung des HPV44-Genotyps,
15) einem Oligonucleotid mit der in SEQ ID Nr. 56, 57, 58, 59, 60, 61 oder 124 dargestellten Nucleotidsequenz zum Nachweis und/oder zur Bestimmung des HPV45-Genotyps,
16) einem Oligonucleotid mit der in SEQ ID Nr. 62, 63, 64 oder 125 dargestellten Nucleotidsequenz zum Nachweis und/oder zur Bestimmung des HPV51-Genotyps,
17) einem Oligonucleotid mit der in SEQ ID Nr. 65, 66, 67 oder 126 dargestellten Nucleotidsequenz zum Nachweis und/oder zur Bestimmung des HPV52-Genotyps,
18) einem Oligonucleotid mit der in SEQ ID Nr. 68, 69, 70, 71, 72, 73 oder 127 dargestellten Nucleotidsequenz zum Nachweis und/oder zur Bestimmung des HPV53-Genotyps,
19) einem Oligonucleotid mit der in SEQ ID Nr. 74, 75, 76 oder 77 dargestellten Nucleotidsequenz zum Nachweis und/oder zur Bestimmung des HPV54-Genotyps,
20) einem Oligonucleotid mit der in SEQ ID Nr. 78, 79, 80, 81 oder 128 dargestellten Nucleotidsequenz zum Nachweis und/oder zur Bestimmung des HPV56-Genotyps,
21) einem Oligonucleotid mit der in SEQ ID Nr. 82, 83, 84, 85 oder 86 dargestellten Nucleotidsequenz zum Nachweis und/oder zur Bestimmung des HPV58-Genotyps,
22) einem Oligonucleotid mit der in SEQ ID Nr. 87 oder 129 dargestellten Nucleotidsequenz zum Nachweis und/oder zur Bestimmung des HPV59-Genotyps,
23) einem Oligonucleotid mit der in SEQ ID Nr. 88, 89, 90, 91, 92, 93 oder 94 dargestellten Nucleotidsequnz zum Nachweis und/oder zur Bestimmung des HPV61-Genotyps,
24) einem Oligonucleotid mit der in SEQ ID Nr. 95, 96, 97 oder 130 dargestellten Nucleotidsequenz zum Nachweis und/oder zur Bestimmung des HPV66-Genotyps,
25) einem Oligonucleotid mit der in SEQ ID Nr. 98, 99, 100, 101 oder 102 dargestellten Nucleotidsequenz zum Nachweis und/oder zur Bestimmung des HPV67-Genotyps,
26) einem Oligonucleotid mit der in SEQ ID Nr. 103, 104, 105, 131 oder 132 dargestellten Nucleotidsequenz zum Nachweis und/oder zur Bestimmung des HPV68-Genotyps,
27) einem Oligonucleotid mit der in SEQ ID Nr. 106, 107, 108 oder 133 dargestellten Nucleotidsequenz zum Nachweis und/oder zur Bestimmung des HPV70-Genotyps,
28) einem Oligonucleotid mit der in SEQ ID Nr. 109, 110, 111, 112 oder 134 dargestellten Nucleotidsequenz zum Nachweis und/oder zur Bestimmung des HPV73-Genotyps, und
29) einem Oligonucleotid mit der in SEQ ID Nr. 113, 114, 115, 116 oder 135 dargestellten Nucleotidsequenz zum Nachweis und/oder zur Bestimmung des HPV82-Genotyps.

Eine besonders bevorzugte Ausführungsform betrifft die Bereitstellung eines Oligonucleotides, das als Sonde zum Nachweis und/oder zur Bestimmung genitaler HPV-Genotypen eingesetzt werden kann, ausgewählt aus den Oligonucleotiden mit einer der in SEQ ID Nr. 19, SEQ ID Nr. 32, SEQ ID Nr. 41, SEQ ID Nr. 44, SEQ ID Nr. 48, SEQ ID Nr. 82 oder SEQ ID Nr. 117 bis SEQ ID Nr. 135 dargestellten Nucleotidsequenzen.

Die erfindungsgemäßen Oligonucleotide, insbesondere die mit den in SEQ ID Nr. 1 bis 7 dargestellten Nucleotidsequenzen, erlauben in vorteilhafter Weise eine äußerst spezifische Amplifikation von Nucleinsäure-Bereichen bei einer Vielzahl unterschiedlicher humaner HPV-Typen, wobei erfindungsgemäß insbesondere vorgesehen ist, dass ein spezifischer Bereich des HPV-Gens E1 amplifiziert wird. Das E1-Gen als Zielbereich zur Amplifikation und damit zum Nachweis von HPV-Typen bietet gegenüber anderen Bereichen des HPV-Genoms eine Reihe erheblicher Vorteile.

Das ringförmige HPV-Genom mit einer Größe von etwa 7,9 kB besteht aus den Genen E6, E7, E1, E2, E4, E5, L2, L1 und der "long control region" (LCR), wobei die Gene in dieser Reihenfolge auf dem Genom angeordnet sind. Bei HPV-Infektionen kommt es sehr häufig zu einer Integration des Virus in das humane Genom, wobei in vielen Fällen Teile des Virus-Genoms deletiert werden. So ist bekannt, dass beispielsweise in Karzinomen die Gene E2, E4, E5 und L2 zumindest teilweise deletiert werden können. Die Gene E2, E4, E5 und L2 kommen somit als Zielregion zur Amplifikation nicht in Frage, da ein gesicherter HPV-DNA-Nachweis in einer Probe nicht möglich ist. Hingegen ist bekannt, dass E1 bei Integration des Virus in das humane Genom sehr häufig erhalten bleibt, wobei E1 zumindest weit weniger häufig als das L1-Gen deletiert wird. Ob E1 allerdings immer erhalten bleibt, ist derzeit noch nicht völlig geklärt. E1 wird jedoch mit großer Wahrscheinlichkeit auch bei Integration des Virus in das humane Genom amplifiziert und kann deshalb bei sehr vielen HPV-Infektionen nachgewiesen werden. Unter Verwendung der erfindungsgemäßen Oligonucleotid-Primer können daher insbesondere auch persistierende HPV-Infektionen erfasst werden, bei denen das Risiko der Krebsentwicklung sehr groß ist.

Obwohl auch der LCR-Bereich und die Gene E6 und E7 bei Integration des Virus in das humane Genom erhalten bleiben und nicht deletiert werden, sind auch diese Genom-Bereiche als Zielregion zur Amplifikation von HPV-DNA nicht geeignet, da die Sequenzen dieser Bereiche bei den verschiedenen HPV-Typen teilweise stark divergieren können. Die Verwendung dieser Genom-Bereiche als Zielregion zur Amplifikation würde eine Vielzahl unterschiedlicher Primer-Paare erfordern, um alle HPV-Typen zu erfassen. Auch vom L1-Gen ist bekannt, dass seine Sequenz bei den einzelnen HPV-Typen weniger konserviert ist als andere HPV-Genombereiche, so dass nicht alle HPV-Typen erfasst werden können. Im Gegensatz dazu weist das E1-Gen den Vorteil auf, dass es bei den unterschiedlichen HPV-Typen relativ gut konserviert ist, so dass bei Amplifikation des E1-Gens ein äußerst breites Spektrum unterschiedlicher HPV-Typen erfasst wird. Minimale Sequenzunterschiede zwischen einzelnen HPV-Typen werden erfindungsgemäß insbesondere dadurch kompensiert, dass in bevorzugter Ausführungsform der Erfindung ein äquimolares Gemisch verschiedener Oligonucleotide, insbesondere der Oligonucleotide mit den in SEQ ID Nr. 2 bis 6 dargestellten Nucelotidsequenzen, als Forward-Primer eingesetzt wird, so dass mittels einer einzigen Amplifikationsreaktion bei allen HPV-Typen ein Amplifikationsprodukt erhalten und somit ein Nachweis aller HPV-Typen ermöglicht wird.

Erfindungsgemäß ist vorgesehen, dass das unter Verwendung der erfindungsgemäßen Forward- und Reverse-Primer erhaltene Amplifikationsprodukt dann unter Verwendung der erfindungsgemäßen Oligonucleotid-Sonden nachgewiesen wird. Die erfindungsgemäß als Sonden eingesetzten Oligonucleotide erlauben in äußerst vorteilhafter Weise den gezielten Nachweis individueller HPV-Typen und somit eine Typisierung der HPV-Typen in einer biologischen Probe. Unter Verwendung der erfindungsgemäßen Oligonucleotid-Sonden lassen sich mehr als zwanzig sehr häufig auftretende genitalpathogene HPV-Typen nachweisen und unterscheiden. In einer besonders bevorzugten Ausführungsform der Erfindung sind die erfindungsgemäßen Oligonucleotid-Sonden auf einem Nucleotid-Array enthalten, so dass eine Vielzahl von Amplifikationsprodukten in kurzer Zeit auf einfache Art und Weise getestet und eine Typisierung der HPV-Genotypen vorgenommen werden kann. Die erfindungsgemäßen Oligonucleotide erlauben somit eine äußerst effiziente und schnelle Typisierung von Papillomaviren, während die im Stand der Technik bekannten Typisierungs-Verfahren zeitaufwändige und kostenintensive Sequenz-Analysen oder Gelelektrophoresen erfordern.

Erfindungsgemäß ist insbesondere vorgesehen, die erfindungsgemäßen Oligonucleotide beziehungsweise die unter Verwendung dieser Oligonucleotide durchgeführten erfindungsgemäßen Verfahren zur Amplifikation beziehungsweise zum Nachweis und zur Typisierung von humanen Papilloma-Viren insbesondere in solchen Fällen einzusetzen, in denen bereits vorher eine HPV-Infektion festgestellt und nachgewiesen wurde. Erfindungsgemäß ist also vorgesehen, die erfindungsgemäßen Oligonucleotide und Verfahren primär nicht zum Screenen auf HPV-Infektionen einzusetzen, sondern zur Typisierung, d.h. zum Nachweis und zur Bestimmung individueller HPV-Genotypen. Zur Feststellung beziehungsweise zum Nachweis der HPV-Infektion kann ein herkömmlicher Test, beispielsweise der Hybrid Capture-Test von Digene eingesetzt werden. Mit Hilfe des Hybrid Capture-Tests lassen sich Low- von High Risk-Typen unterscheiden. Da dieser Test sehr häufig zur Untersuchung von High Risk-Typen eingesetzt wird, ist zu erwarten, dass mit diesem Test hauptsächlich die klinisch relevanten High Risk-Typen erfasst werden, die dann unter Verwendung der erfindungsgemäßen Oligonucleotide beziehungsweise der erfindungsgemäßen Verfahren typisiert werden können.

Im Zusammenhang mit der vorliegenden Erfindung wird unter einem "Oligonucleotid" ein isoliertes und gereinigtes Molekül verstanden, das aus zwei bis weniger als einhundert Nucleotiden besteht. Bei den erfindungsgemäßen Oligonucleotiden kann es sich um, vorzugsweise gereinigte und isolierte, DNA-, RNA-, PNA- oder LNA-Moleküle oder Mischformen davon handeln.

Bei "PNA" ("Peptide Nucleic Acid" oder "Polyamide Nucleic Acid")-Sequenzen handelt es sich um Moleküle, die nicht negativ geladen sind und in gleicher Weise wie DNA wirken (Nielsen et al., Science, 254 (1991), 1497-1500; Nielsen et al., Biochemiestry, 26 (1997), 5072-5077; Weiler et al., Nuc. Acids Res., 25 (1997), 2792-2799). PNA-Sequenzen umfassen ein Polyamid-Grundgerüst aus N-(2-Aminoethyl)-glycin-Einheiten und besitzen keine Glucose-Einheiten und keine Phosphat-Gruppen. Die unterschiedlichen Basen sind über Methylen-Carbonyl-Bindungen an das Grundgerüst gebunden. "LNA" (Locked Nucleic Acid)-Moleküle sind **dadurch gekennzeichnet, dass** die Furanose-Ring-Konformation durch einen Methylen-Linker beschränkt ist, der die 2'-O-Position mit der 4'-C-Position verbindet. LNAs werden als einzelne Nucleotide in Nucleinsäuren, beispielsweise DNA- oder RNA, eingebaut. LNA-Oligonucleotide unterliegen ebenfalls wie PNA-Moleküle den Watson-Crick-Basenpaarungs-Regeln und hybridisieren an komplementäre Oligonucleotide. LNA/DNA- oder LNA/RNA-Duplexmoleküle zeigen gegenüber ähnlichen Duplexmolekülen, die ausschließlich aus DNA oder RNA gebildet werden, erhöhte thermische Stabilität.

Erfindungsgemäß ist vorgesehen, dass die erfindungsgemäßen Oligonucleotide Nucleotidsequenzen aufweisen, die partiell oder vollständig zu Sequenzen aus dem E1-Genbereich genitaler HPV-Genotypen komplementär sind. Insbesondere ist vorgesehen, dass die Oligonucleotide mit den in SEQ ID Nr. 1 bis 7 dargestellten Nucleotidsequenzen komplementär zu Konsensus-Sequenzen sind, die im E1-Genbereich von mindestens 29 genitalen HPV-Genotypen vorhanden sind, so dass unter Verwendung dieser Oligonucleotide als Forward- und/oder Reverse-Primer eine Amplifikation des E1-Genbereiches aller dieser 29 genitalen HPV-Genotypen möglich ist. Erfindungsgemäß ist gleichzeitig vorgesehen, dass die Oligonucleotide mit den in SEQ ID Nr. 8 bis 135 dargestellten Nucleotidsequenzen, insbesondere die Oligonucleotide mit den in SEQ ID Nr. 19, 32, 41, 44, 48, 82 und 117 bis 135 dargestellten Sequenzen, hingegen nur zu Sequenzen jeweils eines bestimmten genitalen HPV-Genotyps komplementär sind, jedoch keine Komplementarität zu den Sequenzen anderer genitaler HPV-Genotypen aufweisen, so dass die Verwendung eines der Oligonucleotide mit den in SEQ ID Nr. 8 bis 135 dargestellten Nucleotidsequenzen, insbesondere eines der Oligonucleotide mit den in SEQ ID Nr. 19, 32, 41, 44, 48, 82 und 117 bis 135 dargestellten Sequenzen, den spezifischen Nachweis eines bestimmten genitalen HPV-Genotyps ermöglicht. Im Zusammenhang mit der vorliegenden Erfindung ist eine Nucleinsäure zu einer anderen Nucleinsäure "komplementär", wenn sie mit dieser über ihre gesamte Länge oder zumindest über den größten Teil ihrer Länge hinweg einen Doppelstrang bilden kann, in dem alle Nucleotide nach den Regeln von Watson und Crick durch Wasserstoff-Brückenbindung gepaart vorliegen.

In einer weiteren bevorzugten Ausführungsform der Erfindung weisen die erfindungsgemäßen Oligonucleotide eine Nucleotidsequenz auf, die gegenüber einer der in SEQ ID Nr. 1 bis 135 dargestellten Nucleotidsequenzen, insbesondere gegenüber einer der in SEQ ID Nr. 1 bis 7, 19, 32, 41, 44, 48, 82 oder 117 bis 135 dargestellten Nucleotidsequenzen mutiert ist, wobei die Oligonucleotide erhältlich sind durch:
a) Deletion von 1 bis 10 Nucleotiden in einer der in SEQ ID Nr. 1 bis 135 dargestellten Nucleotidsequenzen, insbesondere in einer der in SEQ ID Nr. 1 bis 7, 19, 32, 41, 44, 48, 82 oder 117 bis 135 dargestellten Nucleotidsequenzen,
b) Addition von 1 bis 10 Nucleotiden in einer der in SEQ ID Nr. 1 bis 135 dargestellten Nucleotidsequenzen, insbesondere in einer der in SEQ ID Nr. 1 bis 7, 19, 32, 41, 44, 48, 82 oder 117 bis 135 dargestellten Nucleotidsequenzen, und/oder
c) Substitution von 1 bis 3 Nucleotiden in einer der in SEQ ID Nr. 1 bis 135 dargestellten Nucleotidsequenzen, insbesondere in einer der in SEQ ID Nr. 1 bis 7, 19, 32, 41, 44, 48, 82 oder 117 bis 135 dargestellten Nucleotidsequenzen.

Erfindungsgemäß bevorzugt liegt die Deletion oder Addition der Nucleotide am 5'-Ende und/oder 3'-Ende einer der in SEQ ID Nr. 1 bis 135 dargestellten Nucleotidsequenzen vor. Im Falle einer Addition ist erfindungsgemäß ferner bevorzugt, dass die zusätzlichen Nucleotide zusammen mit einer der in SEQ ID Nr. 1 bis 135 dargestellten Nucleotidsequenzen eine Sequenz bilden, die über ihre gesamte Länge oder zumindest über den größten Teil ihrer Länge hinweg zu den entsprechenden Zielsequenzen im E1-Genbereich komplementär ist.

Die vorliegende Erfindung umfasst selbstverständlich auch Oligonucleotide, die eine Nucleotidsequenz aufweisen, die über ihre gesamte Länge hinweg zu einer der in SEQ ID Nr. 1 bis 135 dargestellten Nucleotidsequenzen, insbesondere zu einer der in SEQ ID Nr. 1 bis 7, 19, 32, 41, 44, 48, 82 oder 117 bis 135 dargestellten Nucleotidsequenzen, oder zu einer der erfindungsgemäßen mutierten Nucleotidsequenzen, die dadurch charakterisiert sind, dass sie gegenüber einer der in SEQ ID Nr. 1 bis 135 dargestellten Nucleotidsequenzen, insbesondere einer der in SEQ ID Nr. 1 bis 7, 19, 32, 41, 44, 48, 82 oder 117 bis 135 dargestellten Nucleotidsequenzen, eine Addition und/oder Deletion von 1 bis 10 Nucleotiden und/oder eine Substitution von 1-3 Nucleotiden aufweisen, komplementär ist.

Die vorliegende Erfindung löst das ihr zugrunde liegende technische Problem auch durch die Bereitstellung eines Primer-Paars zur Amplifikation eines Nucleinsäure-Bereiches eines genitalen humanen Papilloma-Virus, umfassend einen Forward-Primer und einen Reverse-Primer, wobei der Forward-Primer ausgewählt ist aus der Gruppe bestehend aus:
a) einem Oligonucleotid mit einer der in SEQ ID Nr. 1 bis 6 dargestellten Nucleotidsequenzen,
b) einem Oligonucleotid, das eine gegenüber dem Oligonucleotid nach a) mutierte Nucleotidsequenz aufweist, d.h. mit einer Addition und/oder Substitution von 1 bis 10 Nucleotiden und/oder einer Substitution von 1 bis 3 Nucleotiden, und
c) einem Gemisch der Oligonucleotide nach a) und/oder b)
und der Reverse-Primer ausgewählt ist aus der Gruppe bestehend aus:
d) einem Oligonucleotid mit der in SEQ ID Nr. 7 dargestellten Nucleotidsequenz,
e) einem Oligonucleotid, das eine gegenüber dem Oligonucleotid nach d) mutierte Nucleotidsequenz aufweist, d.h. mit einer Addition und/oder Deletion von 1 bis 10 Nucleotiden und/oder einer Substitution von 1 bis 3 Nucleotiden, und
f) einem Gemisch der Oligonucleotide nach d) und e).

In einer bevorzugten Ausführungsform umfasst das Primer-Paar ein äquimolares Gemisch der Oligonucleotide mit den in SEQ ID Nr. 2 bis 6 dargestellten Nucleotidsequenzen als Forward-Primer und das Oligonucleotid mit der in SEQ ID Nr. 7 dargestellten Nucelotidsequenz als Reverse-Primer.

In einer weiteren bevorzugten Ausführungsform der Erfindung ist vorgesehen, dass Oligonucleotide mit einer der in SEQ ID Nr. 8 bis 135 dargestellten Nucleotidsequenzen, insbesondere einer der in SEQ ID Nr. 19, 32, 41, 44, 48, 82 oder 117 bis 135 dargestellten Nucleotidsequenzen, einer mutierten Nucleotidsequenz davon oder einer komplementären Sequenz davon Teil eines längeren Oligonucleotids oder Polynucleotids sein können. Das längere Oligonucleotid oder Polynucleotid, das im Folgenden auch als Nucleinsäuremolekül bezeichnet wird, umfasst daher neben einer der in SEQ ID Nr. 8 bis 135 dargestellten Nucleotidsequenzen, insbesondere einer der in SEQ ID Nr. 19, 32, 41, 44, 48, 82 oder 117 bis 135 dargestellten Nucleotidsequenzen, einer mutierten Sequenz davon oder einer komplementären Sequenz weitere zusätzliche Nucleotide und/oder Nucleotidsequenzen. Im Zusammenhang mit der vorliegenden Erfindung wird unter einem "Polynucleotid" ein gereinigtes und isoliertes Molekül verstanden, das aus mindestens einhundert Nucleotiden besteht.

Die vorliegende Erfindung betrifft daher auch ein, vorzugsweise gereinigtes und isoliertes Nucleinsäuremolekül, umfassend mindestens einen Bereich, der eine der in SEQ ID Nr. 8 bis 135 dargestellten Nucleotidsequenzen, insbesondere eine der in SEQ ID Nr. 1 bis 7, 19, 32, 41, 44, 48, 82 oder 117 bis 135 dargestellten Nucleotidsequenzen, eine mutierte Nucleotidsequenz davon, erhältlich durch Deletion und/oder Addition von 1 bis 10 Nucleotiden und/oder Substitution von 1 bis 3 Nucleotiden einer der in SEQ ID Nr. 8 bis 135 dargestellten Nucleotidsequenzen, insbesondere einer der in SEQ ID Nr. 1 bis 7, 19, 32, 41, 44, 48, 82 oder 117 bis 135 dargestellten Nucleotidsequenzen, oder eine zu diesen Sequenzen komplementäre Nucleotidsequenz aufweist, und einen oder mehr zusätzliche Bereiche mit einer Gesamtlänge von mindestens 1 Nucleotid. Bei dem erfindungsgemäßen Nucleinsäuremolekül kann es sich um ein längeres Oligonucleotid oder ein Polynucleotid handeln. Wenn das erfindungsgemäße Nucleinsäuremolekül als längeres Oligonucleotid vorliegt, beträgt die Gesamtlänge des erfindungsgemäßen längeren Oligonucleotids höchstens 99 Nucleotide. Wenn das erfindungsgemäße längere Oligonucleotid beispielsweise eine der in SEQ ID Nr. 8 bis 135 dargestellten Nucleotidsequenzen umfasst, können die zusätzlichen Bereiche daher eine Gesamtlänge von mindestens 1 Nucleotid bis höchstens 69 bis 72 Nucleotiden aufweisen. Wenn das erfindungsgemäße längere Oligonucleotid eine Nucleotidsequenz umfasst, die gegenüber einer der in SEQ ID Nr. 8 bis 135 dargestellten Nucleotidsequenzen durch Addition von 1 bis 10 Nucleotiden mutiert ist, können die zusätzlichen Bereiche eine Gesamtlänge von mindestens 1 Nucleotid bis höchstens 68-71 Nucleotiden (im Falle der Addition eines Nucleotids) beziehungsweise 59-62 Nucleotiden (im Falle der Addition von 10 Nucleotiden) aufweisen. Die Gesamtlänge der zusätzlichen Bereiche des als längeres Oligonucleotid vorliegenden Nucleinsäuremoleküls beträgt insbesondere 1 bis etwa 60, vorzugsweise 1 bis etwa 50, besonders bevorzugt 1 bis etwa 40 und am bevorzugtesten 1 bis etwa 30 Nucleotide. Wenn das erfindungsgemäße Nucleinsäuremolekül als Polynucleotid vorliegt, beträgt dessen Gesamtlänge mindestens 100 Nucleotide. Die Gesamtlänge der zusätzlichen Bereiche des erfindungsgemäßen Polynucleotids beträgt in Abhängigkeit davon, ob das erfindungsgemäße Polynucleotid eine der in SEQ ID Nr. 8 bis 135 dargestellten Sequenzen oder eine mutierte Sequenz davon umfasst, mindestens etwa 70 bis 80 Nucleotide bis etwa 1000 Nucleotide, vorzugsweise etwa 70-80 Nucleotide bis etwa 500 Nucleotide, besonders bevorzugt etwa 70-80 Nucleotide bis etwa 250 Nucleotide und am bevorzugtesten etwa 70-80 Nucleotide bis etwa 100 Nucleotide.

Der in dem erfindungsgemäßen Nucleinsäuremolekül enthaltene mindestens eine Bereich, der eine der in SEQ ID Nr. 8 bis 135 dargestellten Nucleotidsequenzen, insbesondere eine der in SEQ ID Nr. 1 bis 7, 19, 32, 41, 44, 48, 82 oder 117 bis 135 dargestellten Nucleotidsequenzen, eine mutierte Nucleotidsequenz davon oder eine komplementäre Nucleotidsequenz davon aufweist, kann am 5'-Ende und/oder am 3'-Ende der Sequenz des Nucleinsäuremoleküls und/oder zwischen den zusätzlichen Bereichen angeordnet sein.

In einer Ausführungsform der Erfindung ist vorgesehen, dass diese zusätzlichen Bereiche des erfindungsgemäßen Nucleinsäuremoleküls Nucleotidsequenzen aufweisen, die zu Sequenzen eines Amplifikationsproduktes, das mittels eines Amplifikationsverfahrens unter Verwendung eines Nucleinsäure-Bereiches eines genitalen humanen Papillomavirus als Matrize und eines erfindungsgemäßen Primer-Paares erhältlich ist, komplementär sind. Beispielsweise können die zusätzlichen Bereiche mehrfache Wiederholungen des Bereiches, der eine der in SEQ ID Nr. 8 bis 135 dargestellten Nucleotidsequenzen, eine mutierte Nucleotidsequenz davon oder eine komplementäre Nucleotidsequenz davon aufweist, umfassen. Erfindungsgemäß ist es möglich, dass die Wiederholungen dieses Bereiches jeweils durch Spacer mit einer Länge von mindestens einem Nucleotid oder mindestens einem Phosphat oder mindestens einem Kohlenstoffatom oder mindestens einer Aminogruppe getrennt sind.

In einer weiteren Ausführungsform der Erfindung ist vorgesehen, dass die zusätzlichen Bereiche der erfindungsgemäßen Nucleinsäuremoleküle Nucleotidsequenzen aufweisen, die zu Sequenzen eines Amplifikationsproduktes, das mittels eines Amplifikationsverfahrens unter Verwendung eines Nucleinsäure-Bereiches eines genitalen humanen Papillomavirus als Matrize und eines erfindungsgemäßen Primer-Paares erhältlich ist, keine Komplementarität aufweisen und somit mit dem Amplifikationsprodukt nicht hybridisieren. Beispielsweise können die zusätzlichen Bereiche der erfindungsgemäßen Nucleinsäuremoleküle Poly-A- oder Poly-T-Nucleotidsequenzen aufweisen.

Die vorliegende Erfindung betrifft ebenfalls Nucleinsäuremoleküle, die eine Nucleotidsequenz aufweisen, die über ihre gesamte Länge hinweg zu der Nucleotidsequenz eines erfindungsgemäßen Nucleinsäuremoleküls komplementär ist. Erfindungsgemäß ist ferner vorgesehen, dass die erfindungsgemäßen Nucleinsäuremoleküle als, vorzugsweise gereinigte und isolierte, DNA-Moleküle, RNA-Moleküle, PNA-Moleküle, LNA-Moleküle oder Mischformen davon vorliegen.

Die vorliegende Erfindung löst das ihr zugrunde liegende technische Problem auch durch die Bereitstellung eines Verfahrens zur Amplifikation eines Bereiches einer in einer biologischen Probe vorhandenen Nucleinsäure eines genitalen humanen Papillomavirus, umfassend die Durchführung eines Nucleinsäureamplifikations-Verfahrens unter Verwendung eines einen Forward-Primer und eine Reverse-Primer umfassenden Primer-Paares, wobei der Forward-Primer ausgewählt ist aus der Gruppe bestehend aus:
a) einem Oligonucleotid mit einer der in SEQ ID Nr. 1 bis 6 dargestellten Nucleotidsequenz,
b) einem Oligonucleotid, das eine gegenüber dem Oligonucleotid nach a) mutierte Nucleotidsequenz aufweist und
c) einem Gemisch der Oligonucleotide nach a) und/oder b),
und der Reverse-Primer ausgewählt ist aus der Gruppe bestehend aus:
d) einem Oligonucleotid mit der in SEQ ID Nr. 7 dargestellten Nucleotidsequenz,
e) einem Oligonucleotid, das eine gegenüber dem Oligonucleotid nach d) mutierte Nucleotidsequenz aufweist und
f) einem Gemisch der Oligonucleotide nach d) und e).

Unter Verwendung des erfindungsgemäßen Verfahrens können in vorteilhafter Weise spezifische Nucleinsäurebereiche, insbesondere ein Bereich des konservierten E1-Gens, einer Vielzahl genitaler humaner Papilloma-Viren amplifiziert werden. Die dabei erhaltenen Amplifikationsprodukte erlauben den Nachweis der Gegenwart eines Papilloma-Virus beziehungsweise mehrerer Papilloma-Viren in einer biologischen Probe, während das Fehlen eines Amplifikationsproduktes darauf hinweist, dass in der untersuchten Probe kein HPV-Virus vorhanden ist. Das unter Verwendung des erfindungsgemäßen Amplifikationsverfahrens erhaltene Amplifikationsprodukt kann dann mittels der erfindungsgemäßen Oligonucleotid- und/oder Polynucleotid-Sonden zur Typisierung des nachgewiesenen Papilloma-Virus beziehungsweise der nachgewiesenen Papilloma-Viren eingesetzt werden.

Im Zusammenhang mit der vorliegenden Erfindung wird unter einer "biologischen Probe" ein Haut- oder Schleimhautabstrich, eine Organbiopsie, eine Gewebebiopsie, eine Körperflüssigkeit oder ein Körpersekret verstanden. Die Probe kann sowohl einem lebenden oder toten Organismus, Organ oder Gewebe entnommen sein. Eine "biologische Probe" kann erfindungsgemäß auch eine Kultur oder ein Kulturmedium sein, beispielsweise ein Medium, in dem menschliche oder tierische Zellen kultiviert wurden. Bei einer Probe im Sinne der Erfindung kann es sich auch um eine wässrige Lösung, Emulsion, Dispersion oder Suspension handeln, die isolierte und aufgereinigte humane Papillomaviren oder Bestandteile davon enthält. Eine biologische Probe kann bereits Aufreinigungsschritten unterworfen worden sein, kann aber auch ungereinigt vorliegen.

In einer bevorzugten Ausführungsform der Erfindung ist die biologische Probe ein Abstrich der Cervix uteri, eine frische Cervix uteri-Gewebeprobe, eine fixierte Cervix uteri-Gewebeprobe oder ein Schnittpräparat einer Cervix uteri-Gewebeprobe. Erfindungsgemäß ist vorgesehen, dass es sich bei der Probe insbesondere um eine solche Probe handelt, die im Rahmen einer zytologischen Untersuchung und/oder einer Kolposkopie zur Erfassung HPV-assoziierter Epithelveränderungen des subklinischen beziehungsweise klinisch-manifesten Stadiums einer HPV-Infektion entnommen wird.

Erfindungsgemäß ist vorgesehen, dass die zu amplifizierende Nucleinsäure vor der Amplifikation unter Verwendung der erfindungsgemäßen Primer aus der biologischen Probe aufgereinigt und/oder isoliert werden kann. Die Aufreinigung und Isolierung der zu amplifizierenden Nucleinsäure, d.h. der zu amplifizierenden HPV-DNA, kann unter Verwendung der im Stand der Technik bekannten Verfahren erfolgen.

In bevorzugter Ausführungsform der Erfindung ist vorgesehen, dass zur Nucleinsäureamplifikation ein PCR (Polymerase-Ketten-Reaktion; polymerase chain reaction)-Verfahren eingesetzt wird. Bei dem PCR-Verfahren handelt es sich um ein Verfahren zur gezielten Vervielfältigung einer spezifischen Nucleinsäure oder eines Bereiches davon in einem Gemisch unterschiedlicher oder ähnlicher Sequenzen. Das Verfahren basiert darauf, dass bestimmte Reaktionsschritte, nämlich Denaturierung eines zu amplifizierenden doppelsträngigen Nucleinsäure-Moleküls, Anlagerung von Primern an die erhaltenen einzelsträngigen Nucleinsäure-Moleküle (annealing) und eine von den angelagerten Primern ausgehende Synthese von komplementären Strängen (extension), wobei die einzelsträngigen Nucleinsäure-Moleküle als Matrize dienen, mehrfach wiederholt werden. In einem ersten Schritt wird daher die zu amplifizierende doppelsträngige Nucleinsäure bei einer geeigneten Temperatur denaturiert, wobei als Matrizen dienende einzelsträngige Nucleinsäure erhalten werden. Danach erfolgt ein Absenken der Temperatur, wobei im Reaktionsansatz im Überschuss enthaltene Oligonucleotide (Primer), deren Sequenzen zum Anfang beziehungsweise zum Ende der zu amplifizierenden Nucleinsäuren komplementär sind, mit den komplementären Nucleinsäurebereichen hybridisieren. Bei einer Temperatur, die der Hybridisierungstemperatur von etwa 50°C bis 72°C entsprechen kann, die aber auch auf das Optimum von 72°C bis 75°C der, vorzugsweise hitzestabilen, DNA-Polymerase, eingestellt sein kann, synthetisiert die Polymerase dann ausgehend von den Primern Kopien der Ausgangs-Nucleinsäure in Form von Primer-Verlängerungsprodukten, wobei die Länge der Kopien durch den Abstand zwischen den Primern bestimmt wird. Eine Kettenreaktion wird dadurch erhalten, dass ein Zyklus durchgeführt wird, der die Denaturierung des Primer-Verlängerungsproduktes von der Matrize, die Behandlung der so erzeugten einsträngigen Moleküle mit den gleichen Primern und die Bildung weiterer Primer-Verlängerungsprodukte umfasst. Der Zyklus wird solange wiederholt, bis die zu amplifizierende Ziel-Nucleinsäure in einer gewünschten Menge vorliegt.

In bevorzugter Ausführungsform der Erfindung wird die PCR-Amplifikation der nachzuweisenden HPV-DNA unter folgenden Temperatur-Bedingungen durchgeführt:
a) Erhitzen auf 95°C, wobei die Temperatur pro Sekunde um 1°C erhöht wird,
b) Halten der Temperatur bei 95°C für 10 Minuten,
c) Durchführung von 40 Zyklen, jeweils umfassend 30 Sekunden bei 95°C, 30 Sekunden bei 55°C und 1 Minute bei 72°C,
d) Halten der Temperatur bei 72°C für 5 Minuten und
e) Abkühlen auf 4°C.

Erfindungsgemäß ist vorgesehen, dass die als Forward-Primer und Reverse-Primer eingesetzten Oligonucleotide in der Nucleinsäure-Amplifikationsreaktion jeweils in einer Konzentration von 0,5-1 pMol/µl eingesetzt werden. Besonders bevorzugt wird als Forward-Primer ein äquimolares Gemisch der Oligonucleotide mit den in SEQ ID Nr. 2 bis 6 dargestellten Nucleotidsequenzen eingesetzt, wobei jedes Oligonucleotid in einer Konzentration von 0,1-0,2 pMol/µl vorliegt, und als Reverse-Primer das Oligonucleotid mit der in SEQ ID Nr. 7 dargestellten Nucleotidsequenz in einer Konzentration von 0,5-1 pMol/µl. Die als Forward- und Reverse-Primer eingesetzten Oligonucleotide können als DNA-, RNA-, PNA- oder LNA-Moleküle oder Mischformen davon eingesetzt werden.

In einer anderen Ausführungsform der Erfindung ist vorgesehen, dass das Nucleinsäureamplifikations-Verfahren ein LCR (ligase chain reaction)-Verfahren, ein NASBA-Verfahren oder ein isothermisches Verfahren ist. Das LCR-Verfahren basiert auf der wiederholten Durchführung von zwei Reaktionsschritten, wobei im ersten Schritt eine doppelsträngige Nucleinsäure bei hoher Temperatur denaturiert wird. Im zweiten Schritt werden zwei Sätze benachbarter komplementärer Oligonucleotide an die im ersten Schritt erhaltene einzelsträngige Nucleinsäure hybridisiert und miteinander ligiert. Die Produkte der Ligierung aus dieser Reaktion dienen bei Wiederholung des Verfahrens als Matrizen. Wie die PCR resultiert auch das LCR-Verfahren in einer exponentiellen Amplifikation der Ligierungsprodukte.

Erfindungsgemäß ist insbesondere vorgesehen, dass im erfindungsgemäßen Amplifikationsverfahren ein spezifischer Bereich des HPV-Gens E1 amplifiziert wird. Erfindungsgemäß ist selbstverständlich vorgesehen, dass der amplifizierte Nucleinsäurebereich im Anschluss aufgereinigt und/oder isoliert werden kann, um beispielsweise eine HPV-Typisierung vorzunehmen.

Erfindungsgemäß ist ebenfalls vorgesehen, dass das unter Verwendung des erfindungsgemäßen Verfahrens hergestellte Amplifikationsprodukt während oder nach der Amplifikation mit einer Markierung versehen werden kann, wodurch der spätere Nachweis des Amplifikationsproduktes, insbesondere unter Verwendung eines oder mehrerer der erfindungsgemäß als Sonden eingesetzten erfindungsgemäßen Oligonucleotide mit den in SEQ ID Nr. 8 bis 135 dargestellten Nucleotidsequenzen, vorzugsweise mit den in SEQ ID Nr. 1 bis 7, 19, 32, 41, 44, 48, 82 und 117 bis 135 dargestellten Nucleotidsequenzen, mutierten Nucleotidsequenzen davon oder komplementären Nucleotidsequenzen davon und/oder der erfindungsgemäßen-Nucleinsäuremoleküle, ermöglicht wird. Eine Markierung des Amplifikationsproduktes während der Amplifikationsreaktion kann beispielsweise unter Verwendung von markierten Primern und/oder markierten Nucleotiden erfolgen. Das Amplifikationsprodukt kann selbstverständlich auch nach Beendigung der Amplifikationsreaktion unter Verwendung von im Stand der Technik bekannten Verfahren erfolgen. Erfindungsgemäß kann das Amplifikationsprodukt zum Beispiel unter Verwendung von Fluoreszenz-Farbstoffen, Biotin, Haptenen, Antigenen, chemischen Gruppen, beispielsweise durch Verwendung aminoallyl-markierter Nucleotide oder durch Anbringen eines Markers mittels einer Succinimid-Reaktion mit dem aminoallyl-markierten Nucelotide, radioaktiven Stoffen, enzymatischen Markern etc. markiert werden. Die Detektion des markierten Amplifikationsproduktes kann beispielsweise unter Verwendung von Fluoreszenz-Verfahren, Chemolumineszenz-Verfahren, Densitometrie-Verfahren, Photometrie-Verfahren, Fällungsreaktionen, enzymatischen Reaktionen einschließlich enzymatischer Verstärkungsreaktionen, SPR ("surface plamon resonance")-Verfahren, Ellipsometrie-Verfahren, Messung des Brechungsindex, Messung der Reflektivität und ähnlichen Verfahren erfolgen.

Die vorliegende Erfindung löst das ihr zugrunde liegende technische Problem auch durch die Bereitstellung eines Verfahrens zum Nachweis und/oder zur Bestimmung eines individuellen genitalen HPV-Genotyps, umfassend die Untersuchung einer in einer biologischen Probe vorhandenen Nucleinsäure eines genitalen humanen Papillomavirus, insbesondere die Untersuchung des HPV-Gens E1 oder eines Teils davon, durch Hybridisierung mit mindestens einer Sonde, wobei die Sonde ausgewählt ist aus der Gruppe bestehend aus:
a) HPV-Genotyp-spezifischen Oligonucleotiden mit den in SEQ ID Nr. 8 bis 135 dargestellten Nucleotidsequenzen, insbesondere den in SEQ ID Nr. 1 bis 7, 19, 32, 41, 44, 48, 82 oder 117 bis 135 dargestellten Nucleotidsequenzen,
b) Oligonucleotiden, die eine gegenüber einem der Oligonucleotide nach a) mutierte Nucleotidsequenz aufweisen,
c) Oligonucleotiden, die eine Nucleotidsequenz aufweisen, die gegenüber der eines Oligonucleotids nach a) oder b) komplementär ist
d) Nucleinsäuremolekülen, die mindestens einen Bereich umfassen, der eine der in SEQ ID Nr. 8 bis 135 dargestellten Nucleotidsequenzen, insbesondere eine der in SEQ ID Nr. 1 bis 7, 19, 32, 41, 44, 48, 82 oder 117 bis 135 dargestellten Nucleotidsequenzen, eine mutierte Nucleotidsequenz davon oder eine komplementäre Nucleotidsequenz davon aufweist, und
e) Gemischen der Oligonucleotide nach a) bis c) und/oder der Nucleinsäuremoleküle nach d),
   und den Nachweis der Hybridisierung.

Das erfindungsgemäße Verfahren zum Nachweis und/oder zur Bestimmung eines individuellen genitalen HPV-Genotyps dient insbesondere zur Typisierung von HPV-Genotypen, deren Vorhandensein in einer biologischen Probe bereits mittels eines anderen Verfahrens gesichert nachgewiesen wurde. Der Nachweis des Vorliegens einer HPV-Infektion kann beispielsweise unter Verwendung des HCM-Verfahrens erfolgt sein, mit dessen Hilfe high risk-Typen von low risk-Typen unterschieden werden können.

In einer bevorzugten Ausführungsform des erfindungsgemäßen Verfahrens ist vorgesehen, dass die in der biologischen Probe vorhandene HPV-Nucleinsäure vor Hybridisierung mit der/den Oligonucleoitid- oder Nucleinsäuremolekül-Sonde(n) amplifiziert und ein Amplifikationsprodukt erhalten wurde, dass im erfindungsgemäßen Verfahren zur Typisierung mittels der erfindungsgemäßen Oligonucleotid- und/oder Nucleinsäuremolekül-Sonden untersucht werden kann. Besonders bevorzugt erfolgt die Amplifikation der HPV-Nucleinsäure unter Verwendung des erfindungsgemäßen Verfahrens zur Amplifikation von HPV-Nucleinsäuren, das heißt unter Verwendung der Oligonucleotide mit den in SEQ ID Nr. 1, 2, 3, 4, 5 und 6 dargestellten Nucleotidsequenzen als Forward-Primer und des Oligonucleotids mit der in SEQ ID Nr. 7 dargestellten Nucleotidsequenz als Reverse-Primer. Erfindungsgemäß ist daher vorgesehen, dass die biologische Probe, die einer erfindungsgemäßen Typisierung unterworfen werden soll, einen amplifizierten Nucleinsäurebereich umfasst, der mittels des erfindungsgemäßen Verfahrens zur NucleinsäureAmplifikation erhalten wurde.

Selbstverständlich kann das erfindungsgemäße Verfahren zum Nachweis und/oder zur Bestimmung, d.h. zur Typisierung eines genitalen HPV-Genotyps auch an einer biologischen Probe durchgeführt werden, die keiner Amplifikation eines HPV-Nucleinsäurebereiches unterworfen wurde. In einer anderen Ausführungsform der Erfindung ist daher vorgesehen, dass die biologische Probe ein Abstrich der Cervix uteri, eine frische Cervix uteri-Gewebeprobe, eine fixierte Cervix uteri-Gewebeprobe oder ein Schnittpräparat einer solchen Gewebeprobe ist.

Die Hybridisierung mit den erfindungsgemäßen Oligonucleotid- oder Nucleinsäuremolekül-Sonden wird unter Bedingungen durchgeführt, die im Stand der Technik beschrieben sind.

Erfindungsgemäß ist vorgesehen, dass eine Hybridisierung mit einem Oligonucleotid mit der in SEQ ID Nr. 8, 9 oder 117 dargestellten Nucleotidsequenz, vorzugsweise der in SEQ ID Nr. 117 dargestellten Nucleotidsequenz, einer mutierten Nucleotidsequenz davon oder einer komplementären Nucleotidsequenz davon oder mit einem Nucleinsäuremolekül, das eine der in SEQ ID Nr. 8, 9 oder 117 dargestellten Nucleotidsequenzen, vorzugsweise die in SEQ ID Nr. 117 dargestellte Nucleotidsequenz, eine mutierte Nucleotidsequenz davon oder eine komplementäre Nucleotidsequenz davon umfasst, den HPV6-Genotyp, insbesondere HPV6b-Genotyp anzeigt.

Eine Hybridisierung mit einem Oligonucleotid mit der in SEQ ID Nr. 10, 11, 12, 13, 14, 15, 16, 17 oder 118 dargestellten Nucleotidsequenz, vorzugsweise der in SEQ ID Nr. 118 dargestellten Nucleotidsequenz, einer mutierten Nucleotidsequenz davon oder einer komplementären Nucleotidsequenz davon oder mit einem Nucleinsäuremolekül, das eine der in SEQ ID Nr. 10, 11, 12, 13, 14, 15, 16, 17 oder 118 dargestellten Nucleotidsequenzen, vorzugsweise die in SEQ ID Nr. 118 dargestellte Nucleotidsequenz, eine mutierte Nucleotidsequenz davon oder eine komplementäre Nucleotidsequenz davon umfasst, zeigt erfindungsgemäß den HPV11-Genotyp an.

Erfindungsgemäß ist vorgesehen, dass eine Hybridisierung mit einem Oligonucleotid mit der in SEQ ID Nr. 18, 19 oder 20, vorzugsweise der in SEQ ID Nr. 19 dargestellten Nucleotidsequenz,dargestellten Nucleotidsequenz, einer mutierten Nucleotidsequenz davon oder einer komplementären Nucleotidsequenz davon oder mit einem Nucleinsäuremolekül, das eine der in SEQ ID Nr. 18, 19 oder 20 dargestellten Nucleotidsequenzen, vorzugsweise die in SEQ ID Nr. 19 dargestellte Nucleotidsequenz, eine mutierte Nucleotidsequenz davon oder eine komplementäre Nucleotidsequenz davon umfasst, den HPV16-Genotyp anzeigt.

Erfindungsgemäß ist vorgesehen, dass eine Hybridisierung mit einem Oligonucleotid mit der in SEQ ID Nr. 21, 22, 23, 24 oder 119 dargestellten Nucleotidsequenz, vorzugsweise der in SEQ ID Nr. 119 dargestellten Nucleotidsequenz, einer mutierten Nucleotidsequenz davon oder einer komplementären Nucleotidsequenz davon oder mit einem Nucleinsäuremolekül, das eine der in SEQ ID Nr. 21, 22, 23, 24 oder 119 dargestellten Nucleotidsequenzen, vorzugsweise die in SEQ ID Nr. 119 dargestellte Nucleotidsequenz, eine mutierte Nucleotidsequenz davon oder eine komplementäre Nucleotidsequenz davon umfasst, den HPV18-Genotyp anzeigt.

Erfindungsgemäß ist darüber hinaus vorgesehen, dass eine Hybridisierung mit einem Oligonucleotid mit der in SEQ ID Nr. 25, 26, 27 oder 28 dargestellten Nucleotidsequenz, einer mutierten Nucleotidsequenz davon oder einer komplementären Nucleotidsequenz davon oder mit einem Nucleinsäuremolekül, das eine der in SEQ ID Nr. 25, 26, 27 oder 28 dargestellten Nucleotidsequenzen, eine mutierte Nucleotidsequenz davon oder eine komplementäre Nucleotidsequenz davon umfasst, den HPV26-Genotyp anzeigt.

Eine Hybridisierung mit einem Oligonucleotid mit der in SEQ ID Nr. 29, 30, 31 oder 120 dargestellten Nucleotidsequenz, vorzugsweise der in SEQ ID Nr. 120 dargestellten Nucleotidsequenz, einer mutierten Nucleotidsequenz davon oder einer komplementären Nucleotidsequenz davon oder mit einem Nucleinsäuremolekül, das eine der in SEQ ID Nr. 29, 30, 31 oder 120 dargestellten Nucleotidsequenzen, vorzugsweise die in SEQ ID Nr. 120 dargestellte Nucleotidsequenz, eine mutierte Nucleotidsequenz davon oder eine komplementäre Nucleotidsequenz davon umfasst, zeigt erfindungsgemäß den HPV31-Genotyp an.

Erfindungsgemäß ist darüber hinaus vorgesehen, dass eine Hybridisierung mit einem Oligonucleotid mit der in SEQ ID Nr. 32, 33 oder 34 dargestellten Nucleotidsequenz, vorzugsweise der in SEQ ID Nr. 32 dargestellten Nucleotidsequenz, einer mutierten Nucleotidsequenz davon oder einer komplementären Nucleotidsequenz davon oder mit einem Nucleinsäuremolekül, das eine der in SEQ ID Nr. 32, 33 oder 34 dargestellten Nucleotidsequenzen, vorzugsweise die in SEQ ID Nr. 32 dargestellte Nucleotidsequenz, eine mutierte Nucleotidsequenz davon oder eine komplementäre Nucleotidsequenz davon umfasst, den HPV33-Genotyp anzeigt.

In einer weiteren Ausführungsform der Erfindung ist vorgesehen, dass eine Hybridisierung mit einem Oligonucleotid mit der in SEQ ID Nr. 35, 36, 37, 38 oder 39 dargestellten Nucleotidsequenz, einer mutierten Nucleotidsequenz davon oder einer komplementären Nucleotidsequenz davon oder mit einem Nucleinsäuremolekül, das eine der in SEQ ID Nr. 35, 36, 37, 38 oder 39 dargestellten Nucleotidsequenzen, eine mutierte Nucleotidsequenz davon oder eine komplementäre Nucleotidsequenz davon umfasst, den HPV34-Genotyp anzeigt.

Erfindungsgemäß ist vorgesehen, dass eine Hybridisierung mit einem Oligonucleotid mit der in SEQ ID Nr. 40 oder 41 dargestellten Nucleotidsequenz, vorzugsweise der in SEQ ID Nr. 41 dargestellten Nucleotidsequenz, einer mutierten Nucleotidsequenz davon oder einer komplementären Nucleotidsequenz davon oder mit einem Nucleinsäuremolekül, das eine der in SEQ ID Nr. 40 oder 41 dargestellten Nucleotidsequenzen, vorzugsweise die in SEQ ID Nr. 41 dargestellte Nucleotidsequenz, eine mutierte Nucleotidsequenz davon oder eine komplementäre Nucleotidsequenz davon umfasst, den HPV35-Genotyp, insbesondere HPV35h-Genotyp anzeigt.

Erfindungsgemäß ist ferner vorgesehen, dass eine Hybridisierung mit einem Oligonucleotid mit der in SEQ ID Nr. 42, 43, 44, 45 oder 46 dargestellten Nucleotidsequenz, vorzugsweise der in SEQ ID Nr. 44 dargestellten Nucleotidsequenz, einer mutierten Nucleotidsequenz davon oder einer komplementären Nucleotidsequenz davon oder mit einem Nucleinsäuremolekül, das eine der in SEQ ID Nr. 42, 43, 44, 45 oder 46 dargestellten Nucleotidsequenzen, vorzugsweise die in SEQ ID Nr. 44 dargestellte Nucleotidsequenz, eine mutierte Nucleotidsequenz davon oder eine komplementäre Nucleotidsequenz davon umfasst, den HPV39-Genotyp anzeigt.

Eine Hybridisierung mit einem Oligonucleotid mit der in SEQ ID Nr. 47, 48, 49 oder 50 dargestellten Nucleotidsequenz, vorzugsweise der in SEQ ID Nr. 48 dargestellten Nucleotidsequenz, einer mutierten Nucleotidsequenz davon oder einer komplementären Nucleotidsequenz davon oder mit einem Nucleinsäuremolekül, das eine der in SEQ ID Nr. 47, 48, 49 oder 50 dargestellten Nucleotidsequenzen, vorzugsweise die in SEQ ID Nr. 48 dargestellte Nucleotidsequenz, eine mutierte Nucleotidsequenz davon oder eine komplementäre Nucleotidsequenz davon umfasst, zeigt erfindungsgemäß den HPV40-Genotyp an.

Erfindungsgemäß ist darüber hinaus vorgesehen, dass eine Hybridisierung mit einem Oligonucleotid mit der in SEQ ID Nr. 51, 52 oder 121 dargestellten Nucleotidsequenz, vorzugsweise der in SEQ ID Nr. 121 dargestellten Nucleotidsequenz, einer mutierten Nucleotidsequenz davon oder einer komplementären Nucleotidsequenz davon oder mit einem Nucleinsäuremolekül, das eine der in SEQ ID Nr. 51, 52 oder 121 dargestellten Nucleotidsequenzen, vorzugsweise die in SEQ ID Nr. 121 dargestellte Nucleotidsequenz, eine mutierte Nucleotidsequenz davon oder eine komplementäre Nucleotidsequenz davon umfasst, den HPV42-Genotyp anzeigt.

Erfindungsgemäß ist darüber hinaus vorgesehen, dass eine Hybridisierung mit einem Oligonucleotid mit der in SEQ ID Nr. 122 dargestellten Nucleotidsequenz, einer mutierten Nucleotidsequenz davon oder einer komplementären Nucleotidsequenz davon oder mit einem Nucleinsäuremolekül, das eine der in SEQ ID Nr. 122 dargestellten Nucleotidsequenzen, eine mutierte Nucleotidsequenz davon oder eine komplementäre Nucleotidsequenz davon umfasst, den HPV43-Genotyp anzeigt.

Eine Hybridsierung mit einem Oligonucleotid mit der in SEQ ID Nr. 53, 54, 55 oder 123 dargestellten Nucleotidsequenz, vorzugsweise der in SEQ ID Nr. 123 dargestellten Nucleotidsequenz, einer mutierten Nucleotidsequenz davon oder einer komplementären Nucleotidsequenz davon oder mit einem Nucleinsäuremolekül, das eine der in SEQ ID Nr. 53, 54, 55 oder 123 dargestellten Nucleotidsequenzen, vorzugsweise die in SEQ ID Nr. 123 dargestellte Nucleotidsequenz, eine mutierte Nucleotidsequenz davon oder eine komplementäre Nucleotidsequenz davon umfasst, zeigt erfindungsgemäß den HPV44-Genotyp an.

Erfindungsgemäß ist ferner vorgesehen, dass eine Hybridisierung mit einem Oligonucleotid mit der in SEQ ID Nr. 56, 57, 58, 59, 60, 61 oder 124 dargestellten Nucleotidsequenz, vorzugsweise der in SEQ ID Nr. 124 dargestellten Nucleotidsequenz, einer mutierten Nucleotidsequenz davon oder einer komplementären Nucleotidsequenz davon oder mit einem Nucleinsäuremolekül, das eine der in SEQ ID Nr. 56, 57, 58, 59, 60, 61 oder 124 dargestellten Nucleotidsequenzen, vorzugsweise die in SEQ ID Nr. 124 dargestellte Nucleotidsequenz, eine mutierte Nucleotidsequenz davon oder eine komplementäre Nucleotidsequenz davon umfasst, den HPV45-Genotyp anzeigt.

Eine Hybridisierung mit einem Oligonucleotid mit der in SEQ ID Nr. 62, 63, 64 oder 125 dargestellten Nucleotidsequenz, vorzugsweise der in SEQ ID Nr. 125 dargestellten Nucleotidsequenz, einer mutierten Nucleotidsequenz davon oder einer komplementären Nucleotidsequenz davon oder mit einem Nucleinsäuremolekül, das eine der in SEQ ID Nr. 62, 63, 64 oder 125 dargestellten Nucleotidsequenzen, vorzugsweise die in SEQ ID Nr. 125 dargestellte Nucleotidsequenz, eine mutierte Nucleotidsequenz davon oder eine komplementäre Nucleotidsequenz davon umfasst, zeigt erfindungsgemäß den HPV51-Genotyp an.

Erfindungsgemäß ist ferner vorgesehen, dass eine Hybridisierung mit einem Oligonucleotid mit der in SEQ ID Nr. 65, 66, 67 oder 126 dargestellten Nucleotidsequenz, vorzugsweise der in SEQ ID Nr. 126 dargestellten Nucleotidsequenz, einer mutierten Nucleotidsequenz davon oder einer komplementären Nucleotidsequenz davon oder mit einem Nucleinsäuremolekül, das eine der in SEQ ID Nr. 65, 66, 67 oder 126 dargestellten Nucleotidsequenzen, vorzugsweise die in SEQ ID Nr. 126 dargestellte Nucleotidsequenz, eine mutierte Nucleotidsequenz davon oder eine komplementäre Nucleotidsequenz davon umfasst, den HPV52-Genotyp anzeigt.

Eine Hybridisierung mit einem Oligonucleotid mit der in SEQ ID Nr. 68, 69, 70, 71, 72, 73 oder 127 dargestellten Nucleotidsequenz, vorzugsweise der in SEQ ID Nr. 127 dargestellten Nucleotidsequenz, einer mutierten Nucleotidsequenz davon oder einer komplementären Nucleotidsequenz davon oder mit einem Nucleinsäuremolekül, das eine der in SEQ ID Nr. 68, 69, 70, 71, 72, 73 oder 127 dargestellten Nucleotidsequenzen, vorzugsweise die in SEQ ID Nr. 127 dargestellte Nucleotidsequenz, eine mutierte Nucleotidsequenz davon oder eine komplementäre Nucleotidsequenz davon umfasst, zeigt erfindungsgemäß den HPV53-Genotyp an.

In einer weiteren Ausführungsform des erfindungsgemäßen Verfahrens ist vorgesehen, dass eine Hybridisierung mit einem Oligonucleotid mit der in SEQ ID Nr. 74, 75, 76 oder 77 dargestellten Nucleotidsequenz, einer mutierten Nucleotidsequenz davon oder einer komplementären Nucleotidsequenz davon oder mit einem Nucleinsäuremolekül, das eine der in SEQ ID Nr. 74, 75, 76 oder 77 dargestellten Nucleotidsequenzen, eine mutierte Nucleotidsequenz davon oder eine komplementäre Nucleotidsequenz davon umfasst, den HPV54-Genotyp anzeigt.

Eine Hybridisierung mit einem Oligonucleotid mit der in SEQ ID Nr. 78, 79, 80, 81 oder 128 dargestellten Nucleotidsequenz, vorzugsweise der in SEQ ID Nr. 128 dargestellten Nucleotidsequenz, einer mutierten Nucleotidsequenz davon oder einer komplementären Nucleotidsequenz davon oder mit einem Nucleinsäuremolekül, das eine der in SEQ ID Nr. 78, 79, 80, 81 oder 128 dargestellten Nucleotidsequenzen, vorzugsweise die in SEQ ID Nr. 128 dargestellte Nucleotidsequenz, eine mutierte Nucleotidsequenz davon oder eine komplementäre Nucleotidsequenz davon umfasst, zeigt erfindungsgemäß den HPV56-Genotyp an.

Erfindungsgemäß ist ferner vorgesehen, dass eine Hybridisierung mit einem Oligonucleotid mit der in SEQ ID Nr. 82, 83, 84, 85 oder 86 dargestellten Nucleotidsequenz, vorzugsweise der in SEQ ID Nr. 82 dargestellten Nucleotidsequenz, einer mutierten Nucleotidsequenz davon oder einer komplementären Nucleotidsequenz davon oder mit einem Nucleinsäuremolekül, das eine der in SEQ ID Nr. 82, 83, 84, 85 oder 86 dargestellten Nucleotidsequenzen, vorzugsweise die in SEQ ID Nr. 82 dargestellte Nucleotidsequenz, eine mutierte Nucleotidsequenz davon oder eine komplementäre Nucleotidsequenz davon umfasst, den HPV58-Genotyp anzeigt.

Eine Hybridisierung mit einem Oligonucleotid mit der in SEQ ID Nr. 87 oder 129 dargestellten Nucleotidsequenz, vorzugsweise der in SEQ ID Nr. 129 dargestellten Nucleotidsequenz, einer mutierten Nucleotidsequenz davon oder einer komplementären Nucleotidsequenz davon oder mit einem Nucleinsäuremolekül, das die in SEQ ID Nr. 87 oder 129 dargestellte Nucleotidsequenz, vorzugsweise die in SEQ ID Nr. 129 dargestellten Nucleotidsequenz, eine mutierte Nucleotidsequenz davon oder eine komplementäre Nucleotidsequenz davon umfasst, zeigt erfindungsgemäß den HPV59-Genotyp an.

In einer weiteren Ausführungsform der Erfindung ist vorgesehen, dass eine Hybridisierung mit einem Oligonucleotid mit der in SEQ ID Nr. 88, 89, 90, 91, 92, 93 oder 94 dargestellten Nucleotidsequenz, einer mutierten Nucleotidsequenz davon oder einer komplementären Nucleotidsequenz davon oder mit einem Nucleinsäuremolekül, das eine der in SEQ ID Nr. 88, 89, 90, 91, 92, 93 oder 94 dargestellten Nucleotidsequenzen, eine mutierte Nucleotidsequenz davon oder eine komplementäre Nucleotidsequenz davon umfasst, den HPV61-Genotyp anzeigt.

Eine Hybridisierung mit einem Oligonucleotid mit der in SEQ ID Nr. 95, 96, 97 oder 130 dargestellten Nucleotidsequenz, vorzugsweise der in SEQ ID Nr. 130 dargestellten Nucleotidsequenz, einer mutierten Nucleotidsequenz davon oder einer komplementären Nucleotidsequenz davon oder mit einem Nucleinsäuremolekül, das eine der in SEQ ID Nr. 95, 96, 97 oder 130 dargestellten Nucleotidsequenzen, vorzugsweise die in SEQ ID Nr. 130 dargestellte Nucleotidsequenz, eine mutierte Nucleotidsequenz davon oder eine komplementäre Nucleotidsequenz davon umfasst, zeigt erfindungsgemäß den HPV66-Genotyp an.

Erfindungsgemäß ist darüber hinaus vorgesehen, dass eine Hybridisierung mit einem Oligonucleotid mit der in SEQ ID Nr. 98, 99, 100, 101 oder 102 dargestellten Nucleotidsequenz, einer mutierten Nucleotidsequenz davon oder einer komplementären Nucleotidsequenz davon oder mit einem Nucleinsäuremolekül, das eine der in SEQ ID Nr. 98, 99, 100, 101 oder 102 dargestellten Nucleotidsequenzen, eine mutierte Nucleotidsequenz davon oder eine komplementäre Nucleotidsequenz davon umfasst, den HPV67-Genotyp anzeigt.

Eine Hybridisierung mit einem Oligonucleotid mit der in SEQ ID Nr. 103, 104, 105, 131 oder 132 dargestellten Nucleotidsequenz, vorzugsweise der in SEQ ID Nr. 131 oder 132 dargestellten Nucleotidsequenz,einer mutierten Nucleotidsequenz davon oder einer komplementären Nucleotidsequenz davon oder mit einem Nucleinsäuremolekül, das eine der in SEQ ID Nr. 103, 104, 105, 131 oder 132 dargestellten Nucleotidsequenzen, vorzugsweise die in SEQ ID Nr. 131 oder 132 dargestellte Nucleotidsequenz, eine mutierte Nucleotidsequenz davon oder eine komplementäre Nucleotidsequenz davon umfasst, zeigt erfindungsgemäß den HPV68-Genotyp an.

In einer weiteren Ausführungsform der Erfindung ist vorgesehen, dass eine Hybridisierung mit einem Oligonucleotid mit der in SEQ ID Nr. 106, 107, 108 oder 133 dargestellten Nucleotidsequenz, vorzugsweise der in SEQ ID Nr. 133 dargestellten Nucleotidsequenz, einer mutierten Nucleotidsequenz davon oder einer komplementären Nucleotidsequenz davon oder mit einem Nucleinsäuremolekül, das eine der in SEQ ID Nr. 106, 107, 108 oder 133 dargestellten Nucleotidsequenzen, vorzugsweise die in SEQ ID Nr. 133 dargestellte Nucleotidsequenz, eine mutierte Nucleotidsequenz davon oder eine komplementäre Nucleotidsequenz davon umfasst, den HPV70-Genotyp anzeigt.

Eine Hybridisierung mit einem Oligonucleotid mit der in SEQ ID Nr. 109, 110, 111, 112 oder 134 dargestellten Nucleotidsequenz, vorzugsweise der in SEQ ID Nr. 134 dargestellten Nucleotidsequenz, einer mutierten Nucleotidsequenz davon oder einer komplementären Nucleotidsequenz davon oder mit einem Nucleinsäuremolekül, das eine der in SEQ ID Nr. 109, 110, 111, 112 oder 134 dargestellten Nucleotidsequenzen, vorzugsweise die in SEQ ID Nr. 134 dargestellte Nucleotidsequenz, eine mutierte Nucleotidsequenz davon oder eine komplementäre Nucleotidsequenz davon umfasst, zeigt erfindungsgemäß den HPV73-Genotyp an.

Eine Hybridisierung mit einem Oligonucleotid mit der in SEQ ID Nr. 113, 114, 115, 116 oder 135 dargestellten Nucleotidsequenz, vorzugsweise der in SEQ ID Nr. 135 dargestellten Nucleotidsequenz, einer mutierten Nucleotidsequenz davon oder einer komplementären Nucleotidsequenz davon oder mit einem Nucleinsäuremolekül, das eine der in SEQ ID Nr. 113, 114, 115, 116 oder 135 dargestellten Nucleotidsequenzen, vorzugsweise die in SEQ ID Nr. 135 dargestellte Nucleotidsequenz, eine mutierte Nucleotidsequenz davon oder eine komplementäre Nucleotidsequenz davon umfasst, zeigt erfindungsgemäß den HPV82-Genotyp an.

Das als Sonde eingesetzte Oligonucleotid oder Nucleinsäuremolekül kann als DNA-, RNA-, PNA- oder LNA-Molekül oder Mischform davon vorliegen.

Die erfindungsgemäßen Verfahren zur Amplifikation von Nucleinsäurebereichen humaner Papillomaviren und zum Nachweis und/oder zur Bestimmung genitaler HPV-Genotypen können insbesondere zur Diagnose und/oder zur Früherkennung von Erkrankungen, die von genitalen humanen Papillomaviren verursacht werden, eingesetzt werden. Die erfindungsgemäßen Verfahren können in bevorzugter Ausführungsform als Teil eines Verfahrens zur Diagnose und/oder Früherkennung von Krankheiten, insbesondere Krebserkrankungen, beispielsweise Gebärmutterhalskrebs, eingesetzt werden. Die erfindungsgemäßen Verfahren können ebenfalls im Rahmen einer Früherkennungs-Untersuchung von Krebserkrankungen eingesetzt werden. Die erfindungsgemäße Verfahren führen zu gesicherten positiven Befunden hinsichtlich einer HPV-Infektion und können somit als Ausgangspunkt für eine zielgerichtete Therapie dienen.

Die vorliegende Erfindung löst das ihr zugrunde liegende technische Problem auch durch die Bereitstellung eines Nucleotid-Arrays zum Nachweis und/oder zur Bestimmung des Genotyps eines in einer biologischen Probe enthaltenen humanen Papillomavirus, insbesondere unter Verwendung des erfindungsgemäßen Verfahrens zum Nachweis und/oder zur Bestimmung des Genotyps von HPV-Viren, umfassend einen festen Träger mit einer Oberfläche und mindestens ein an die Träger-Oberfläche gebundenes erstes Oligonucleotid oder Nucleinsäuremolekül, das zum Nachweis und/oder zur Bestimmung eines genitalen HPV-Genotyps geeignet ist, ausgewählt aus der Gruppe bestehend aus:
a) HPV-Genotyp-spezifischen Oligonucleotiden mit den in SEQ ID Nr. 8 bis 135 dargestellten Nucleotidsequenzen, vorzugsweise den in SEQ ID Nr. 19, 32, 41, 44, 48, 82 und 117 bis 135 dargestellten Nucleotidsequenzen,
b) Oligonucleotiden, die eine gegenüber einem der Oligonucleotide nach a) mutierte Nucleotidsequenz aufweisen,
c) Oligonucleotiden, die eine Nucleotidsequenz aufweisen, die gegenüber der eines Oligonucleotids nach a) oder b) komplementär ist,
d) Nucleinsäuremolekülen, die einen Bereich umfassen, der eine der in SEQ ID Nr. 8 bis 135 dargestellten Nucleotidsequenzen, vorzugsweise eine der in SEQ ID Nr. 19, 32, 41, 44, 48, 82 und 117 bis 135 dargestellten Nucleotidsequenzen, eine mutierte Nucleotidsequenz davon oder eine komplementäre Nucleotidsequenz davon aufweist, und
e) Gemischen der Oligonucleotide nach a) bis c) und/oder der Nucleinsäuremoleküle nach d).

Im Zusammenhang mit der vorliegenden Erfindung wird unter einem "Nucleotid-Array" oder einem "Nucleotid-Chip" eine Vorrichtung verstanden, die eine Vielzahl verschiedener Nucleinsäuremoleküle oder Nucleotidsequenzen, beispielsweise DNA-Moleküle, RNA-Moleküle, PNA-Moleküle, LNA-Moleküle und/oder Mischformen davon, in immobilisierter Form enthält und mit deren Hilfe mittels Nucleinsäure-Hybridisierung eine kleine Menge einer komplementären Nucleinsäure in einer kleinen Probenflüssigkeit nachgewiesen werden kann. Unter Verwendung der erfindungsgemäßen Nucleotid-Arrays ist einerseits der einfache Nachweis von humanen Papillomaviren in einer biologischen Probe möglich, andererseits kann aber auch eine Unterscheidung zwischen genitalen HPV-Genotypen, insbesondere eine Unterscheidung zwischen Hoch-Risiko- und Niedrig-Risiko-Genotypen sowie eine Typisierung individueller genitaler HPV-Genotypen vorgenommen werden.

Unter dem "Träger" des Nucleotid-Arrays wird im Zusammenhang mit der vorliegenden Erfindung eine Vorrichtung verstanden, die eine Trägerplatte mit einer Oberfläche umfasst, an der ein biologisch aktives Molekül, beispielsweise eine Nucleinsäure, immobilisiert oder fixiert werden kann. Der Träger des Nucleotid-Arrays wird also zur Herstellung eines Nucleotid-Arrays verwendet, indem biologisch aktive Moleküle, insbesondere Nucleinsäuren oder Teile davon an der Oberfläche des Trägers immobilisiert oder fixiert werden. Die biologisch aktiven Moleküle sind dabei in Form von Spots auf der Trägeroberfläche angeordnet.

Unter der "Trägerplatte" des Nucleotid-Array-Trägers wird ein dünnes und ebenes Element mit beispielsweise rechteckiger Form verstanden, das insbesondere aus einem Metall, einem Metalloxid, einem Kunststoff, einer Membran, Glas, Keramik, Gel etc. oder einem Hybrid beziehungsweise einer Kombination dieser Materialien besteht. Im Zusammenhang mit der vorliegenden Erfindung bedeutet dies, dass die Trägerplatte des Trägers beispielsweise vollständig aus einem der vorstehend als Beispiel genannten Materialien besteht oder diese im Wesentlichen enthält oder vollständig aus einer Kombination dieser Materialien besteht oder diese im Wesentlichen enthält. Die Trägerplatte oder ihre Oberfläche besteht dabei zumindest zu etwa 50 %, 60 %, vorzugsweise zu etwa 70 %, bevorzugt zu etwa 80 % und am bevorzugtesten zu etwa 100 % aus einem der vorstehend als Beispiel genannten Materialien oder einer Kombination solcher Materialien. In bevorzugter Ausführungsform besteht die Trägerplatte des erfindungsgemäßen Nucleotid-Arrays zu etwa 100 % aus Kunststoff.

Die Oberfläche des Trägers des erfindungsgemäßen Trägers umfasst vorzugsweise ein Material, das nicht glatt ist, sondern rau, und/oder das nicht impermeabel ist, sondern permeabel, oder besteht daraus oder enthält dieses. In einer bevorzugten Ausführungsform der Erfindung besteht die Oberfläche des Trägers aus einem anderen Material als die Trägerplatte. Beispielsweise kann die Oberfläche aus einem Material wie Nitrocellulose bestehen oder dieses enthalten, während der Träger selbst aus einem Kunststoff, Glas, Keramik oder einem Metall besteht. In einer weiteren bevorzugten Ausführungsform der Erfindung kann die Oberfläche des Trägers auch aus dem zur Herstellung der Trägerplatte verwendeten Material bestehen. Vorzugsweise weist dieses Material eine raue und/oder permeable Oberfläche auf.

In einer bevorzugten Ausführungsform der Erfindung ist der Träger des erfindungsgemäßen Nucleotid-Arrays plättchenförmig, beispielsweise in Form eines Objektträgers, oder plättchenförmig mit Vertiefungen, beispielsweise als Chamberslide oder als Mikrotiterplatte mit Abmessungen gemäß Empfehlung der SBS (Society of Biomolecular Screening) ausgebildet.

Erfindungsgemäß sind die ersten Oligonucleotide oder Nucleinsäuremoleküle, insbesondere die Oligonucleotide mit den in SEQ ID Nr. 8 bis 135 dargestellten Nucleotidsequenzen, vorzugsweise die Oligonucleotide mit den in SEQ ID Nr. 19, 32, 41, 44, 48, 82 und 117 bis 135 dargestellten Nucleotidsequenzen, auf der Oberfläche des Trägers innerhalb eines definierten Analysebereiches in Form von Spots angeordnet. Erfindungsgemäß ist ferner vorgesehen, dass die Oberfläche des Trägers neben dem Analysebereich mindestens einen Kontrollbereich aufweist. Der Analysebereich und der Kontrollbereich können dabei auf der Oberfläche des Trägers an jeder beliebigen, definierten Stelle angeordnet sein.

Der auf der Trägeroberfläche angeordnete Kontrollbereich umfasst erfindungsgemäß eine Kontrolle zur Orientierung des Trägers, eine Amplifikations-Kontrolle, eine Hybridisierungs-Kontrolle, eine Proben-Kontrolle und/oder eine Print-Kontrolle.

In einer Ausführungsform der Erfindung ist vorgesehen, dass die Kontrolle zur Orientierung des Trägers mindestens ein zweites Oligonucleotid oder Nucleinsäuremolekül umfasst. Das zweite Oligonucleotid oder Nucleinsäuremolekül ist vorzugsweise markiert, vorzugsweise fluoreszenzmarkiert. Das zweite Oligonucleotid oder Nucleinsäuremolekül wird vorzugsweise in mindestens drei Spots so auf der Träger-Oberfläche angeordnet, dass eine Orientierung des Nucleotid-Arrays möglich ist.

In einer weiteren Ausführungsform der Erfindung ist vorgesehen, dass die Amplifikationskontrolle mindestens ein drittes Oligonucleotid oder Nucleinsäuremolekül umfasst. Vorzugsweise ist das dritte Oligonucleotid oder Nucleinsäuremolekül als Sonde zum Nachweis eines Amplifikationsproduktes geeignet, wobei das Amplifikationsprodukt mittels eines Amplifikationsverfahrens unter Verwendung einer Kontroll-Nucleinsäure als Matrize und eines erfindungsgemäßen Primer-Paares, d.h. eines Oligonucleotides mit einer der in SEQ ID Nr. 1 bis 6 dargestellten Nucleotidsequenzen als Forward-Primer und eines Oligonucleotids mit der in SEQ ID Nr. 7 dargestellten Nucleotidsequenz als Reverse-Primer, erhältlich ist. In besonders bevorzugter Ausführungsform ist vorgesehen, dass die Kontroll-Nucleinsäure vorzugsweise eine Länge und einen GC-Gehalt aufweist, der der Länge und dem GC-Gehalt des Amplifikationsproduktes entspricht, das mittels des erfindungsgemäßen Amplifikationsverfahrens unter

Verwendung des Nucleinsäurebereiches, insbesondere des E1-Gens, eines genitalen humanen Papillomavirus als Matrize, eines Oligonucleotids mit einer der in SEQ ID Nr. 1 bis 6 dargestellten Nucleotidsequenzen als Forward-Primer und eines Oligonucleotids mit der in SEQ ID Nr. 7 dargestellten Nucleotidsequenz als Reverse-Primer erhältlich ist.

In einer weiteren bevorzugten Ausführungsform der Erfindung ist vorgesehen, dass die Hybridisierungskontrolle mindestens ein viertes Oligonucleotid oder Nucleinsäuremolekül umfasst. Vorzugsweise sind mindestens zwei bis zehn Spots des vierten Oligonucleotids oder Nucleinsäuremoleküls auf der Träger-Oberfläche angeordnet. Erfindungsgemäß ist vorgesehen, dass die einzelnen Spots der Hybridisierungskontrolle unterschiedliche definierte Mengen des vierten Oligonucleotids oder Nucleinsäuremoleküls aufweisen. Vorzugsweise umfasst die Hybridisierungskontrolle Spots mit einer Verdünnungsreihe des vierten Oligonucleotids oder Nucleinsäuremoleküls.

In einer weiteren bevorzugten Ausführungsform der Erfindung ist vorgesehen, dass die Proben-Kontrolle mindestens ein fünftes Oligonucleotid oder Nucleinsäuremolekül umfasst. Das fünfte Oligonucleotid oder Nucleinsäuremolekül ist vorzugsweise als Sonde zum Nachweis eines in allen Zellen eines menschlichen Organismus vorkommenden Gens geeignet. In bevorzugter Ausführungsform handelt es sich bei dem unter Verwendung des fünften Oligonucleotids oder Nucleinsäuremoleküls nachzuweisenden Gen um das humane ADAT1- (t-RNA-spezifische Adenosindeaminase. 1; Gene Bank Accession: NM_012091)-Gen.

In einer weiteren bevorzugten Ausführungsform der Erfindung ist vorgesehen, dass die Print-Kontrolle mindestens ein sechstes Oligonucleotid oder Nucleinsäuremolekül umfasst. Das sechste Oligonucleotid oder Nucleinsäuremolekül kann in Form separater Spots auf der Träger-Oberfläche angeordnet sein. Besonders bevorzugt ist das als Print-Kontrolle eingesetzte sechste Oligonucleotid oder Nucleinsäuremolekül jedoch in allen auf dem Nucleotid-Array-Träger angeordneten Spots mit Ausnahme der Spots des als Hybridisierungskontrolle dienenden vierten Oligonucleotids/Nucleinsäuremoleküls enthalten. Das heißt, in dieser Ausgestaltung umfasst jeder Spot des zum HPV-Genotyp-Nachweis dienenden ersten Oligonucleotids/Nucleinsäuremoleküls, jeder Spot des als Kontrolle zur Träger-Orientierung dienenden zweiten Oligonucleotids/Nucleinsäuremoleküls, jeder Spot des als Amplifikationskontrolle dienenden dritten Oligonucleotids/Nucleinsäuremoleküls und jeder Spot des als Proben-Kontrolle dienenden fünften Oligonucleotids/Nucleinsäuremoleküls auch das als Print-Kontrolle dienende sechste Oligonucleotid/Nucleinsäuremolekül. Vorzugsweise wird das sechste Oligonucleotid oder Nucleinsäuremolekül gemeinsam mit dem ersten, zweiten, dritten beziehungsweise fünften Oligonucleotid oder Nucleinsäuremolekül in einem Print-Schritt als Spot auf den Träger des Nucleotid-Arrays aufgebracht.

Die auf dem Nucleotid-Array fixierten Oligonucleotide oder Nucleinsäuremoleküle können erfindungsgemäß sowohl als DNA-Moleküle, RNA-Moleküle, PNA-Moleküle, LNA-Moleküle oder Mischformen davon ausgebildet sein.

In einer weiteren Ausführungsform der Erfindung ist vorgesehen, dass die auf dem Träger des Nucleotid-Arrays aufgebrachten ersten, dritten, vierten, fünften und sechsten Oligonucleotide oder Nucleinsäuremoleküle keine Markierung aufweisen, während das als Orientierungskontrolle dienende zweite Oligonucleotid oder Nucleinsäuremolekül eine Markierung, vorzugsweise eine Fluoreszenz-Markierung aufweist.

In einer weiteren bevorzugten Ausführungsform der Erfindung ist vorgesehen, dass der Nucleotid-Array einen Dot-Code aufweist, anhand dessen es möglich ist, den erfindungsgemäßen Nucleotide-Array eindeutig zu identifizieren und von anderen Nucleotid-Chips, die beispielsweise die gleiche Form und/oder die gleiche oder eine ähnliche Anordnung von Spots wie der erfindungsgemäße NucleotidArray aufweisen, jedoch für andere Zwecke eingesetzt werden und daher andere fixierte Sonden aufweisen, zu unterscheiden. Der erfindungsgemäße Dot-Code sichert darüber hinaus auch, dass bei der Software-gestützten Auswertung der unter Verwendung des erfindungsgemäßen Nucleotid-Arrays erhaltenen Ergebnisse die korrekte Software zur Analyse eingesetzt wird. Da die relevanten Informationen bezüglich des Nucleotid-Arrays direkt auf dem Nucleotid-Chip platziert werden, ist eine zusätzliche Markierung des Chips nicht mehr erforderlich. Der Chip kann dennoch eine zusätzliche Markierung aufweisen. Falls diese Markierung während der Verwendung des Nucleotid-Arrays verloren geht, sichert der erfindungsgemäße Dot-Code die eindeutige Identifizierbarkeit des Chips. Erfindungsgemäß ist vorgesehen, dass der Dot-Code während der Chip-Herstellung auf diesen aufgebracht wird. Der erfindungsgemäße Dot-Code kann selbstverständlich nicht nur für den erfindungsgemäßen Nucleotid-Array, der zum Nachweis und/oder zur Bestimmung des Genotyps eines humanen Papillomavirus dient, eingesetzt werden, sondern auch für andere Nucleotid-Chips, die beispielsweise zur Bestimmung oder zum Nachweis eines spezifischen Gens oder Genproduktes, zum Nachweis spezifischer Bakterien etc. dienen.

Erfindungsgemäß ist vorgesehen, dass der erfindungsgemäße Dot-Code mindestens Informationen bezüglich des vorgesehenen Anwendungstyps eines Nucleotid-Arrays, beispielsweise Nachweis und/oder Bestimmung des Genotyps humaner Papillomaviren, der Batch-Nummer und der Chip-Nummer enthält. Selbstverständlich kann der erfindungsgemäße Dot-Code noch weitere Informationen enthalten.

Erfindungsgemäß ist vorgesehen, dass der Anwendungstyp mittels einer unikalen Nummer gekennzeichnet wird. Beispielsweise kann der erfindungsgemäße Nucleotid-Array, der zum Nachweis und/oder Bestimmung des Genotyps humaner Papillomaviren eingesetzt wird, durch das Zeichen "#1" gekennzeichnet wird. Ein Nucleotid-Chip, der zur Bestimmung und/oder zum Nachweis einer spezifischen Bakterien-Art eingesetzt werden soll, kann beispielsweise durch das Zeichen "#2" gekennzeichnet werden. Dieses Zeichen kann mittels eines beliebigen numerischen Codes codiert werden, beispielsweise mittels eines binären Systems, eines Dezimalsystems, etc.. Bei dem 1-of-n-Codierungsverfahren gibt es n Positionen, wobei n eine ganze Zahl ist und eine der n Positionen positiv ist. Bei dem n-ary-Codierungsverfahren gibt es m Positionen, die n Intensitätsstufen aufweisen können. Bei binärer Codierung bedeutet dies, dass es zwei verschiedene Intensitätsstufen gibt, so dass es m Positionen gibt, die entweder "1" (positives Signal) oder "0" (negatives Signal) sein können. Bei Dezimalcodierung können 2 Positionen 100 verschiedene Nummern codieren, wobei jede Position 10 verschiedene Intensitätsstufen aufweisen kann.

Die Codierung kann erfindungsgemäß auch einen zweidimensionalen Array von Spots umfassen, wobei eine binäre Codierung in zwei Dimensionen entsprechend 2^(n*m) erreicht wird, wobei n und m die Dimensionen des Arrays darstellen. Das Lesen (read out) des Dot-Codes kann erfindungsgemäß binär oder analog erfolgen. Bei einem binären Readout wird einem bestimmten Spot entweder ein "Vorhanden" oder ein "Nicht-Vorhanden" zugewiesen. Im Gegensatz zu dem binären readout wird beim analogen Readout jedem Spot ein numerischer Wert zugewiesen. Dieser numerische Wert enthält selbst Informationen und kann beispielsweise die Größe des Spots oder die Signalintensität des Spots betreffen.

Zusätzliche Informationen auf dem Nucleotid-Array können darüber hinaus durch die Verwendung und/oder Kombination unterschiedlicher Mengen von zwei oder mehr Farben auf dem Nucleotid-Array erhalten werden, wobei die zwei oder mehr Farben in einem oder mehr Spots enthalten sein können.

Erfindungsgemäß ist bei Verwendung des erfindungsgemäßen Nucleotid-Arrays zum Nachweis und/oder zur Bestimmung des Genotyps eines humanen Papillomavirus, wobei insbesondere das Amplifikationsprodukt eingesetzt wird, das unter Verwendung des erfindungsgemäßen Verfahrens zur Amplifikation eines Bereiches eines genitalen humanen Papillomavirus erhalten wird, vorgesehen, dass das zu analysierende Amplifikationsprodukt in markierter Form eingesetzt wird. Das eingesetzte Amplifikationsprodukt kann bereits während der Amplifikationsreaktion mit einer Markierung versehen werden, kann aber auch erst nach Beendigung der Amplifikationsreaktion, also vor Analyse mittels des erfindungsgemäßen Nucleotid-Mikroarrays, markiert werden. Eine Markierung des Amplifikationsproduktes während der Amplifikationsreaktion kann beispielsweise unter Verwendung von markierten Primern und/oder markierten Nucleotiden erfolgen. Erfindungsgemäß kann das Amplifikationsprodukt zum Beispiel unter Verwendung von Fluoreszenz-Farbstoffen wie Cy3 oder Cy5, Biotin, Haptenen, Antigenen, chemischen Gruppen, beispielsweise durch Verwendung aminoallyl-markierter Nucleotide oder durch Anbringen eines Markers mittels einer Succinimid-Reaktion mit dem aminoallyl-markierten Nucleotid, radioaktiven Stoffen, enzymatischen Markern etc. markiert werden. Die Detektion des markierten Amplifikationsproduktes nach Hybridisierung des Amplifikationsproduktes mit einem komplementären Oligonucleotid oder Nucleinsäuremolekül an der Trägeroberfläche des erfindungsgemäßen Nucleotid-Arrays kann beispielsweise unter Verwendung von Fluoreszenz-Verfahren, Chemolumineszenz-Verfahren, radiometrischen Verfahren, Densitometrie-Verfahren, Photometrie-Verfahren, Fällungsreaktionen, enzymatischen Reaktionen einschließlich enzymatischen Verstärkungsreaktionen, SPR, Ellipsometrie-Verfahren, Messung des Brechungsindex, Messung der Reflektivität und ähnlichen Verfahren erfolgen.

In einer bevorzugten Ausführungsform können die OrientierungsKontrolle, die Hybridisierungs-Kontrolle und die Print-Kontrolle mittels Cy3 im Cy3-Kanal detektiert werden, während die Amplifikations-Kontrolle, die Proben-Kontrolle und die nachzuweisenden HPV-Genotypen mittels Cy5 im Cy5-Kanal detektiert werden.

Die vorliegende Erfindung betrifft ebenfalls einen Kit zum Nachweis und/oder zur Bestimmung genitaler HPV-Genotypen, umfassend mindestens einen ersten Behälter mit mindestens einem Primer zur Amplifikation von Nucleinsäurebereichen eines genitalen humanen Papillomavirus, insbesondere Bereichen des HPV-Gens E1, und mindestens einen zweiten Behälter mit mindestens einer Sonde zum Nachweis und/oder Bestimmung genitaler HPV-Genotypen, insbesondere zum Nachweis eines amplifizierten Bereiches des HPV-Gens E1. Der zur Amplifikation verwendete Primer ist erfindungsgemäß ausgewählt aus Oligonucleotiden mit einer der in SEQ ID Nr. 1 bis 7 dargestellten Nucleotidsequenzen, Oligonucleotiden mit einer Nucleotidsequenz, die gegenüber einer der in SEQ ID Nr. 1 bis 7 dargestellten Nucleotidsequenzen mutiert ist, und erfindungsgemäßen Primer-Paaren. Die Sonde zum Nachweis und/oder Bestimmung genitaler HPV-Genotypen ist erfindungsgemäß ausgewählt aus Oligonucleotiden mit einer der in SEQ ID Nr. 8 bis 135 dargestellten Nucleotidsequenzen, vorzugsweise mit einer der in SEQ ID Nr. 19, 32, 41, 44, 48, 82 und 117 bis 135 dargestellten Nucleotidsequenzen, Oligonucleotiden mit einer Nucleotidsequenz, die gegenüber einer der in SEQ ID Nr. 8 bis 135 dargestellten Nucleotidsequenzen, vorzugsweise einer der in SEQ ID Nr. 19, 32, 41, 44, 48, 82 und 117 bis 135 dargestellten Nucleotidsequenzen, mutiert ist, Oligonucleotiden mit einer Nucleotidsequenz, die zu einer der in SEQ ID Nr. 8 bis 135 dargestellten Nucleotidsequenzen, vorzugsweise einer der in SEQ ID Nr. 19, 32, 41, 44, 48, 82 und 117 bis 135 dargestellten Nucleotidsequenzen, oder einer mutierten Nucleotidsequenz davon komplementär ist, oder Nucleinsäuremolekülen, die eine der in SEQ ID Nr. 8 bis 135 dargestellten Nucleotidsequenzen, vorzugsweise eine der in SEQ ID Nr. 19, 32, 41, 44, 48, 82 und 117 bis 135 dargestellten Nucleotidsequenzen, eine mutierte Nucleotidsequenz davon oder eine komplementäre Nucleotidsequenz davon umfassen.

In bevorzugter Ausführungsform der Erfindung umfasst der Kit mindestens 29 zweite Behälter mit mindestens 29 verschiedenen Sonden zum Nachweis und/oder zur Bestimmung der HPV6-, insbesondere HPV6b-, HPV11-, HPV16-, HPV18-, HPV26-, HPV31-, HPV33-, HPV34-, HPV35-, insbesondere HPV35h-, HPV39-, HPV40-; HPV42-, HPV43-, HPV44-, HPV45-, HPV51-, HPV52, HPV53-, HPV54-, HPV56-, HPV58-, HPV59-, HPV61-, HPV66-, HPV67-, HPV68-, HPV70-, HPV73- und HPV82-Genotypen, wobei jeder Behälter mindestens eine Sonde enthält und wobei alle in einem Behälter enthaltenen Sonden nur einen spezifischen HPV-Genotyp nachweisen können. Besonders bevorzugt umfasst der Kit mindestens 24 zweite Behälter mit mindestens 24 verschiedenen Sonden zum Nachweis und/oder zur Bestimmung der HPV6-, insbesondere HPV6b-, HPV11-, HPV16-, HPV18-, HPV31-, HPV33-, HPV35-, insbesondere HPV35h-, HPV39-, HPV40-, HPV42-, HPV43-, HPV44-, HPV45-, HPV51-, HPV52, HPV53-, HPV56-, HPV58-, HPV59-, HPV66-, HPV68-, HPV70-, HPV73- und HPV82-Genotypen.

Wenn beispielsweise die in einem Behälter enthaltenen Sonden zum Nachweis des HPV6-Genotyps, insbesondere HPV6b-Genotyps, verwendet werden sollen, kann der Behälter ein Oligonucleotid mit der in SEQ ID Nr. 8, 9 bzw. 117 dargestellten Nucleotidsequenz, vorzugsweise der in SEQ ID Nr. 117 dargestellten Nucleotidsequenz, ein Oligonucleotid mit einer Nucleotidsequenz, die gegenüber der Nucleotidsequenz von SEQ ID Nr. 8, 9 bzw. 117 mutiert ist, ein Oligonucleotid mit einer Nucleotidsequenz, die gegenüber SEQ ID Nr. 8, 9 bzw. 117 oder einer mutierten Sequenz davon komplementär ist, ein Nucleinsäuremolekül, das die Sequenz von SEQ ID Nr. 8, 9 bzw. 117, vorzugsweise die Sequenz von SEQ ID Nr. 117, eine mutierte Sequenz davon oder eine komplementäre Sequenz davon aufweist, oder ein Gemisch dieser Oligonucleotide und/oder Nucleinsäuremoleküle enthalten. Vorzugsweise enthält jeder Behälter jedoch kein Gemisch, sondern nur eine Oligonucleotid- oder Nucleinsäuremolekül-Spezies.

Die vorliegende Erfindung betrifft ebenfalls einen Kit zum Nachweis und/oder zur Bestimmung genitaler HPV-Genotypen, umfassend mindestens einen ersten Behälter mit mindestens einem Primer zur Amplifikation von Nucleinsäurebereichen eines genitalen humanen Papillomavirus, insbesondere Bereichen des HPV-Gens E1, und einen erfindungsgemäßen Nucleotid-Array zum Nachweis und/oder Bestimmung genitaler HPV-Genotypen, insbesondere zum Nachweis eines amplifizierten Bereiches des HPV-Gens E1. Der zur Amplifikation verwendete Primer ist erfindungsgemäß ausgewählt aus Oligonucleotiden mit einer der in SEQ ID Nr. 1 bis 7 dargestellten Nucleotidsequenzen, Oligonucleotiden mit einer Nucleotidsequenz, die gegenüber einer der in SEQ ID Nr. 1 bis 7 dargestellten Nucleotidsequenzen mutiert ist, und den erfindungsgemäßen Primer-Paaren. Der Nucleotid-Array des erfindungsgemäßen Kits umfasst vorzugsweise Oligonucleotide mit einer der in SEQ ID Nr. 8 bis 135 dargestellten Nucleotidsequenzen, besonders bevorzugt einer der in SEQ ID Nr. 19, 32, 41, 44, 48, 82 und 117 bis 135 dargestellten Nucleotidsequenzen, Oligonucleotiden mit einer Nucleotidsequenz, die gegenüber einer der in SEQ ID Nr. 8 bis 135 dargestellten Nucleotidsequenzen, insbesondere einer der in SEQ ID Nr. 19, 32, 41, 44, 48, 82 und 117 bis 135 dargestellten Nucleotidsequenzen, mutiert ist,

Oligonucleotiden mit einer Nucleotidsequenz, die zu einer der in SEQ ID Nr. 8 bis 135 dargestellten Nucleotidsequenzen, vorzugsweise einer der in SEQ ID Nr. 19, 32, 41, 44, 48, 82 und 117 bis 135 dargestellten Nucleotidsequenzen, oder einer mutierten Nucleotidsequenz davon komplementär ist, und/oder Nucleinsäuremoleküle, die eine der in SEQ ID Nr. 8 bis 135 dargestellten Nucleotidsequenzen, vorzugsweise eine der in SEQ ID Nr. 19, 32, 41, 44, 48, 82 und 117 bis 135 dargestellten Nucleotidsequenzen, eine mutierte Nucleotidsequenz davon oder eine komplementäre Nucleotidsequenz davon umfassen.

In bevorzugter Ausführungsform der Erfindung umfasst der erfindungsgemäße Kit mindestens zwei erste Behälter, wobei ein Behälter davon äquimolare Mengen der Oligonucleotide mit den in SEQ ID Nr. 2 bis 6 dargestellten Nucleotidsequenzen enthält und der andere Behälter das Oligonucleotid mit der in SEQ ID Nr. 7 dargestellten Nucleotidsequenz enthält. In einer alternativen Ausführungsform umfasst der erfindungsgemäße Kit sechs erste Behälter, wobei fünf Behälter davon jeweils eines der Oligonucleotide mit den in SEQ ID Nr. 2 bis 6 dargestellten Nucleotidsequenzen enthalten und der sechste Behälter das Oligonucleotid mit der in SEQ ID Nr. 7 dargestellten Nucleotidsequenz enthält.

Der erfindungsgemäße Kit kann zusätzlich einen Behälter mit einer Kontroll-Nucleinsäure umfassen, die unter Verwendung eines Oligonucleotids mit einer der in SEQ ID Nr. 1 bis 6 dargestellten Nucleotidsequenzen als Forward-Primer und eines Oligonucleotids mit der in SEQ ID Nr. 7 dargestellten Nucleotidsequenz als Reverse-Primer amplifiziert werden kann.

Die vorliegende Erfindung betrifft selbstverständlich auch die Verwendung eines Oligonucleotids mit einer der in SEQ ID Nr. 8 bis 135 dargestellten Nucleotidsequenzen, vorzugsweise einer der in SEQ ID Nr. 19, 32, 41, 44, 48, 82 und 117 bis 135 dargestellten Nucleotidsequenzen, eines Oligonucleotids, dessen Nucleotidsequenz gegenüber einer der in SEQ ID Nr. 8 bis 135 dargestellten Nucleotidsequenzen, insbesondere einer der in SEQ ID Nr. 19, 32, 41, 44, 48, 82 und 117 bis 135 dargestellten Nucleotidsequenzen mutiert ist, eines Oligonucleotids, dessen Nucleotidsequenz zu einer der in SEQ ID Nr. 8 bis 135 dargestellten Nucleotidsequenzen, insbesondere einer der in SEQ ID Nr. 19, 32, 41, 44, 48, 82 und 117 bis 135 dargestellten Nucleotidsequenzen, oder einer mutierten Nucleotidsequenz davon komplementär ist, oder eines Nucleinsäuremoleküls, das eine der in SEQ ID Nr. 8 bis 135 dargestellten Nucleotidsequenzen, insbesondere eine der in SEQ ID Nr. 19, 32, 41, 44, 48, 82 und 117 bis 135 dargestellten Nucleotidsequenzen, eine mutierte Sequenz davon oder eine komplementäre Sequenz davon umfasst, zum Nachweis und/oder zur Bestimmung eines genitalen HPV-Genotyps, das heißt zur Typisierung eines Papillomavirus, dessen Vorhandensein in einer biologischen Probe vorzugsweise zuvor bereits nachgewiesen wurde.

Die vorliegende Erfindung betrifft ebenfalls die Verwendung eines Oligonucleotids mit einer der in SEQ ID Nr. 1 bis 7 dargestellten Nucleotidsequenzen und/oder eines Oligonucleotids, dessen Nucleotidsequenz gegenüber einer der in SEQ ID Nr. 1 bis 7 dargestellten Nucleotidsequenzen mutiert ist, oder eines erfindungsgemäßen Primer-Paares zur Amplifikation eines Nucleinsäure-Bereiches eines genitalen humanen Papilloma-Virus, wobei in bevorzugter Ausführungsform der zu amplifizierende Nucleinsäure-Bereich ein Bereich des HPV-Gens E1 ist.

Die vorliegende Erfindung betrifft auch die Verwendung eines Oligonucleotids mit einer der in SEQ ID Nr. 1 bis 135 dargestellten Nucleotidsequenzen, eines Oligonucleotids, dessen Nucleotidsequenz gegenüber einer der in SEQ ID Nr. 1 bis 135 dargestellten Nucleotidsequenzen mutiert ist, eines Oligonucleotides, das eine Nucleotidsequenz aufweist, die zu einer der in SEQ ID Nr. 8 bis 135 dargestellten Nucleotidsequenzen oder einer mutierten Sequenz davon komplementär ist, eines Nucleinsäuremoleküles, das eine der in SEQ ID Nr. 8 bis 135 dargestellten Nucleotidsequenzen, eine mutierte Sequenz davon oder eine komplementäre Sequenz davon umfasst, oder eines erfindungsgemäßen Primer-Paares zur Diagnose und/oder Früherkennung von Krankheiten, die von genitalen humanen Papillomaviren verursacht werden.

Die vorliegende Erfindung betrifft ebenfalls die Verwendung eines Oligonucleotids mit einer der in SEQ ID Nr. 1 bis 135 dargestellten Nucleotidsequenzen, insbesondere einer der in SEQ ID Nr. 1 bis 7, 19, 32, 41, 44, 48, 82 und 117 bis 135 dargestellten Nucleotidsequenzen, eines Oligonucleotids, dessen Nucleotidsequenz gegenüber einer der in SEQ ID Nr. 1 bis 135 dargestellten Nucleotidsequenzen, insbesondere einer der in SEQ ID Nr. 1 bis 7, 19, 32, 41, 44, 48, 82 und 117 bis 135 dargestellten Nucleotidsequenzen, mutiert ist, eines Oligonucleotides, das eine Nucleotidsequenz aufweist, die zu einer der in SEQ ID Nr. 8 bis 135 dargestellten Nucleotidsequenzen, insbesondere einer der in SEQ ID Nr. 1 bis 7, 19, 32, 41, 44, 48, 82 und 117 bis 135 dargestellten Nucleotidsequenzen, oder einer mutierten Sequenz davon komplementär ist, eines Nucleinsäuremoleküles, das eine der in SEQ ID Nr. 8 bis 135 dargestellten Nucleotidsequenzen, insbesondere eine der in SEQ ID Nr. 19, 32, 41, 44, 48, 82 und 117 bis 135 dargestellten Nucleotidsequenzen, eine mutierte Sequenz davon oder eine komplementäre Sequenz davon umfasst, oder eines erfindungsgemäßen Primer-Paares zur Herstellung eines Mittels zur Diagnose von Krankheiten, die von genitalen humanen Papillomaviren verursacht werden.

Bei dem herzustellenden Mittel kann es sich beispielsweise um einen erfindungsgemäßen Kit oder einen erfindungsgemäßen Nucleotid-Array handeln.

Die vorliegende Erfindung wird durch das folgende Sequenzprotokoll, die folgenden Figuren und die folgenden Beispiele näher erläutert.

Das Sequenzprotokoll ist Teil dieser Beschreibung und enthält die Sequenzen SEQ ID Nr. 1 bis 135.

SEQ ID Nr. 1 bis SEQ ID Nr. 6 zeigen die Sequenzen von Oligonucleotiden, die als Forward-Primer zur Amplifikation von Bereichen des HPV-Gens E1 geeignet sind. Die als Forward-Primer verwendeten Oligonucleotide mit den in SEQ ID Nr. 2 bis 6 dargestellten Nucleotidsequenzen werden im Folgenden auch als Loma 1, Loma 2, Loma 3, Loma 4 beziehungsweise Loma 5 bezeichnet.

SEQ ID Nr. 7 zeigt die Sequenz eines Oligonucleotids, das als Reverse-Primer zur Amplifikation von Bereichen des HPV-Gens E1 geeignet ist. Das als Reverse-Primer verwendete Oligonucleotid mit der in SEQ ID Nr. 7 dargestellten Nucleotidsequenz wird im Folgenden auch als Loma-rev bezeichnet.

SEQ ID Nr. 8, 9 und 117 zeigen die Sequenzen von Oligonucleotiden, die zum Nachweis und zur Bestimmung des HPV6-Genotyps, insbesondere HPV6b-Genotyps geeignet sind.

SEQ ID Nr. 10 bis SEQ ID Nr. 17 und SEQ ID Nr. 118 zeigen die Sequenzen von Oligonucleotiden, die zum Nachweis und zur Bestimmung des HPV11-Genotyps geeignet sind.

SEQ ID Nr. 18 bis SEQ ID Nr. 20 zeigen die Sequenzen von Oligonucleotiden, die zum Nachweis und zur Bestimmung des HPV16-Genotyps geeignet sind.

SEQ ID Nr. 21 bis SEQ ID Nr. 24 und SEQ ID Nr. 119 zeigen die Sequenzen von Oligonucleotiden, die zum Nachweis und zur Bestimmung des HPV18-Genotyps geeignet sind.

SEQ ID Nr. 25 bis SEQ ID Nr. 28 zeigen die Sequenzen von Oligonucleotiden, die zum Nachweis und zur Bestimmung des HPV26-Genotyps geeignet sind.

SEQ ID Nr. 29 bis SEQ ID Nr. 31 und SEQ ID Nr. 120 zeigen die Sequenzen von Oligonucleotiden, die zum Nachweis und zur Bestimmung des HPV31-Genotyps geeignet sind.

SEQ ID Nr. 32 bis SEQ ID Nr. 34 zeigen die Sequenzen von Oligonucleotiden, die zum Nachweis und zur Bestimmung des HPV33-Genotyps geeignet sind.

SEQ ID Nr. 35 bis SEQ ID Nr. 39 zeigen die Sequenzen von Oligonucleotiden, die zum Nachweis und zur Bestimmung des HPV34-Genotyps geeignet sind.

SEQ ID Nr. 40 und SEQ ID Nr. 41 zeigen die Sequenzen von Oligonucleotiden, die zum Nachweis und zur Bestimmung des HPV35-Genotyps, insbesondere HPV35h-Genotyps geeignet sind.

SEQ ID Nr. 42 bis SEQ ID Nr. 46 zeigen die Sequenzen von Oligonucleotiden, die zum Nachweis und zur Bestimmung des HPV39-Genotyps geeignet sind.

SEQ ID Nr. 47 bis SEQ ID Nr. 50 zeigen die Sequenzen von Oligonucleotiden, die zum Nachweis und zur Bestimmung des HPV40-Genotyps geeignet sind.

SEQ ID Nr. 51, SEQ ID Nr. 52 und SEQ ID Nr. 121 zeigen die Sequenzen von Oligonucleotiden, die zum Nachweis und zur Bestimmung des HPV42-Genotyps geeignet sind.

SEQ ID Nr. 122 zeigt die Sequenz eines Oligonucleotids, das zum Nachweis und zur Bestimmung des HPV43-Genotyps geeignet ist.

SEQ ID Nr. 53 bis SEQ ID Nr. 55 und SEQ ID Nr. 123 zeigen die Sequenzen von Oligonucleotiden, die zum Nachweis und zur Bestimmung des HPV44-Genotyps geeignet sind.

SEQ ID Nr. 56 bis SEQ ID Nr. 61 und SEQ ID Nr. 124 zeigen die Sequenzen von Oligonucleotiden, die zum Nachweis und zur Bestimmung des HPV45-Genotyps geeignet sind.

SEQ ID Nr. 62 bis SEQ ID Nr. 64 und SEQ ID Nr. 125 zeigen die Sequenzen von Oligonucleotiden, die zum Nachweis und zur Bestimmung des HPV51-Genotyps geeignet sind.

SEQ ID Nr. 65 bis SEQ ID Nr. 67 und SEQ ID Nr. 126 zeigen die Sequenzen von Oligonucleotiden, die zum Nachweis und zur Bestimmung des HPV52-Genotyps geeignet sind.

SEQ ID Nr. 68 bis SEQ ID Nr. 73 und SEQ ID Nr. 127 zeigen die Sequenzen von Oligonucleotiden, die zum Nachweis und zur Bestimmung des HPV53-Genotyps geeignet sind.

SEQ ID Nr. 74 bis SEQ ID Nr. 77 zeigen die Sequenzen von Oligonucleotiden, die zum Nachweis und zur Bestimmung des HPV54-Genotyps geeignet sind.

SEQ ID Nr. 78 bis SEQ ID Nr. 81 und SEQ ID Nr. 128 zeigen die Sequenzen von Oligonucleotiden, die zum Nachweis und zur Bestimmung des HPV56-Genotyps geeignet sind.

SEQ ID Nr. 82 bis SEQ ID Nr. 86 zeigen die Sequenzen von Oligonucleotiden, die zum Nachweis und zur Bestimmung des HPV58-Genotyps geeignet sind.

SEQ ID Nr. 87 und SEQ ID Nr. 129 zeigen die Sequenzen von Oligonucleotiden, die zum Nachweis und zur Bestimmung des HPV59-Genotyps geeignet sind.

SEQ ID Nr. 88 bis SEQ ID Nr. 94 zeigen die Sequenzen von Oligonucleotiden, die zum Nachweis und zur Bestimmung des HPV61-Genotyps geeignet sind.

SEQ ID Nr. 95 bis SEQ ID Nr. 97 und SEQ ID Nr. 130 zeigen die Sequenzen von Oligonucleotiden, die zum Nachweis und zur Bestimmung des HPV66-Genotyps geeignet sind.

SEQ ID Nr. 98 bis SEQ ID Nr. 102 zeigen die Sequenzen von Oligonucleotiden, die zum Nachweis und zur Bestimmung des HPV67-Genotyps geeignet sind.

SEQ ID Nr. 103 bis SEQ ID Nr. 105, SEQ ID Nr. 131 und SEQ ID Nr. 132 zeigen die Sequenzen von Oligonucleotiden, die zum Nachweis und zur Bestimmung des HPV68-Genotyps geeignet sind.

SEQ ID Nr. 106 bis SEQ ID Nr. 108 und SEQ ID Nr. 133 zeigen die Sequenzen von Oligonucleotiden, die zum Nachweis und zur Bestimmung des HPV70-Genotyps geeignet sind.

SEQ ID Nr. 109 bis SEQ ID Nr. 112 und SEQ ID Nr. 134 zeigen die Sequenzen von Oligonucleotiden, die zum Nachweis und zur Bestimmung des HPV73-Genotyps geeignet sind.

SEQ ID Nr. 113 bis SEQ ID Nr. 116 und SEQ ID Nr. 135 zeigen die Sequenzen von Oligonucleotiden, die zum Nachweis und zur Bestimmung des HPV82-Genotyps geeignet sind.

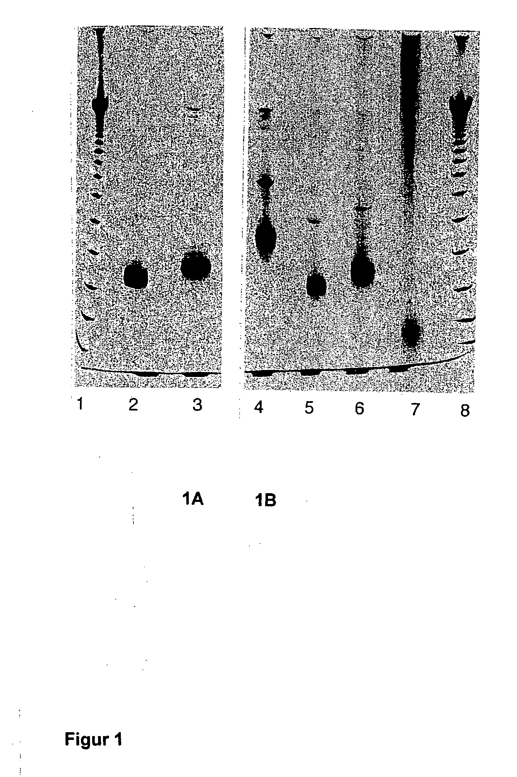

Figur 1 zeigt die Ergebnisse einer Acrylamid-Gelelektrophorese (nicht-denaturierend) von Amplifikationsprodukten, die unter Verwendung der erfindungsgemäßen Primer und der DNA verschiedener Papilloma-Viren als Matrize erhalten wurden. Als Forward-Primer wurde ein äquimolares Gemisch der Primer Loma 1, Loma 2, Loma 3, Loma 4 und Loma 5 (die den Oligonucleotiden mit den in SEQ ID Nr. 2 bis 6 dargestellten Nucleotidsequenzen entsprechen) eingesetzt. Als Reverse-Primer wurde der Primer Loma-rev (entspricht dem Oligonucleotid mit der in SEQ ID Nr. 7 dargestellten Nucleotidsequenz) eingesetzt. Als Matrize wurden Plasmide verwendet, die das gesamte HPV6-, HPV16-, HPV58-, HPV59- beziehungsweise HPV82-Genom enthielten. Als Negativ-Kontrolle wurde humane DNA verwendet.

Figur 1A: Nucleinsäure-Größenstandard (Laufspur 1), Amplifikationsprodukt unter Verwendung von HPV6-DNA (Laufspur 2), Amplifikationsprodukt unter Verwendung von HPV16-DNA (Laufspur 3),

Figur 1B: Amplifikationsprodukt unter Verwendung von HPV58-DNA (Laufspur 4), Amplifikationsprodukt unter Verwendung von HPV59-DNA (Laufspur 5), Amplifikationsprodukt unter Verwendung von HPV82-DNA (Laufspur 6), Amplifikationsreaktion unter Verwendung humaner-DNA (Laufspur 7; Negativ-Kontrolle), Nucleinsäure-Größenstandard (Laufspur 8).

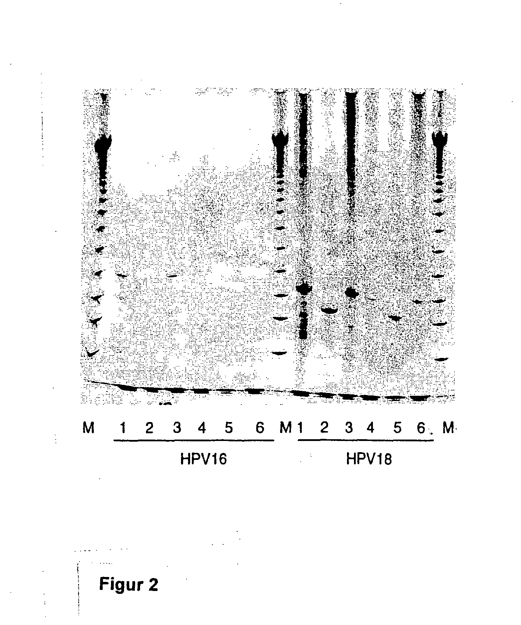

Figur 2 zeigt die Ergebnisse einer Acrylamid-Gelelektrophorese (nicht-denaturierend) von Amplifikationsprodukten, die unter Verwendung von HPV16- oder HPV18-DNA als Matrize erhalten wurden. Als Primer wurden die erfindungsgemäße Primer-Kombination Loma1-5/Loma-rev (Kombination 1), die Primer-Kombination Loma1-5/P2 (SEQ ID Nr. 2 aus DE 100 09 143 A1) (Kombination 2), die Primer-Kombination Loma1-5/P3 (SEQ ID Nr. 3 aus DE 100 09 143 A1) (Kombination 3), die Primer-Kombination P1 (SEQ ID Nr. 1 aus DE 100 09 143 A1)/Loma-rev (Kombination 4), die Primer-Kombination P1/P2 (Kombination 5) und die Primer-Kombination P1/P3 (Kombination 6) eingesetzt. M = DNA-Größenstandard (123 bp-Leiter), 1 = Primer-Kombination 1, 2 = Primer-Kombination 2, 3 = Primer-Kombination 3, 4 = Primer-Kombination 4, 5 = Primer-Kombination 5, 6 = Primer-Kombination 6.

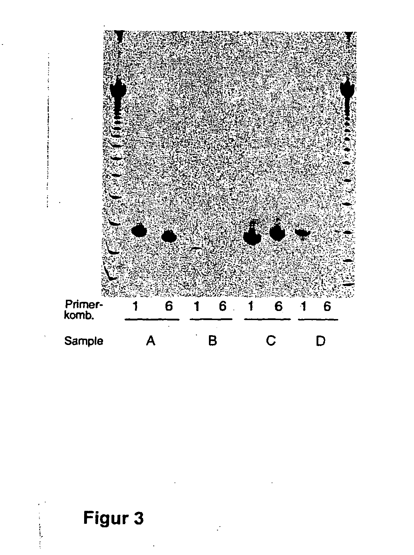

Figur 3 zeigt die Ergebnisse einer Acrylamid-Gelelektrophorese (nicht-denaturierend) von Amplifikationsprodukten, die unter Verwendung von Patientenproben erhalten wurden. Als Primer wurden die Primer-Kombinationen 1 (Lomal-5/Loma-rev) und 6 (P1/P3) eingesetzt. A = Patientenprobe, die mittels in situ-Hybridisierung als HPV16-positiv eingestuft wurde, B = Patientenprobe, die mittels in situ-Hybridisierung als HPV73-positiv eingestuft wurde, C = Patientenprobe, die mittels in situ-Hybridisierung als negativ eingestuft wurde, D = Patientenprobe, die mittels in situ-Hybridisierung als HPV33-positiv eingestuft wurde. In den Laufspuren links außen und rechts außen ist jeweils ein DNA-Größenstandard (123 bp-Leiter) aufgetragen.

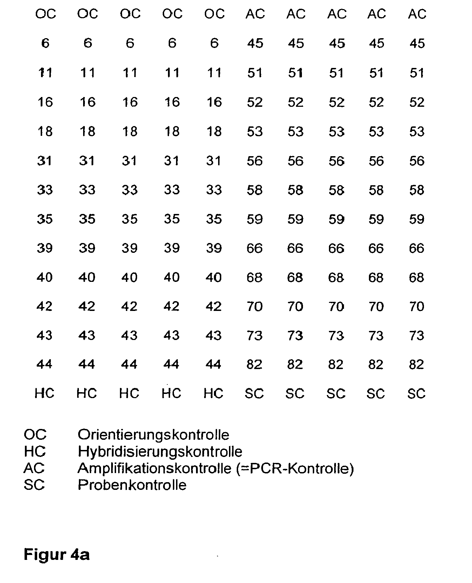

Figur 4a zeigt das Design eines erfindungsgemäßen Nucleotid-Arrays oder Nucleotid-Chips, wobei auf der Oberfläche des Microarray-Trägers eine Kontrolle zur Orientierung des Microarray-Trägers (OC), eine Hybridisierungskontrolle (HC), eine PCR-Kontrolle (AC), eine Proben-Kontrolle (Sample-Kontrolle, SC) sowie Sonden zur Typisierung von HPV-Genotypen angeordnet sind. Der Microarray weist darüber hinaus Spots zur Chip-Codierung auf. Die Hybridisierungskontrolle und PCR-Kontrolle können jeweils als Verdünnungsreihen der entsprechenden Oligonucleotide ausgeführt sein. Zahlen geben die Bezeichnungen des HPV-Typen an, für die die jeweilige Sonde spezifisch ist. Eine Printkontrolle ist zusätzlich auf allen Spots außer der Orientierungskontrolle und der Hybridisierungskontrolle angebracht.

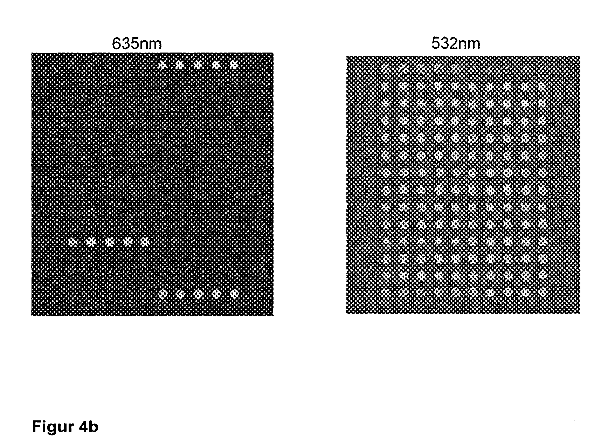

Figur 4b zeigt eine konkret durchgeführte Hybridisierung für HPV42. OC, HC und Printkontrollen werden durch Fluoreszenzlicht nach Anregung mit Licht der Wellenlänge 532nm detektiert. AC, SC und die HPC-spezifischen Signale durch Fluoreszenzlicht nach Anregung mit Licht der Wellenlänge 635nm.

### Beispiel 1

### Amplifikation von HPV-DNA unter Verwendung der erfindungsgemäßen Primer Loma/Loma-rev

Zur Amplifikation wurden Plasmide mit den Genomen von HPV6, HPV16, HPV58, HPV59 und HPV82 als Matrize (Template) verwendet. Diese Plasmide enthalten das gesamte Genom des entsprechenden HPV-Typs. Als Negativ-Kontrolle wurde humane DNA verwendet. Tabelle 1 zeigt die Zusammensetzung des Reaktionsansatzes und die Konzentration der eingesetzten Primer.

**Tabelle 1**

| Lösung | µl | Endkonzentration |
|---|---|---|
| H₂O | 12,6 | |
| 10 x PCR-Buffer (für AmpliTaq Gold; ohne MgCl₂ | 2 | 1x |
| 25 mM MgCl₂ | 2 | 2,5 mM |
| 25 mM dNTP (f.c.: 0,25 mM) | 0,2 | 0,25 mM |
| Loma-Mix mit 4 pMol/µl von jedem der Primer Loma1-Loma5 | 1 | 0,2 pMol/µl von jedem der 5 Loma Primer |
| 20 pMol/µl Loma-rev Cy5-markiert | 1 | 1 pMol/µl |
| 5 u/µl Ampli Taq Gold | 0,2 | 0,05 u/µl |
| DNA-Template (HPV-Plasmid) | 1 | |
| Summe | 20 | |

Die PCR wurde unter folgenden Temperaturbedingungen durchgeführt. Zunächst wurde der Reaktionsansatz auf eine Temperatur auf 95°C gebracht, wobei die Temperatur mit einer Geschwindigkeit von 1 °C/sec erhöht wurde. Die Temperatur des Reaktionsansatzes wurde 10 Minuten bei 95°C gehalten. Danach wurde der Reaktionsansatz folgenden Temperaturbedingungen unterworfen: pro Zyklus 30 Sekunden bei 95°C, 30 Sekunden bei 55°C und 1 Minute bei 72°C. Insgesamt wurden 40 Zyklen durchgeführt. Nach Durchführung der 40 Zyklen wurde die Temperatur des Reaktionsansatzes 5 Minuten bei 72°C gehalten und dann auf 4°C abgekühlt.

Jeweils 2 µl der erhaltenen PCR-Produkte wurde auf einem nicht-denaturierenden Acrylamid-Gel aufgetrennt und mittels Silber-Färbung sichtbar gemacht. Die Ergebnisse sind in den Figuren 1A und 1B dargestellt. Sequenzunterschiede zwischen den verschiedenen HPV-Typen führen zu einem unterschiedlichen Laufverhalten im nicht-denaturierenden Gel. Wie aus Figur 1 ersichtlich, wurde bei der Kontroll-DNA als Negativ-Kontrolle kein PCR-Produkt erhalten.

### Beispiel 2

### PCR-Amplifikation unter Verwendung der erfindungsgemäßen Primer im Vergleich zur PCR-Amplifikation unter Verwendung von im Stand der Technik bekannten Primern

In diesem Versuch sollte die Eignung der erfindungsgemäßen Forward- und Reverse-Primer bei der Amplifikation von Nucleinsärue-Bereichen genitaler HPV-Viren im Vergleich zu der PCR-Amplifikation unter Verwendung von aus dem Stand der Technik bekannten Primerpaaren bestimmt werden.

In der DE 100 09 143 A1 werden zwei PCR-Primersysteme beschrieben. Diese Primersysteme umfassen entweder den Primer mit SEQ ID Nr. 1 (im Folgenden auch als P1 bezeichnet) und mit SEQ ID Nr. 2 (im Folgenden auch als P2 bezeichnet) oder der Primer mit SEQ ID Nr. 1 und SEQ ID Nr. 3 (im Folgenden auch als P3 bezeichnet). Da nur das PCR-System mit den Primern P1 und P3 zur Typisierung von Papilloma-Viren geeignet sind, stellt dieses die maßgebende Vergleichsbasis für das erfindungsgemäße Primersystem Loma/Loma-rev dar. Aufgrund der Lage der Primer am HPV-Genom können sowohl der Primer P1 als auch das erfindungsgemäße äquimolare Primer-Gemisch, umfassend die Primer Loma 1, Loma 2, Loma 3, Loma 4 und Loma 5, mit jedem der Primer P2, P3 und Loma-rev kombiniert werden, um ein PCR-Produkt zu erhalten. In dem Versuch wurden alle sechs möglichen Primer-Kombinationen ausgetestet. Die verwendeten Primer-Kombinationen sind in Tabelle 2 dargestellt.

Als DNA-Templates wurden das HPV16-Plasmid mit dem Genom des HPV16-Typs sowie HPV18-DNA, die aus HeLa-Zellen extrahiert worden war, eingesetzt. Die Konzentrationen der jeweiligen Templates wurden in Vorversuchen so eingestellt, dass sie in der Nähe der Grenze der Amplifizierbarkeit mit dem Primersystem Loma/Loma-rev kommen, aber noch ein deutliches PCR-Amplifikationsprodukt ergeben. Die PCR wurde unter folgenden Temperaturbedingungen durchgeführt: Rampe: 1°C/sec, Halten der Temperatur bei 95°C über 10 min, 40 Zyklen mit jeweils 30 sec bei 95°C, 30 sec bei 55°C, 1 min bei 72°C, danach 5 min Halten der Temperatur bei 72°C und Abkühlen auf 4°C.

**Tabelle 2**

| Primer-kombination | Forward-Primer | Reverse-Primer |
|---|---|---|
| 1 | Loma 1-5 | Loma-rev |
| 2 | Loma 1-5 | SEQ ID Nr. 2 aus DE 100 09 143 (P2) |
| 3 | Loma 1-5 | SEQ ID Nr. 3 aus DE 100 09 143 (P3) |
| 4 | SEQ ID Nr. 1 aus DE 100 09 143 (P1) | Loma-rev |
| 5 | SEQ ID Nr. 1 aus DE 100 09 143 (P1) | SEQ ID Nr. 2 aus DE 100 09 143 (P2) |
| 6 | SEQ ID Nr. 1 aus DE 100 09 143 (P1) | SEQ ID Nr. 3 aus DE 100 09 143 (P3) |

Die mit den vorstehenden Primer-Kombinationen erhaltenen Ergebnissse sind in Figur 2 dargestellt. Die Primer-Kombination 1 ergibt sowohl bei der Amplifikation der HPV16-DNA als auch bei der Amplifikation der HPV18-DNA ein PCR-Produkt. Die Primer-Kombination 2 ergibt im Falle der HPV16-DNA kein nachweisbares PCR-Produkt.
Die Primer-Kombination 3 ergibt bei beiden getesteten Nucleinsäure-Templates ein PCR-Produkt. Die Primer-Kombinationen 4 bis 6 ergeben bei der gewählten Verdünnung der HPV16-DNA kein PCR-Produkt und bei der HPV18-DNA eine deutlich geringere Amplifikationsprodukt-Menge als die Primer-Kombinationen 1 bis 3.

### Beispiel 3

### Amplifikation von HPV-Templates aus Patientinnen-Proben

Unter Verwendung von DNA-Extrakten aus vier Proben von Patientinnen (Paraffinschnitte von Cervicalproben) wurden PCR-Reaktionen mit den Primer-Kombinationen 1 und 6 (vergleiche Tabelle 2) durchgeführt. Diese Proben waren zuvor mittels in situ-Hybridisierung auf HPV-Infektionen untersucht worden. Die Ergebnisse sind in Tabelle 3 und Figur 3 dargestellt.

**Tabelle 3**

| Patienten-probe | Ergebnis In-situ Hybridisierung | Ergebnis PCR Loma1-5/ Loma rev | Ergebnis PCR SEQ ID Nr. 1 (P1)/ SEQ ID Nr. 3 (P3) |
|---|---|---|---|
| A | HPV16 | + | + |
| B | HPV73 | + | - |
| C | Negativ | + | + |
| D | HPV33 | + | - |

Aus den Ergebnissen ist erkennbar, dass beide PCR-Reaktionen im Fall der Probe C sensitiver als die Hybridisierung sind, da diese Probe bei beiden Primerpaaren positiv war. Die Proben B und D sind nur mit den erfindungsgemäßen Primern Loma/Loma-rev positiv, nicht jedoch mit der aus dem Stand der Technik bekannten Primer-Kombination P1/P3.

### Beispiel 4

### Hybridisierung eines Amplifikationsprodukts der Loma-Primer auf einen erfndungsgemäßen Nucleotidarray

Zur Amplifikation wurde als Template ein Plasmid mit einem Teil des Genoms von HPV42 eingesetzt, gemischt mit humaner DNA und mit einem Plasmid, das ein synthetisches DNA-Konstrukt, das einen kurzen Ausschnitt aus dem Genom des Lambda-Phagen flankiert von Bindungsstellen für die Primer nach SEQ ID Nr. 4 und SEQ ID Nr. 7, enthält. Die Sonde der Amplifikationskontrolle am Nucleotidarray ist spezifisch für diesen Ausschnitt des Lambda-Phagen-Genoms.

Im PCR-Ansatz wurden ausser den Loma Primern auch Primer zur Amplifikation eines Ausschnitts aus dem humanen ADAT1-Gen verwendet.

Einer der Primer zur Amplifikation des humanen ADAT1-Gens und der Primer nach SEQ ID Nr. 6 sind mit dem Fluoreszenzfarbstoff Cy5 markiert.

Nach erfolgter DNA-Amplifikation wurden 5µl des PCR-Produkts mit 30µl Hybridisierungspuffer (0.5% Laurylsarcosin, 0.225M NaCl, 0,0225M Natrium-Citrat, 20nm eines Cy3-markierten Oligonukleotids, das komplementär zur Hybridisierungs- und Printkontrolle am Nucleotidarray ist) gemischt. Die Mischung wurde 3min bei 95°C denaturiert, auf Eiswasser abgekühlt und für 10min bei 50°C auf den Nucleotidarray hybridisiert. Der Nucleotidarray wurde 3x für jeweils 20 Sekunden bei 50°C in Waschpuffer (0.2% Natriumdodecylsulfat, 0.3M NaCl, 0.03M Natrium Citrat, pH7) gewaschen. In einem handelsüblichen Microarrayscanner wurde der Nucleotidarray bei Anregung mit Licht einer Wellenlänge von 532nm und 635nm gescannt. Das Ergebnis ist in Figur 4b gezeigt. Figur 4a zeigt die Position der Sonden auf dem verwendeten Nucleotidarray.

Die vorliegende Erfindung betrifft auch die folgenden Aspekte:

Aspekt 1: Oligonucleotid, das als Primer, insbesondere Forward-Primer zur Amplifikation eines Nucleinsäure-Bereiches eines genitalen humanen Papillomavirus (HPV) eingesetzt werden kann und das die Sequenz 5'-CAR GCI AAA WWW KTD AAR GAY TGT G-3' oder 5'-CAR GCN AAA WWW KTD AAR GAY TGT G-3' (SEQ ID Nr. 1) aufweist, wobei R = A oder G ist, W = T oder A ist, K = T oder G ist, I = Inosin ist, N = A, T, G oder C ist, D = A, T oder G ist und Y = C oder T ist.

Aspekt 2: Oligonucleotid nach Aspekt 1, wobei das Oligonucleotid ausgewählt ist aus der Gruppe bestehend aus:
a) einem Oligonucleotid mit der Nucleotidsequenz 5'-CAR GCI AAA TAT KTR AAA GAT TGT G-3' oder 5'-CAR GCN AAA TAT KTR AAA GAT TGT G-3' (SEQ ID Nr. 2),
b) einem Oligonucleotid mit der Nucleotidsequenz 5'-CAR GCA AAA TAT GTW AAG GAT TGT G-3 '(SEQ ID Nr. 3),
c) einem Oligonucleotid mit der Nucleotidsequenz 5'-CAR GCW AAA ATT GTA AAR GAT TGT G-3' (SEQ ID Nr. 4),
d) einem Oligonucleotid mit der Nucleotidsequenz 5'-CAA GCA AAA ATA GTA AAR GAC TGT G-3' (SEQ ID Nr. 5), und
e) einem Oligonucleotid mit der Nucleotidsequenz 5'-CAR GCA AAA TAT GTA AAA GAC TGT G-3' (SEQ ID Nr. 6),
wobei R = A oder G ist, W = T oder A ist, K = T oder G ist, I = Inosin ist und N = A, T, G oder C ist.

Aspekt 3: Oligonucleotid, das als Primer, insbesondere Reverse-Primer zur Amplifikation eines Nucleinsäure-Bereiches eines genitalen humanen Papillomavirus eingesetzt werden kann, mit der Nucleotidsequenz 5'-ARY GGY TSY ARC CAA AAR TGR CT-3' (SEQ ID Nr. 7), wobei R = A oder G ist, Y = C oder T ist und S = C oder G ist.

Aspekt 4: Oligonucleotid, das als Sonde zum Nachweis und/oder zur Bestimmung genitaler HPV-Genotypen eingesetzt werden kann, ausgewählt aus der Gruppe bestehend aus:
1) einem Oligonucleotid mit der in SEQ ID Nr. 117 dargestellten Nucleotidsequenz zum Nachweis und/oder zur Bestimmung des HPV6-Genotyps,
2) einem Oligonucleotid mit der in SEQ ID Nr. 118 dargestellten Nucleotidsequenz zum Nachweis und/oder zur Bestimmung des HPV11-Genotyps,
3) einem Oligonucleotid mit der in SEQ ID Nr. 19 dargestellten Nucleotidsequenz zum Nachweis und/oder zur Bestimmung des HPV16-Genotyps,
4) einem Oligonucleotid mit der in SEQ ID Nr. 119 dargestellten Nucleotidsequenz zum Nachweis und/oder zur Bestimmung des HPV18-Genotyps,
5) einem Oligonucleotid mit der in SEQ ID Nr. 120 dargestellten Nucleotidsequenz zum Nachweis und/oder zur Bestimmung des HPV31-Genotyps,
6) einem Oligonucleotid mit der in SEQ ID Nr. 32 dargestellten Nucleotidsequenz zum Nachweis und/oder zur Bestimmung des HPV33-Genotyps,
7) einem Oligonucleotid mit der in SEQ ID Nr. 41 dargestellten Nucleotidsequenz zum Nachweis und/oder zur Bestimmung des HPV35h-Genotyps,
8) einem Oligonucleotid mit der in SEQ ID Nr. 44 dargestellten Nucleotidsequenz zum Nachweis und/oder zur Bestimmung des HPV39-Genotyps,
9) einem Oligonucleotid mit der in SEQ ID Nr. 48 dargestellten Nucleotidsequenz zum Nachweis und/oder zur Bestimmung des HPV40-Genotyps,
10) einem Oligonucleotid mit der in SEQ ID Nr. 121 dargestellten Nucleotidsequenz zum Nachweis und/oder zur Bestimmung des HPV42-Genotyps,
11) einem Oligonucleotid mit der in SEQ ID Nr. 122 dargestellten Nucleotidsequenz zum Nachweis und/oder zur Bestimmung des HPV43-Genotyps,
12) einem Oligonucleotid mit der in SEQ ID Nr. 123 dargestellten Nucleotidsequenz zum Nachweis und/oder zur Bestimmung des HPV44-Genotyps,
13) einem Oligonucleotid mit der in SEQ ID Nr. 124 dargestellten Nucleotidsequenz zum Nachweis und/oder zur Bestimmung des HPV45-Genotyps,
14) einem Oligonucleotid mit der in SEQ ID Nr. 125 dargestellten Nucleotidsequenz zum Nachweis und/oder zur Bestimmung des HPV51-Genotyps,
15) einem Oligonucleotid mit der in SEQ ID Nr. 126 dargestellten Nucleotidsequenz zum Nachweis und/oder zur Bestimmung des HPV52-Genotyps,
16) einem Oligonucleotid mit der in SEQ ID Nr. 127 dargestellten Nucleotidsequenz zum Nachweis und/oder zur Bestimmung des HPV53-Genotyps,
17) einem Oligonucleotid mit der in SEQ ID Nr. 128 dargestellten Nucleotidsequenz zum Nachweis und/oder zur Bestimmung des HPV56-Genotyps,
18) einem Oligonucleotid mit der in SEQ ID Nr. 82 dargestellten Nucleotidsequenz zum Nachweis und/oder zur Bestimmung des HPV58-Genotyps,
19) einem Oligonucleotid mit der in SEQ ID Nr. 129 dargestellten Nucleotidsequenz zum Nachweis und/oder zur Bestimmung des HPV59-Genotyps,
20) einem Oligonucleotid mit der in SEQ ID Nr. 130 dargestellten Nucleotidsequenz zum Nachweis und/oder zur Bestimmung des HPV66-Genotyps,
21) einem Oligonucleotid mit der in SEQ ID Nr. 131 oder 132 dargestellten Nucleotidsequenz zum Nachweis und/oder zur Bestimmung des HPV68-Genotyps,
22) einem Oligonucleotid mit der in SEQ ID Nr. 133 dargestellten Nucleotidsequenz zum Nachweis und/oder zur Bestimmung des HPV70-Genotyps,
23) einem Oligonucleotid mit der in SEQ ID Nr. 134 dargestellten Nucleotidsequenz zum Nachweis und/oder zur Bestimmung des HPV73-Genotyps, und
24) einem Oligonucleotid mit der in SEQ ID Nr. 135 dargestellten Nucleotidsequenz zum Nachweis und/oder zur Bestimmung des HPV82-Genotyps.

Aspekt 5: Oligonucleotid nach einem der Aspekte 1 bis 4, das eine Nucleotidsequenz aufweist, die gegenüber einer der in SEQ ID Nr. 1 bis 7, 19, 32, 41, 44, 48, 82 oder 117 bis 135 dargestellten Nucleotidsequenzen mutiert ist, erhältlich durch:
a) Deletion von 1 bis 10 Nucleotiden in einer der in SEQ ID Nr. 1 bis 7, 19, 32, 41, 44, 48, 82 oder 117 bis 135 dargestellten Nucleotidsequenzen,
b) Addition von 1 bis 10 Nucleotiden in einer der in SEQ ID Nr. 1 bis 7, 19, 32, 41, 44, 48, 82 oder 117 bis 135 dargestellten Nucleotidsequenzen, und/oder
c) Substitution von 1 bis 3 Nucleotiden in einer der in SEQ ID Nr. 1 bis 7, 19, 32, 41, 44, 48, 82 oder 117 bis 135 dargestellten Nucleotidsequenzen.

Aspekt 6: Oligonucleotid nach Aspekt 5, wobei die Deletion oder Addition der Nucleotide am 5'-Ende und/oder 3'-Ende einer der in SEQ ID Nr. 1 bis 7, 19, 32, 41, 44, 48, 82 oder 117 bis 135 dargestellten Nucleotidsequenzen vorliegt.

Aspekt 7: Oligonucleotid nach einem der Aspekte 1 bis 6, wobei es sich um ein DNA-Molekül, RNA-Molekül, PNA-Molekül, LNA-Molekül oder eine Mischform davon handelt.

Aspekt 8: Oligonucleotid nach einem der Aspekte 1 bis 7, wobei dessen Nucleotidsequenz zu einer Sequenz aus dem E1-Genbereich von mindestens einem genitalen HPV-Genotyp komplementär ist.

Aspekt 9: Oligonucleotid, das eine Nucleotidsequenz aufweist, die über die gesamte Länge zu der Nucleotidsequenz eines Oligonucleotids nach einem der Aspekte 1 bis 8 komplementär ist.

Aspekt 10: Primer-Paar zur Amplifikation eines Nucleinsäure-Bereiches eines genitalen humanen Papillomavirus (HPV), umfassend einen Forward-Primer und einen Reverse-Primer, wobei der Forward-Primer ausgewählt ist aus der Gruppe bestehend aus:
a) einem Oligonucleotid nach Aspekt 1 oder 2 mit einer der in SEQ ID Nr. 1 bis 6 dargestellten Nucleotidsequenzen,
b) einem Oligonucleotid nach Aspekt 5 oder 6, das eine gegenüber dem Oligonucleotid nach a) mutierte Nucleotidsequenz aufweist, und
c) einem Gemisch der Oligonucleotide nach a) und/oder b),
und der Reverse-Primer ausgewählt ist aus der Gruppe bestehend aus:
d) einem Oligonucleotid nach Aspekt 3 mit der in SEQ ID Nr. 7 dargestellten Nucleotidsequenz,
e) einem Oligonucleotid nach Aspekt 5 oder 6, das eine gegenüber dem Oligonucleotid nach d) mutierte Nucleotidsequenz aufweist, und
f) einem Gemisch der Oligonucleotide nach d) und e).

Aspekt 11: Primer-Paar nach Aspekt 10, wobei der Forward-Primer ein äquimolares Gemisch der Oligonucleotide mit den in SEQ ID Nr. 2 bis 6 dargestellten Nucleotidsequenzen ist und der Reverse-Primer das Oligonucleotid mit der in SEQ ID Nr. 7 dargestellten Nucleotidsequenz.

Aspekt 12: Nucleinsäuremolekül, umfassend mindestens einen Bereich, der eine der in SEQ ID Nr. 19, 32, 41, 44, 48, 82 oder 117 bis 135 dargestellten Nucleotidsequenzen, eine mutierte Nucleotidsequenz davon, erhältlich durch Deletion und/oder Addition von 1 bis 10 Nucleotiden und/oder Substitution von 1 bis 3 Nucleotiden einer der in SEQ ID Nr. 19, 32, 41, 44, 48, 82 oder 117 bis 135 dargestellten Nucleotidsequenzen, oder eine komplementäre Nucleotidsequenz davon und einen oder mehrere zusätzliche Bereiche mit einer Gesamtlänge von mindestens 1 Nucleotid aufweist.

Aspekt 13: Nucleinsäuremolekül nach Aspekt 12, wobei die zusätzlichen Bereiche keine Komplementarität zu Sequenzen eines Amplifikationsproduktes aufweisen, das mittels eines Amplifikationsverfahrens unter Verwendung eines Nucleinsäure-Bereiches eines genitalen humanen Papillomavirus als Matrize und eines Primer-Paares nach Aspekt 10 oder 11 erhältlich ist.

Aspekt 14: Nucleinsäuremolekül nach Aspekt 13, wobei die zusätzlichen Bereiche Poly-A- oder Poly-T-Nucleotidsequenzen aufweisen.

Aspekt 15: Nucleinsäuremolekül nach Aspekt 12, wobei die zusätzlichen Bereiche mehrfache Wiederholungen des Bereiches umfassen, der eine der in SEQ ID Nr. 19, 32, 41, 44, 48, 82 oder 117 bis 135 dargestellten Nucleotidsequenzen, eine mutierte Nucleotidsequenz davon oder eine komplementäre Nucleotidsequenz aufweist.

Aspekt 16: Nucleinsäuremolekül nach Aspekt 15, wobei die Wiederholungen durch Spacer mit einer Länge von mindestens einem Nucleotid oder mindestens einem Phosphat oder mindestens einem Kohlenstoffatom oder mindestens einer Aminogruppe getrennt sind.

Aspekt 17: Nucleinsäuremolekül, das eine Nucleotidsequenz aufweist, die über die gesamte Länge zu der Nucleotidsequenz eines Nucleinsäuremoleküls nach einem der Aspekte 12 bis 16 komplementär ist.

Aspekt 18: Nucleinsäuremolekül nach einem der Aspekte 12 bis 17, wobei es sich um ein DNA-Molekül, RNA-Molekül, PNA-Molekül, LNA-Molekül oder eine Mischform davon handelt.

Aspekt 19: Verfahren zur Amplifikation eines Bereiches einer in einer biologischen Probe vorhandenen Nucleinsäure eines genitalen humanen Papillomavirus, umfassend die Durchführung eines Nucleinsäure-Amplifikationsverfahrens unter Verwendung eines einen Forward-Primer und einen Reverse-Primer umfassenden Primer-Paares, wobei der Forward-Primer ausgewählt ist aus der Gruppe bestehend aus:
a) einem Oligonucleotid nach Aspekt 1 oder 2 mit einer der in SEQ ID Nr. 1 bis 6 dargestellten Nucleotidsequenzen,
b) einem Oligonucleotid nach Aspekt 5 oder 6, das eine gegenüber dem Oligonucleotid nach a) mutierte Nucleotidsequenz aufweist, und
c) einem Gemisch der Oligonucleotide nach a) und/oder b),
   und der Reverse-Primer ausgewählt ist aus der Gruppe bestehend aus:
d) einem Oligonucleotid nach Aspekt 3 mit der in SEQ ID Nr. 7 dargestellten Nucleotidsequenz,
e) einem Oligonucleotid nach Aspekt 5 oder 6, das eine gegenüber dem Oligonucleotid nach d) mutierte Nucleotidsequenz aufweist, und
f) einem Gemisch der Oligonucleotide nach d) und e).

Aspekt 20: Verfahren nach Aspekt 19, wobei das Oligonucleotid ein DNA-Molekül, RNA-Molekül, PNA-Molekül, LNA-Molekül oder eine Mischform davon ist.

Aspekt 21: Verfahren nach Aspekt 19 oder 20, wobei die biologische Probe ein Abstrich der Cervix uteri, eine frische Gewebeprobe, eine fixierte Gewebeprobe oder ein Schnittpräparat einer Gewebeprobe ist.

Aspekt 22: Verfahren nach einem der Aspekte 19 bis 21, wobei die zu amplifizierende Nucleinsäure aus der biologischen Probe aufgereinigt und/oder isoliert wird.

Aspekt 23: Verfahren nach Aspekt 22, wobei die zu amplifizierende Nucleinsäure eine DNA ist.

Aspekt 24: Verfahren nach einem der Aspekte 19 bis 23, wobei das Nucleinsäureamplifikations-Verfahren ein PCR (polymerase chain reaction)-Verfahren ist.

Aspekt 25: Verfahren nach Aspekt 24, wobei der Forward-Primer und der Reverse-Primer in der Nucleinsäure-Amplifikationsreaktion jeweils in einer Konzentration von 0,5-1 pMol/µl eingesetzt werden.

Aspekt 26: Verfahren nach Aspekt 25, wobei als Forward-Primer ein äquimolares Gemisch der Oligonucleotide mit den in SEQ ID Nr. 2 bis 6 dargestellten Nucleotidsequenzen eingesetzt wird, wobei jedes Oligonucleotid in einer Konzentration von 0,1-0,2 pMol/µl vorliegt, und als Reverse-Primer das Oligonucleotid mit der in SEQ ID Nr. 7 dargestellten Nucleotidsequenz in einer Konzentration von 0,5-1 pMol/µl.

Aspekt 27: Verfahren nach einem der Aspekte 24 bis 26, wobei die Nucleinsäureamplifikation unter folgenden Temperatur-Bedingungen durchgeführt wird:
a) Erhitzen auf 95°C, wobei die Temperatur pro sec um 1°C erhöht wird,
b) Halten der Temperatur bei 95°C für 10 min,
c) Durchführung von 40 Zyklen, jeweils umfassend 30 sec bei 95°C, 30 sec bei 55°C und 1 min bei 72°C,
d) Halten der Temperatur bei 72°C für 5 min und
e) Abkühlen auf 4°C.

Aspekt 28: Verfahren nach einem der Aspekte 19 bis 23, wobei das Nucleinsäureamplifikations-Verfahren ein LCR (ligase chain reaction)-Verfahren, ein NASBA-Verfahren oder ein isothermisches Verfahren ist.

Aspekt 29: Verfahren nach einem der Aspekte 19 bis 28, wobei ein Bereich des HPV-Gens E1 amplifiziert wird.

Aspekt 30: Verfahren nach einem der Aspekte 19 bis 29, wobei der amplifizierte Nucleinsäurebereich aufgereinigt und/oder isoliert wird.

Aspekt 31: Verfahren nach einem der Aspekte 19 bis 30, wobei das Amplifikationsprodukt während oder nach der Amplifikationsreaktion mit einer Markierung versehen wird.

Aspekt 32: Verfahren zum Nachweis und/oder zur Bestimmung eines genitalen HPV-Genotyps, umfassend die Untersuchung einer in einer biologischen Probe vorhandenen Nucleinsäure eines genitalen humanen Papillomavirus, insbesondere die Untersuchung des HPV-Gens E1 oder eines Teils davon, durch Hybridisierung mit mindestens einer Sonde, wobei die Sonde ausgewählt ist aus der Gruppe bestehend aus:
a) HPV-Genotyp-spezifischen Oligonucleotiden nach Aspekt 4 mit den in SEQ ID Nr. 19, 32, 41, 44, 48, 82 oder 117 bis 135 dargestellten Nucleotidsequenzen,
b) Oligonucleotiden nach Aspekt 5 oder 6, die eine gegenüber einem der Oligonucleotide nach a) mutierte Nucleotidsequenz aufweisen,
c) Oligonucleotiden nach Aspekt 9, die eine Nucleotidsequenz aufweisen, die gegenüber der eines Oligonucleotids nach a) oder b) komplementär ist,
d) Nucleinsäuremolekülen nach einem der Aspekte 12 bis 18, und
e) Gemischen der Oligonucleotide nach a) bis c) und/oder der Nucleinsäuremoleküle nach d),
und den Nachweis der Hybridisierung.

Aspekt 33: Verfahren nach Aspekt 32, wobei das als Sonde verwendete Oligonucleotid oder Nucleinsäuremolekül ein DNA-Molekül, RNA-Molekül, PNA-Molekül, LNA-Molekül oder eine Mischform davon ist.

Aspekt 34: Verfahren nach Aspekt 32 oder 33, wobei vor Hybridisierung mit der Sonde die in der biologischen Probe vorhandene HPV-Nucleinsäure amplifiziert wird.

Aspekt 35: Verfahren nach Aspekt 34, wobei die Amplifikation der Nucleinsäure mittels eines Verfahrens nach einem der Aspekte 19 bis 31 erfolgt.

Aspekt 36: Verfahren nach Aspekt 32 oder 33, wobei die biologische Probe einen amplifizierten Nucleinsäurebereich, erhältlich mittels eines Verfahrens nach einem der Aspekte 19 bis 31, umfasst.

Aspekt 37: Verfahren nach einem der Aspekte 32 bis 36, wobei die biologische Probe ein Abstrich der Cervix uteri, eine frische Gewebeprobe, eine fixierte Gewebeprobe oder ein Schnittpräparat einer Gewebeprobe ist.

Aspekt 38: Verfahren nach einem der Aspekte 19 bis 37, wobei das Verfahren zur Diagnose und/oder zur Früherkennung von Erkrankungen, Erkrankungsvorstufen, Erkrankungsrisiken und/oder krankhaften Veränderungen, die von genitalen humanen Papillomaviren verursacht werden, eingesetzt wird.

Aspekt 39: Verfahren nach Aspekt 38, wobei die Erkrankung eine Krebserkrankung ist.

Aspekt 40: Nucleotid-Array zum Nachweis und/oder zur Bestimmung des Genotyps eines in einer biologischen Probe enthaltenen humanen Papillomavirus, insbesondere nach einem Verfahren nach einem der Aspekte 32 bis 39, umfassend einen festen Träger mit einer Oberfläche und mindestens ein an die Träger-Oberfläche gebundenes erstes Oligonucleotid oder Nucleinsäuremolekül, das zum Nachweis und/oder zur Bestimmung eines genitalen HPV-Genotyps geeignet ist, ausgewählt aus der Gruppe bestehend aus:
a) HPV-Genotyp-spezifischen Oligonucleotiden nach Aspekt 4 mit den in SEQ ID Nr. 19, 32, 41, 44, 48, 82 oder 117 bis 135 dargestellten Nucleotidsequenzen,
b) Oligonucleotiden nach Aspekt 5 oder 6, die eine gegenüber einem der Oligonucleotide nach a) mutierte Nucleotidsequenz aufweisen,
c) Oligonucleotiden nach Aspekt 9, die eine Nucleotidsequenz aufweisen, die gegenüber der eines Oligonucleotids nach a) oder b) komplementär ist,
d) Nucleinsäuremolekülen nach einem der Aspekte 12 bis 18, und
e) Gemischen der Oligonucleotide nach a) bis c) und/oder der Nucleinsäuremoleküle nach d).

Aspekt 41: Nucleotid-Array nach Aspekt 40, wobei der Träger plättchenförmig, beispielsweise in Form eines Objektträgers oder plättchenförmig mit Vertiefungen, beispielsweise als Chamberslide oder als Mikrotiterplatte mit Abmessungen gemäß Empfehlung der SBS (Society of Biomolecular Screening), ausgebildet ist.

Aspekt 42: Nucleotid-Array nach Aspekt 40 oder 41, wobei die ersten Oligonucleotide oder Nucleinsäuremoleküle auf der Oberfläche des Trägers in einem definierten Analysebereich angeordnet sind.

Aspekt 43: Nucleotid-Array nach einem der Aspekte 40 bis 42, wobei die Oberfläche des Trägers einen Kontrollbereich aufweist.

Aspekt 44: Nucleotid-Array nach Aspekt 43, wobei der Kontrollbereich eine Kontrolle zur Orientierung des Trägers, eine Amplifikations-Kontrolle, eine Hybridisierungs-Kontrolle, eine Proben-Kontrolle und/oder eine Print-Kontrolle umfasst.

Aspekt 45: Nucleotid-Array nach Aspekt 44, wobei die Kontrolle zur Orientierung des Trägers mindestens ein zweites Oligonucleotid oder Nucleinsäuremolekül umfasst.

Aspekt 46: Nucleotid-Array nach Aspekt 45, wobei das zweite Oligonucleotid ein fluoreszierendes Oligonucleotid ist und die Kontrolle zur Träger-Orientierung mindestens drei Spots des fluoreszierenden Oligonucleotids umfasst.

Aspekt 47: Nucleotid-Array nach Aspekt 44, wobei die Amplifikations-Kontrolle mindestens ein drittes Oligonucleotid oder Nucleinsäuremolekül umfasst.

Aspekt 48: Nucleotid-Array nach Aspekt 47, wobei das dritte Oligonucleotid oder Nucleinsäuremolekül als Sonde zum Nachweis eines Amplifikationsproduktes geeignet ist, das mittels eines Amplifikationsverfahrens unter Verwendung einer Kontroll-Nucleinsäure als Matrize und eines Primer-Paares nach Aspekt 10 oder 11 erhältlich ist.

Aspekt 49: Nucleotid-Array nach Aspekt 48, wobei die Kontroll-Nucleinsäure eine Länge und einen GC-Gehalt aufweist, der der Länge und dem GC-Gehalts des Amplifikationsproduktes entspricht, das mittels eines Amplifikationsverfahrens unter Verwendung des Nucleinsäure-Bereiches eines genitalen humanen Papillomavirus als Matrize und eines Primer-Paares nach Aspekt 10 oder 11 erhältlich ist.

Aspekt 50: Nucleotid-Array nach Aspekt 44, wobei die Hybridisierungskontrolle mindestens ein viertes Oligonucleotid oder Nucleinsäuremolekül umfasst.

Aspekt 51: Nucleotid-Array nach Aspekt 50, wobei die Hybridisierungskontrolle mindestens zwei bis 10 Spots des vierten Oligonucleotids oder Nucleinsäuremoleküls umfasst und die Spots unterschiedliche definierte Mengen des vierten Oligonucleotids oder Nucleinsäuremoleküls aufweisen.

Aspekt 52: Nucleotid-Array nach Aspekt 51, wobei die Hybridisierungskontrolle Spots mit einer Verdünnungsreihe des vierten Oligonucleotids oder Nucleinsäuremoleküls umfasst.

Aspekt 53: Nucleotid-Array nach Aspekt 44, wobei die Proben-Kontrolle mindestens ein fünftes Oligonucleotid oder Nucleinsäuremolekül umfasst.

Aspekt 54: Nucleotid-Array nach Aspekt 53, wobei das fünfte Oligonucleotid oder Nucleinsäuremolekül als Sonde zum Nachweis des humanen ADAT1-Gens geeignet ist.

Aspekt 55: Nucleotid-Array nach Aspekt 44, wobei die Print-Kontrolle mindestens ein sechstes Oligonucleotid oder Nucleinsäuremolekül umfasst.

Aspekt 56: Nucleotid-Array nach einem der Aspekte 40 bis 55, wobei die Oligonucleotide und Nucleinsäuremoleküle als DNA-Moleküle, RNA-Moleküle, PNA-Moleküle, LNA-Moleküle oder Mischformen davon ausgebildet sind.

Aspekt 57: Kit zum Nachweis und/oder zur Bestimmung genitaler HPV-Genotypen, umfassend mindestens einen ersten Behälter mit mindestens einem Primer zur Amplifikation von Bereichen des HPV-Gens E1, ausgewählt aus Oligonucleotiden nach einem der Aspekte 1 bis 3, Oligonucleotiden nach Aspekt 5 oder 6 mit einer Nucleotidsequenz, die gegenüber einer der in SEQ ID Nr. 1 bis 7 dargestellten Nucleotidsequenzen mutiert ist, und/oder Primer-Paaren nach Aspekt 10 oder 11, und mindestens einen zweiten Behälter mit mindestens einer Sonde zum Nachweis eines amplifizierten Bereiches des HPV-Gens E1, ausgewählt aus Oligonucleotiden nach einem der Aspekte 4 bis 9, das eine der in SEQ ID Nr. 19, 32, 41, 44, 48, 82 oder 117 bis 135 dargestellten Nucleotidsequenzen, eine mutierte Sequenz davon oder eine komplementäre Sequenz davon aufweist, und Nucleinsäuremolekülen nach einem der Aspekte 12 bis 18.

Aspekt 58: Kit nach Aspekt 57, umfassend mindestens 24 zweite Behälter mit mindestens 24 verschiedenen Sonden zum Nachweis und/oder zur Bestimmung der HPV6-, HPV11-, HPV16-, HPV18-, HPV31-, HPV33-, HPV35h-, HPV39-, HPV40-, HPV42-, HPV43-, HPV44-, HPV45-, HPV51-, HPV52-, HPV53-, HPV56-, HPV58-, HPV59-, HPV66-, HPV68-, HPV70-, HPV73- und HPV82-Genotypen, wobei jeder Behälter mindestens eine Sonde enthält und wobei alle in einem Behälter enthaltenen Sonden nur einen spezifischen genitalen HPV-Genotyp nachweisen können.

Aspekt 59: Kit zum Nachweis und/oder zur Bestimmung genitaler HPV-Genotypen, umfassend mindestens einen ersten Behälter mit mindestens einem Primer zur Amplifikation von Bereichen des HPV-Gens E1, ausgewählt aus Oligonucleotiden nach einem der Aspekte 1 bis 3, Oligonucleotiden nach Aspekt 5 oder 6 mit einer Nucleotidsequenz, die gegenüber einer der in SEQ ID Nr. 1 bis 7 dargestellten Nucleotidsequenzen mutiert ist, und/oder Primer-Paaren nach Aspekt 10 oder 11, und einen Nucleotid-Array nach einem der Aspekte 40 bis 56.

Aspekt 60: Kit nach einem der Aspekte 57 bis 59, umfassend zwei erste Behälter, wobei ein Behälter äquimolare Mengen der Oligonucleotide mit den in SEQ ID Nr. 2 bis 6 dargestellten Nucleotidsequenzen oder mutierten Sequenzen davon enthält und ein Behälter ein Oligonucleotid mit der in SEQ ID Nr. 7 dargestellten Nucleotidsequenz oder einer mutierten Nucleotidsequenz davon enthält.

Aspekt 61: Kit nach einem der Aspekte 57 bis 59, umfassend sechs erste Behälter, wobei fünf Behälter jeweils eines der Oligonucleotide mit den in SEQ ID Nr. 2 bis 6 dargestellten Nucleotidsequenzen oder mutierten Sequenzen davon enthalten und ein Behälter ein Oligonucleotid mit der in SEQ ID Nr. 7 dargestellten Nucleotidsequenz oder einer mutierten Nucleotidsequenz davon enthält.

Aspekt 62: Kit nach einem der Aspekte 57 bis 61, umfassend zusätzlich einen Behälter mit einer Kontroll-Nucleinsäure, die unter Verwendung eines Oligonucleotids nach Aspekt 1 oder 2 mit einer der in SEQ ID Nr. 1 bis 6 dargestellten Nucleotidsequenzen als Forward-Primer und eines Oligonucleotids nach Aspekt 3 mit der in SEQ ID Nr. 7 dargestellten Nucleotidsequenz als Reverse-Primer amplifiziert werden kann.

Aspekt 63: Verwendung eines Oligonucleotids nach Aspekt 4, eines Oligonucleotids nach Aspekt 5 oder 6, dessen Nucleotidsequenz gegenüber einer der in SEQ ID Nr. 19, 32, 41, 44, 48, 82 oder 117 bis 135 dargestellten Nucleotidsequenzen mutiert ist, eines Oligonucleotids nach Aspekt 9, dessen Nucleotidsequenz zu einer der in SEQ ID Nr. 19, 32, 41, 44, 48, 82 oder 117 bis 135 dargestellten Nucleotidsequenzen oder einer mutierten Nucleotidsequenz davon komplementär ist, oder eines Nucleinsäuremoleküls nach einem der Aspekte 12 bis 18 zum Nachweis und/oder zur Bestimmung eines genitalen HPV-Genotyps.

Aspekt 64: Verwendung eines Oligonucleotids nach einem der Aspekte 1 bis 3, eines Oligonucleotids nach Aspekt 5 oder 6, dessen Nucleotidsequenz gegenüber einer der in SEQ ID Nr. 1 bis 7 dargestellten Nucleotidsequenzen mutiert ist, oder eines Primer-Paares nach Aspekt 10 oder 11 zur Amplifikation eines Nucleinsäure-Bereiches eines genitalen humanen Papillomavirus.

Aspekt 65: Verwendung eines Oligonucleotids nach einem der Aspekte 1 bis 9, eines Nucleinsäuremoleküls nach einem der Aspekte 12 bis 18 oder eines Primer-Paares nach Aspekt 10 oder 11 zur Diagnose und/oder Früherkennung von Krankheiten, die von genitalen humanen Papillomaviren verursacht werden.

Aspekt 66: Verwendung eines Oligonucleotids nach einem der Aspekte 1 bis 9, eines Nucleinsäuremoleküls nach einem der Aspekte 12 bis 18 oder eines Primer-Paares nach Aspekt 10 oder 11 zur Herstellung eines Mittels zur Diagnose von Krankheiten, die von genitalen humanen Papillomaviren verursacht werden.

Aspekt 67: Verwendung nach Aspekt 66, wobei das Mittel ein Nucleotid-Array nach einem der Aspekte 40 bis 56 ist.

Aspekt 68: Verwendung nach Aspekt 66, wobei das diagnostische Mittel ein Kit nach einem der Aspekte 57 bis 62 ist.

Aspekt 69: Verwendung nach einem der Aspekte 63 bis 68, wobei das Oligonucleotid oder Nucleinsäuremolekül ein DNA-Molekül, RNA-Molekül, PNA-Molekül, LNA-Molekül oder eine Mischform davon ist.

## Patentansprüche

1. Nucleotid-Array zum Nachweis und/oder zur Bestimmung des Genotyps eines in einer biologischen Probe enthaltenen humanen Papillomavirus, umfassend einen festen Träger mit einer Oberfläche und mindestens ein an die Träger-Oberfläche gebundenes erstes Oligonucleotid oder Nucleinsäuremolekül, das als Sonde zur Untersuchung des HPV-Gens E1 oder eines Teils davon, zum Nachweis und zur Bestimmung eines genitalen humanen HPV-Genotyps geeignet ist, ausgewählt aus der Gruppe bestehend aus:
a) HPV-Genotyp-spezifischen Oligonucleotiden mit den in SEQ ID Nr. 18, 20, 21, 22, 23, 24, 29, 30 und 31 dargestellten Nucleotidsequenzen,
b) Oligonucleotiden, die eine gegenüber einem der Oligonucleotide nach a) mutierte Nucleotidsequenz aufweisen, nämlich eine Deletion oder Addition von 1 bis 10 Nucleotiden oder eine Substitution von 1 bis 3 Nucleotiden in einer der in a) dargestellten Nucleotidsequenzen,
c) Oligonucleotiden, die eine Nucleotidsequenz aufweisen, die über die gesamte Länge zu der Nucleotidsequenz eines Oligonucleotids nach a) oder b) komplementär ist,
d) Nucleinsäuremolekülen umfassend mindestens einen Bereich, der eine der in a) bis c) dargestellten Nucleotidsequenzen und einen oder mehrere zusätzliche Bereiche mit einer Gesamtlänge von mindestens einem Nucleotid aufweist, und
e) Gemischen der Oligonucleotide nach a) bis c) und/oder der Nucleinsäuremoleküle nach d).

2. Nucleotid-Array nach Anspruch 1, wobei der Träger plättchenförmig, beispielsweise in Form eines Objektträgers oder plättchenförmig mit Vertiefungen, beispielsweise als Chamberslide oder als Mikrotiterplatte mit Abmessungen gemäß Empfehlung der SBS (Society of Biomolecular Screening), ausgebildet ist.

3. Nucleotid-Array nach Anspruch 1 oder 2, wobei die ersten Oligonucleotide oder Nucleinsäuremoleküle auf der Oberfläche des Trägers in einem definierten Analysebereich angeordnet sind.

4. Nucleotid-Array nach einem der Ansprüche 1 bis 3, wobei die Oberfläche des Trägers einen Kontrollbereich aufweist.

5. Nucleotid-Array nach Anspruch 4, wobei der Kontrollbereich eine Kontrolle zur Orientierung des Trägers, eine Amplifikations-Kontrolle, eine Hybridisierungs-Kontrolle, eine Proben-Kontrolle und/oder eine Print-Kontrolle umfasst.

6. Nucleotid-Array nach Anspruch 5, wobei die Kontrolle zur Orientierung des Trägers mindestens ein zweites Oligonucleotid oder Nucleinsäuremolekül umfasst.

7. Nucleotid-Array nach Anspruch 6, wobei das zweite Oligonucleotid ein fluoreszierendes Oligonucleotid ist und die Kontrolle zur Träger-Orientierung mindestens drei Spots des fluoreszierenden Oligonucleotids umfasst.

8. Nucleotid-Array nach Anspruch 7, wobei die Amplifikations-Kontrolle mindestens ein drittes Oligonucleotid oder Nucleinsäuremolekül umfasst.

9. Nucleotid-Array nach Anspruch 5, wobei die Hybridisierungskontrolle mindestens ein viertes Oligonucleotid oder Nucleinsäuremolekül umfasst.

10. Nucleotid-Array nach Anspruch 5, wobei die Proben-Kontrolle mindestens ein fünftes Oligonucleotid oder Nucleinsäuremolekül umfasst.

11. Nucleotid-Array nach Anspruch 10, wobei das fünfte Oligonucleotid oder Nucleinsäuremolekül als Sonde zum Nachweis des humanen ADAT1-Gens geeignet ist.

12. Nucleotid-Array nach Anspruch 5, wobei die Print-Kontrolle mindestens ein sechstes Oligonucleotid oder Nucleinsäuremolekül umfasst.

13. Kit zum Nachweis und/oder zur Bestimmung genitaler HPV-Genotypen, umfassend mindestens einen ersten Behälter mit mindestens einem Primer zur Amplifikation von Bereichen des HPV-Gens E1, ausgewählt aus den Oligonucleotiden der Gruppe bestehend aus
einem Oligonucleotid, das als Primer, insbesondere Forward-Primer zur Amplifikation eines Nucleinsäure-Bereiches eines genitalen humanen Papillomavirus (HPV) eingesetzt werden kann und das die Sequenz 5'-CAR GCI AAA WWW KTD AAR GAY TGT G-3' oder 5'-CAR GCN AAA WWW KTD AAR GAY TGT G-3' (SEQ ID Nr. 1) aufweist, wobei R = A oder G ist, W = T oder A ist, K = T oder G ist, I = Inosin ist, N = A, T, G oder C ist, D = A, T oder G ist und Y = C oder T ist,
einem Oligonucleotid mit der Nucleotidsequenz 5'-CAR GCI AAA TAT KTR AAA GAT TGT G-3' oder 5'-CAR GCN AAA TAT KTR AAA GAT TGT G-3' (SEQ ID Nr. 2),
einem Oligonucleotid mit der Nucleotidsequenz 5'-CAR GCA AAA TAT GTW AAG GAT TGT G-3 '(SEQ ID Nr. 3),
einem Oligonucleotid mit der Nucleotidsequenz 5'-CAR GCW AAA ATT GTA AAR GAT TGT G-3' (SEQ ID Nr. 4),
einem Oligonucleotid mit der Nucleotidsequenz 5'-CAA GCA AAA ATA GTA AAR GAC TGT G-3' (SEQ ID Nr. 5),
und einem Oligonucleotid mit der Nucleotidsequenz 5'-CAR GCA AAA TAT GTA AAA GAC TGT G-3' (SEQ ID Nr. 6),
wobei in SEQ ID Nr. 2 bis 6 R = A oder G ist, W = T oder A ist, K = T oder G ist, I = Inosin ist und N = A, T, G oder C ist,
einem Oligonucleotid, das als Primer, insbesondere Reverse-Primer zur Amplifikation eines Nucleinsäure-Bereiches eines genitalen humanen Papillomavirus eingesetzt werden kann, mit der Nucleotidsequenz 5'-ARY GGY TSY ARC CAA AAR TGR CT-3' (SEQ ID Nr. 7), wobei R = A oder G ist, Y = C oder T ist und S = C oder G ist,
einem Oligonucleotid, das eine Nucleotidsequenz aufweist, die gegenüber einer der in SEQ ID Nr. 1 bis 7 dargestellten Nucleotidsequenzen mutiert ist und erhältlich ist durch:
a) Deletion von 1 bis 10 Nucleotiden in einer der in SEQ ID Nr. 1 bis 7 dargestellten Nucleotidsequenzen,
b) Addition von 1 bis 10 Nucleotiden in einer der in SEQ ID Nr. 1 bis 7 dargestellten Nucleotidsequenzen, und/oder
c) Substitution von 1 bis 3 Nucleotiden in einer der in SEQ ID Nr. 1 bis 7 dargestellten Nucleotidsequenzen
und einen Nucleotid-Array nach einem der Ansprüche 1 bis 12.

14. Kit nach Anspruch 13, umfassend zwei erste Behälter, wobei ein Behälter äquimolare Mengen der Oligonucleotide mit den in SEQ ID Nr. 2 bis 6 dargestellten Nucleotidsequenzen oder mutierten Sequenzen davon enthält und ein Behälter ein Oligonucleotid mit der in SEQ ID Nr. 7 dargestellten Nucleotidsequenz oder einer mutierten Nucleotidsequenz davon enthält.

15. Kit nach Anspruch 13, umfassend sechs erste Behälter, wobei fünf Behälter jeweils eines der Oligonucleotide mit den in SEQ ID Nr. 2 bis 6 dargestellten Nucleotidsequenzen oder mutierten Sequenzen davon enthalten und ein Behälter ein Oligonucleotid mit der in SEQ ID Nr. 7 dargestellten Nucleotidsequenz oder einer mutierten Nucleotidsequenz davon enthält.

16. Verwendung eines Oligonucleotids mit einer der in SEQ ID Nr. 1 bis 7, 18, 20, 21, 22, 23, 24, 29, 30 und 31 dargestellten Nucleotidsequenzen, eines Oligonucleotids, dessen Nucleotidsequenz gegenüber einer der in SEQ ID Nr. 1 bis 7, 18, 20, 21, 22, 23, 24, 29, 30 und 31 dargestellten Nucleotidsequenzen mutiert ist, eines Oligonucleotides, das eine Nucleotidsequenz aufweist, die zu einer der in SEQ ID Nr. 1 bis 7, 18, 20, 21, 22, 23, 24, 29, 30 und 31 dargestellten Nucleotidsequenzen, oder einer mutierten Sequenz davon komplementär ist, eines Nucleinsäuremoleküles, das eine der in SEQ ID Nr. 18, 20, 21, 22, 23, 24, 29, 30 und 31 dargestellten Nucleotidsequenzen, eine mutierte Sequenz davon oder eine komplementäre Sequenz davon umfasst, zur Herstellung eines Nucleotid-Arrays gemäß der Ansprüche 1 bis 12 oder eines Kits gemäß der Ansprüche 13 bis 15 zur Diagnose von Krankheiten, die von genitalen humanen Papillomaviren verursacht werden.

17. Verwendung nach Anspruch 16, wobei das Oligonucleotid oder Nucleinsäuremolekül ein DNA-Molekül, RNA-Molekül, PNA-Molekül, LNA-Molekül oder eine Mischform davon ist.

18. Verfahren zum Nachweis und/oder Bestimmung des Genotyps eines in einer biologischen Probe enthaltenen humanen Papillomavirus, wobei ein Nucleotidarray nach einem der Ansprüche 1 bis 12 für den Nachweis und/oder die Bestimmung eingesetzt wird.


SEQUENZPROTOKOLL

<110> Greiner Bio-One

<120> Sonden zum Nachweis genitaler HPV-Genotypen

<130> 201889 EP

<140>
<141>

<160> 135

<170> PatentIn Ver. 2.1

<210> 1
<211> 25
<212> DNA
<213> Humanes Papilloma-Virus

<400> 1
cargcnaaaw wwktdaarga ytgtg 25

<210> 2
<211> 25
<212> DNA
<213> Humanes Papilloma-Virus

<400> 2
cargcnaaat atktraaaga ttgtg 25

<210> 3
<211> 25
<212> DNA
<213> Humanes Papilloma-Virus

<400> 3
cargcaaaat atgtwaagga ttgtg 25

<210> 4
<211> 25
<212> DNA
<213> Humanes Papilloma-Virus

<400> 4
cargcwaaaa ttgtaaarga ttgtg 25

<210> 5
<211> 25
<212> DNA
<213> Humanes Papilloma-Virus

<400> 5
caagcaaaaa tagtaaarga ctgtg 25

<210> 6
<211> 25
<212> DNA

<213> Humanes Papilloma-Virus

<400> 6
cargcaaaat atgtaaaaga ctgtg                                          25


<210> 7
<211> 23
<212> DNA
<213> Humanes Papilloma-Virus

<400> 7
aryggytsya rccaaaartg rct                                            23


<210> 8
<211> 30
<212> DNA
<213> Humanes Papilloma-Virus

<400> 8
aagctttcta ggaggtacag ttattagtca                                     30


<210> 9
<211> 30
<212> DNA
<213> Humanes Papilloma-Virus

<400> 9
ataaaacata ggggttctaa aatagaaggc                                     30


<210> 10
<211> 30
<212> DNA
<213> Humanes Papilloma-Virus

<400> 10
actaaagttg acagtgtagg taactggaag                                     30


<210> 11
<211> 30
<212> DNA
<213> Humanes Papilloma-Virus

<400> 11
ttaaacaatg gattaagtat aggggtacta                                     30


<210> 12
<211> 30
<212> DNA
<213> Humanes Papilloma-Virus

<400> 12
aaaagatgtc tattaaacaa tggattaagt                                     30


<210> 13
<211> 29
<212> DNA

<213> Humanes Papilloma-Virus

<400> 13
gggtactaaa gttgacagtg taggtaact                                          29


<210> 14
<211> 30
<212> DNA
<213> Humanes Papilloma-Virus

<400> 14
gggaacagtt attagttatg ttaattcctg                                         30


<210> 15
<211> 30
<212> DNA
<213> Humanes Papilloma-Virus

<400> 15
aattgtatag ccattgtagg gccacctgac                                         30


<210> 16
<211> 30
<212> DNA
<213> Humanes Papilloma-Virus

<400> 16
gggccacctg acactgggaa gtcgtgcttt                                         30


<210> 17
<211> 30
<212> DNA
<213> Humanes Papilloma-Virus

<400> 17
gggccacctg acactgggaa gtcgtgcttt                                         30


<210> 18
<211> 30
<212> DNA
<213> Humanes Papilloma-Virus

<400> 18
tgatggaggt gattggaagc aaattgttat                                         30


<210> 19
<211> 30
<212> DNA
<213> Humanes Papilloma-Virus

<400> 19
tgaaatttct gcaagggtct gtaatatgtt                                         30


<210> 20
<211> 30
<212> DNA

<213> Humanes Papilloma-Virus

<400> 20
atggaggtga ttggaagcaa attgttatgt                                        30


<210> 21
<211> 30
<212> DNA
<213> Humanes Papilloma-Virus

<400> 21
gaccaatagt gcaattcctg cgataccaac                                        30


<210> 22
<211> 30
<212> DNA
<213> Humanes Papilloma-Virus

<400> 22
caaacattat aggcgagccc aaaaacgaca                                        30


<210> 23
<211> 30
<212> DNA
<213> Humanes Papilloma-Virus

<400> 23
tcctgcgata ccaacaaata gagtttataa                                        30


<210> 24
<211> 28
<212> DNA
<213> Humanes Papilloma-Virus

<400> 24
ctgcgatacc aacaaataga gtttataa                                          28


<210> 25
<211> 30
<212> DNA
<213> Humanes Papilloma-Virus

<400> 25
agaaacgatc tatgtgtatg tcacaatggc                                        30


<210> 26
<211> 30
<212> DNA
<213> Humanes Papilloma-Virus

<400> 26
agaagagggc gggtcgtgga aggaaattgc                                        30


<210> 27
<211> 30
<212> DNA

<213> Humanes Papilloma-Virus

<400> 27
ggcgggtcgt ggaaggaaat tgccaaattt                                        30


<210> 28
<211> 30
<212> DNA
<213> Humanes Papilloma-Virus

<400> 28
gcacagaaac gatctatgtg tatgtcacaa                                        30


<210> 29
<211> 30
<212> DNA
<213> Humanes Papilloma-Virus

<400> 29
gtagatgtga caaagttagt gacgaaggtg                                        30


<210> 30
<211> 30
<212> DNA
<213> Humanes Papilloma-Virus

<400> 30
tgagccttat tagcttttta caaggatgta                                        30


<210> 31
<211> 30
<212> DNA
<213> Humanes Papilloma-Virus

<400> 31
caaagttagt gacgaaggtg actggaggga                                        30


<210> 32
<211> 30
<212> DNA
<213> Humanes Papilloma-Virus

<400> 32
acagttttta aaagggtgtg ttatatcatg                                        30


<210> 33
<211> 30
<212> DNA
<213> Humanes Papilloma-Virus

<400> 33
gggtgtgtta tatcatgtgt aaattctaaa                                        30


<210> 34
<211> 30
<212> DNA

<213> Humanes Papilloma-Virus

<400> 34
aaagggtgtg ttatatcatg tgtaaattct                                        30


<210> 35
<211> 30
<212> DNA
<213> Humanes Papilloma-Virus

<400> 35
attacacata gatgtgattt aatagatgat                                        30


<210> 36
<211> 30
<212> DNA
<213> Humanes Papilloma-Virus

<400> 36
tttaatgcag tttatgcaag gtgtggttat                                        30


<210> 37
<211> 30
<212> DNA
<213> Humanes Papilloma-Virus

<400> 37
tgatttaata gatgatggag gaaactggaa                                        30


<210> 38
<211> 30
<212> DNA
<213> Humanes Papilloma-Virus

<400> 38
tgcagtttat gcaaggtgtg gttatttcat                                        30


<210> 39
<211> 30
<212> DNA
<213> Humanes Papilloma-Virus

<400> 39
ttaatagatg atggaggaaa ctggaaacat                                        30


<210> 40
<211> 28
<212> DNA
<213> Humanes Papilloma-Virus

<400> 40
aggtggacga tgacggtgac tggaggga                                          28


<210> 41
<211> 30
<212> DNA

<213> Humanes Papilloma-Virus

<400> 41
gcatttctta caaggagcta ttatatccta                                        30


<210> 42
<211> 30
<212> DNA
<213> Humanes Papilloma-Virus

<400> 42
aaggcaaatg tccatgtctc aatggataaa                                        30


<210> 43
<211> 30
<212> DNA
<213> Humanes Papilloma-Virus

<400> 43
tgtaaacatt acaagcgagc acaaaaaagg                                        30


<210> 44
<211> 30
<212> DNA
<213> Humanes Papilloma-Virus

<400> 44
agccttatgc attttttaca gggcacagtt                                        30


<210> 45
<211> 30
<212> DNA
<213> Humanes Papilloma-Virus

<400> 45
tatttcatat gtaaactcca ccagccactt                                        30


<210> 46
<211> 30
<212> DNA
<213> Humanes Papilloma-Virus

<400> 46
aatttaggtg tagtaaatgt gatgaaggcg                                        30


<210> 47
<211> 27
<212> DNA
<213> Humanes Papilloma-Virus

<400> 47
gaagtgtgat gaaggcgact ggaaacc                                        27


<210> 48
<211> 30
<212> DNA

<213> Humanes Papilloma-Virus

<400> 48
gaatgagcct gatgcacttt atgcaaggta                                        30


<210> 49
<211> 30
<212> DNA
<213> Humanes Papilloma-Virus

<400> 49
cctgatgcac tttatgcaag gtacaataat                                        30


<210> 50
<211> 30
<212> DNA
<213> Humanes Papilloma-Virus

<400> 50
tccatgtgca aacactatag gttagcagaa                                        30


<210> 51
<211> 30
<212> DNA
<213> Humanes Papilloma-Virus

<400> 51
acaaatgaga cgtatgtcta tgggtgcatg                                        30


<210> 52
<211> 30
<212> DNA
<213> Humanes Papilloma-Virus

<400> 52
gacgtatgtc tatgggtgca tggataaaac                                        30


<210> 53
<211> 30
<212> DNA
<213> Humanes Papilloma-Virus

<400> 53
cagtaaattt gaagacacag gaaattggaa                                        30


<210> 54
<211> 30
<212> DNA
<213> Humanes Papilloma-Virus

<400> 54
ggcactgtaa ttagttatgt aaactccagc                                        30


<210> 55
<211> 30
<212> DNA

<213> Humanes Papilloma-Virus

<400> 55
atatgaaaca gtggataaaa tttaggagca                                        30


<210> 56
<211> 30
<212> DNA
<213> Humanes Papilloma-Virus

<400> 56
acgccaaatg aatatgtctc aatggattaa                                        30


<210> 57
<211> 30
<212> DNA
<213> Humanes Papilloma-Virus

<400> 57
cgccaaatga atatgtctca atggattaaa                                        30


<210> 58
<211> 30
<212> DNA
<213> Humanes Papilloma-Virus

<400> 58
tttaagggca ctaaaggaat ttcttaaagg                                        30


<210> 59
<211> 30
<212> DNA
<213> Humanes Papilloma-Virus

<400> 59
ggtgcaataa tatcatttgt aaattcaaac                                        30


<210> 60
<211> 30
<212> DNA
<213> Humanes Papilloma-Virus

<400> 60
ggcactaaag gaatttctta aaggaacacc                                        30


<210> 61
<211> 30
<212> DNA
<213> Humanes Papilloma-Virus

<400> 61
agggcactaa aggaatttct taaaggaaca                                        30


<210> 62
<211> 30
<212> DNA

<213> Humanes Papilloma-Virus

<400> 62
cattatctat gtcagcctgg ataaggtata                                        30


<210> 63
<211> 30
<212> DNA
<213> Humanes Papilloma-Virus

<400> 63
gcacaaagaa aatcattatc tatgtcagcc                                        30


<210> 64
<211> 30
<212> DNA
<213> Humanes Papilloma-Virus

<400> 64
aaatcattat ctatgtcagc ctggataagg                                        30


<210> 65
<211> 30
<212> DNA
<213> Humanes Papilloma-Virus

<400> 65
aaacatatga atattggaca atggatacag                                        30


<210> 66
<211> 30
<212> DNA
<213> Humanes Papilloma-Virus

<400> 66
gaaagaaaac atatgaatat tggacaatgg                                        30


<210> 67
<211> 30
<212> DNA
<213> Humanes Papilloma-Virus

<400> 67
ttaattaggt tcttaagtgg atgtgtaata                                        30


<210> 68
<211> 30
<212> DNA
<213> Humanes Papilloma-Virus

<400> 68
taaagcatgt atgtagcaag gtggatgatg                                        30


<210> 69
<211> 30
<212> DNA

<213> Humanes Papilloma-Virus

<400> 69
gatgaatatg aaacaatgga taaagcatgt                                        30


<210> 70
<211> 30
<212> DNA
<213> Humanes Papilloma-Virus

<400> 70
aagcatgtat gtagcaaggt ggatgatggt                                        30


<210> 71
<211> 30
<212> DNA
<213> Humanes Papilloma-Virus

<400> 71
gttatttatg gtcctccaaa cacgggtaaa                                        30


<210> 72
<211> 30
<212> DNA
<213> Humanes Papilloma-Virus

<400> 72
aaacaatgga taaagcatgt atgtagcaag                                        30


<210> 73
<211> 30
<212> DNA
<213> Humanes Papilloma-Virus

<400> 73
ttagttattt atggtcctcc aaacacgggt                                        30


<210> 74
<211> 30
<212> DNA
<213> Humanes Papilloma-Virus

<400> 74
ttagcttttt aggaggtgta gtgctatcat                                        30


<210> 75
<211> 30
<212> DNA
<213> Humanes Papilloma-Virus

<400> 75
ttgtgattta gtagaggagg aaggtgagtg                                        30


<210> 76
<211> 30
<212> DNA

<213> Humanes Papilloma-Virus

<400> 76
cgttgtgatt tagtagagga ggaaggtgag                                        30


<210> 77
<211> 30
<212> DNA
<213> Humanes Papilloma-Virus

<400> 77
catcgttgtg atttagtaga ggaggaaggt                                        30


<210> 78
<211> 30
<212> DNA
<213> Humanes Papilloma-Virus

<400> 78
gggcacaaca gcaacaaatg aatatgtgcc                                        30


<210> 79
<211> 30
<212> DNA
<213> Humanes Papilloma-Virus

<400> 79
cacaacagca acaaatgaat atgtgccagt                                        30


<210> 80
<211> 30
<212> DNA
<213> Humanes Papilloma-Virus

<400> 80
ctgtttggta ctttgtggac cgccaaatac                                        30


<210> 81
<211> 30
<212> DNA
<213> Humanes Papilloma-Virus

<400> 81
tttcaagggt ctgtcatttc atttgtgaat                                        30


<210> 82
<211> 30
<212> DNA
<213> Humanes Papilloma-Virus

<400> 82
acatttttta aaaggatgca ttatttcata                                        30


<210> 83
<211> 30
<212> DNA

<213> Humanes Papilloma-Virus

<400> 83
cgtggtatga caatgggaca atggatacaa                                        30


<210> 84
<211> 30
<212> DNA
<213> Humanes Papilloma-Virus

<400> 84
gagcagaaaa gcgtggtatg acaatgggac                                        30


<210> 85
<211> 30
<212> DNA
<213> Humanes Papilloma-Virus

<400> 85
aggatgcatt atttcatatg taaattccaa                                        30


<210> 86
<211> 30
<212> DNA
<213> Humanes Papilloma-Virus

<400> 86
aaagcgtggt atgacaatgg gacaatggat                                        30


<210> 87
<211> 30
<212> DNA
<213> Humanes Papilloma-Virus

<400> 87
agcctgctac attttttaca aggaactgta                                        30


<210> 88
<211> 30
<212> DNA
<213> Humanes Papilloma-Virus

<400> 88
gattacacat agaggccgca aggtggcaga                                        30


<210> 89
<211> 30
<212> DNA
<213> Humanes Papilloma-Virus

<400> 89
attacacata gaggccgcaa ggtggcagac                                        30


<210> 90
<211> 30
<212> DNA

<213> Humanes Papilloma-Virus

<400> 90
agtggattac acatagaggc cgcaaggtgg                                        30


<210> 91
<211> 30
<212> DNA
<213> Humanes Papilloma-Virus

<400> 91
acagtggatt acacatagag gccgcaaggt                                        30


<210> 92
<211> 30
<212> DNA
<213> Humanes Papilloma-Virus

<400> 92
gtaccattta ttagtgccct taaattgttt                                        30


<210> 93
<211> 30
<212> DNA
<213> Humanes Papilloma-Virus

<400> 93
ttacggacca agcgacacag ggaagtcgct                                        30


<210> 94
<211> 30
<212> DNA
<213> Humanes Papilloma-Virus

<400> 94
tacggaccaa gcgacacagg gaagtcgcta                                        30


<210> 95
<211> 30
<212> DNA
<213> Humanes Papilloma-Virus

<400> 95
gagccttata aattttttcc aagggtcagt                                        30


<210> 96
<211> 30
<212> DNA
<213> Humanes Papilloma-Virus

<400> 96
ccttataaat tttttccaag ggtcagtcat                                        30


<210> 97
<211> 30
<212> DNA

<213> Humanes Papilloma-Virus

<400> 97
attacaaaag ggcacagcaa cagcaaatga                                        30


<210> 98
<211> 30
<212> DNA
<213> Humanes Papilloma-Virus

<400> 98
atggaggaga ctggagaaca atagtaaagc                                        30


<210> 99
<211> 30
<212> DNA
<213> Humanes Papilloma-Virus

<400> 99
aagttgtatg gtaatatgcg gaccaccaaa                                        30


<210> 100
<211> 30
<212> DNA
<213> Humanes Papilloma-Virus

<400> 100
caaggatgtg taatatcata tgtaaacgcc                                        30


<210> 101
<211> 30
<212> DNA
<213> Humanes Papilloma-Virus

<400> 101
aggagactgg agaacaatag taaagctatt                                        30


<210> 102
<211> 30
<212> DNA
<213> Humanes Papilloma-Virus

<400> 102
gttgtatggt aatatgcgga ccaccaaaca                                        30


<210> 103
<211> 30
<212> DNA
<213> Humanes Papilloma-Virus

<400> 103
aacgacaaat gtcaatgccg caatggatta                                        30


<210> 104
<211> 30
<212> DNA

<213> Humanes Papilloma-Virus

<400> 104
cgggcacaaa aacgacaaat gtcaatgccg                                        30


<210> 105
<211> 30
<212> DNA
<213> Humanes Papilloma-Virus

<400> 105
tcaatgccgc aatggattaa atttagatgc                                        30


<210> 106
<211> 30
<212> DNA
<213> Humanes Papilloma-Virus

<400> 106
atggcgcaat ggattaggtt tagatgtgat                                        30


<210> 107
<211> 30
<212> DNA
<213> Humanes Papilloma-Virus

<400> 107
aaatgactat ggcgcaatgg attaggttta                                        30


<210> 108
<211> 30
<212> DNA
<213> Humanes Papilloma-Virus

<400> 108
attaggttta gatgtgataa atgtgacgat                                        30


<210> 109
<211> 30
<212> DNA
<213> Humanes Papilloma-Virus

<400> 109
taaatttata caaggtgtag ttatttcgta                                        30


<210> 110
<211> 30
<212> DNA
<213> Humanes Papilloma-Virus

<400> 110
atgtgattta actaatgatg gtggtaattg                                        30


<210> 111
<211> 30
<212> DNA

<213> Humanes Papilloma-Virus

<400> 111
taatgatggt ggtaattgga aagatattgt                                        30


<210> 112
<211> 30
<212> DNA
<213> Humanes Papilloma-Virus

<400> 112
taaacaaatg tcaatggcac aatggataca                                        30


<210> 113
<211> 30
<212> DNA
<213> Humanes Papilloma-Virus

<400> 113
ttaattagat ttttgcaagg gtgcgttatt                                        30


<210> 114
<211> 30
<212> DNA
<213> Humanes Papilloma-Virus

<400> 114
agaaaatcac taacaatgtc agcatggatt                                        30


<210> 115
<211> 30
<212> DNA
<213> Humanes Papilloma-Virus

<400> 115
taggtataga tgtgacaaag tgcaagacgg                                        30


<210> 116
<211> 30
<212> DNA
<213> Humanes Papilloma-Virus

<400> 116
ttgcgatacc agggtattaa ctttatgtat                                        30


<210> 117
<211> 30
<212> DNA
<213> Humanes Papilloma-Virus

<400> 117
gaggtacagt tattagtcat gtaaattcca                                        30


<210> 118
<211> 32
<212> DNA

<213> Humanes Papilloma-Virus

<400> 118
taagtttttg gggggaacag ttattagtta tg                                    32


<210> 119
<211> 34
<212> DNA
<213> Humanes Papilloma-Virus

<400> 119
ttatacactt tatacaagga gcagtaatat catt                                  34


<210> 120
<211> 30
<212> DNA
<213> Humanes Papilloma-Virus

<400> 120
caaggatgta taatatcata tgcaaattca                                       30


<210> 121
<211> 35
<212> DNA
<213> Humanes Papilloma-Virus

<400> 121
tgagtttaat aaacttctta gcaggaactg taata                                 35


<210> 122
<211> 30
<212> DNA
<213> Humanes Papilloma-Virus

<400> 122
aggcggtacc atattatcat atgtaaatgc                                       30


<210> 123
<211> 30
<212> DNA
<213> Humanes Papilloma-Virus

<400> 123
ggaggcactg taattagtta tgtaaactcc                                       30


<210> 124
<211> 30
<212> DNA
<213> Humanes Papilloma-Virus

<400> 124
gagttttata catttcctac aaggtgcaat                                       30


<210> 125
<211> 30
<212> DNA

<213> Humanes Papilloma-Virus

<400> 125
tatttgcaat gagcctaatg aagtttatgc                                        30


<210> 126
<211> 32
<212> DNA
<213> Humanes Papilloma-Virus

<400> 126
gagtttaatt aggttcttaa gtggatgtgt aa                                     32


<210> 127
<211> 29
<212> DNA
<213> Humanes Papilloma-Virus

<400> 127
gtttagttat ttatggtcct ccaaacacg                                         29


<210> 128
<211> 30
<212> DNA
<213> Humanes Papilloma-Virus

<400> 128
agtcttataa agttttttca agggtctgtc                                         30


<210> 129
<211> 32
<212> DNA
<213> Humanes Papilloma-Virus

<400> 129
tgagcctgct acatttttta caaggaactg ta                                      32


<210> 130
<211> 27
<212> DNA
<213> Humanes Papilloma-Virus

<400> 130
   tgagccttat aaattttttc caagggt                                            27


<210> 131
<211> 32
<212> DNA
<213> Humanes Papilloma-Virus

<400> 131
atacatttct tgcaaggcac aataatttca ta                                     32


<210> 132
<211> 30
<212> DNA

<213> Humanes Papilloma-Virus

<400> 132
tgagtcttat acatttcttg caaggcacaa                                        30


<210> 133
<211> 30
<212> DNA
<213> Humanes Papilloma-Virus

<400> 133
aatgcacttt ttacaaggta cagtaatttc                                        30


<210> 134
<211> 32
<212> DNA
<213> Humanes Papilloma-Virus

<400> 134
ttaaatttat acaaggtgta gttatttcgt at                                     32


<210> 135
<211> 26
<212> DNA
<213> Humanes Papilloma-Virus

<400> 135
taattagatt tttgcaaggg tgcgtt                                            26